# EUROPEAN PATENT APPLICATION

(11) **EP 1 849 781 A1**
(43) Date of publication of application: **31.10.2007**
(21) Application number: 06384009.4
(22) Date of filing: 28.04.2006
(51) Int. Cl.: C07D 401/04, C07D 207/14, C07D 471/04, C07D 401/12

(54) **Substituted 3-Amino-4-hydroxy pyrrolidines compounds, their preparation and use as medicaments**

(71) Applicant: Laboratorios del Dr. Esteve S.A., 08041 Barcelona (ES)
(72) Inventor: Pericas Brondo, Miguel Angel Institut Català d'Investigació Quimica (ICIQ), 43007 Tarragona (ES)
(74) Representative: Peters, Hajo

(57) **Abstract**

The present invention relates to substituted pyrrolidine compounds of general formula (I), 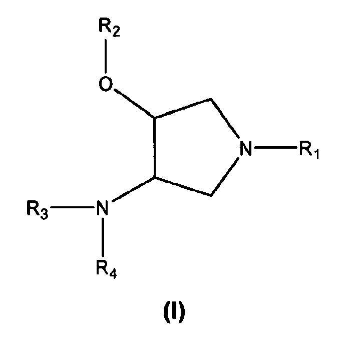 methods for their preparation, medicaments comprising these compounds as well as their use for the preparation of a medicament for the treatment of humans and animal.

## Description

The present invention relates to substituted pyrrolidine compounds, methods for their preparation, medicaments comprising these compounds as well as their use for the preparation of a medicament for the treatment of humans and animals.

Alzheimer's disease (AD) is a dreaded disease which suffer more and more people. By the year 2010 it is expected that 21 million people will have AD (For review on AD see: Lancet Neurol., 2004, 3:184-90); the herewith associated treatment costs are estimated of being over US$5 billion per year. The disease itself as well as the arising costs associated with the disease are a serious problem to be faced and an adequate treatment for AD is at the moment not existing.

The sigma receptor has been identified almost 20 years ago (reviewed in: Psychopharmacol., 2004, 174(3):301-319). At present two subtypes of sigma receptor are known. They were originally suggested being opioid receptors, however in the 90s they have been reclassified as being non-opioid receptors. From the very beginning, the sigma receptors were associated with various central nervous system disorders and recently the sigma receptor has been suggested of being implicated in neurological diseases such as AD (see e.g. U.S. 2005107432).

Recently it has been found that the sigma-1 receptor may be involved in the pathologenesis of Alzheimer's disease (Uchida et al., Am J Geriatr Psychiatry 2005; 13:1062-1066).

Thus, it was an object of the present invention to provide novel compounds for the use as active substances in medicaments, which are suitable for the prevention/treating Alzheimer's disease or binding to sigma receptor.

Said object was achieved by providing the substituted pyrrolidine compounds of general formula (I) given below, their stereoisomers, corresponding salts and corresponding solvates thereof.

Therefore, in one of its aspects the present invention relates to substituted pyrrolidine compounds of general formula (I), 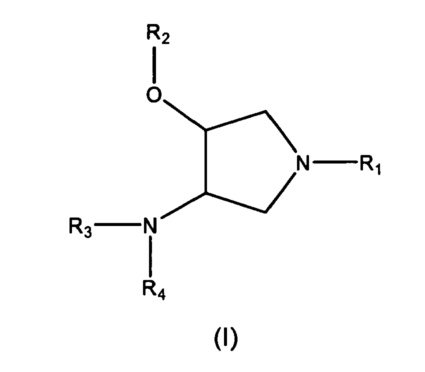 wherein
R¹ represents a hydrogen atom, an unbranched or branched, saturated, optionally at least mono-substituted C₁₋₆-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, optionally at least mono-substituted alkyl-aryl group in which the aryl group may be optionally at least mono-substituted and/or condensed with a mono- or polycyclic ring system; a branched or unbranched, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R⁷a group;
R² represents a hydrogen atom; a substituted or unsubstituted aliphatic group; a (C=O)-R⁸ group; a benzyl group which is optionally substituted by one or more substituents independently selected from the group consisting of F, Cl, Br, I, NH₂, SH, OH;
R³ and R⁴, equal or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
   or
   R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂-group;
      B represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂-group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂-group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵ and R⁶ represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁷ and R^{7a}, identical or different, represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁸ represents a hydrogen atom; a branched or unbranched, saturated or unsaturated, unsubstituted aliphatic group;
R⁹, R¹⁰ and R¹¹ represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R¹² and R ^{12a}, identical or different, represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
   with the provisos that
   if R² represents a hydrogen atom;
   and R¹ represents a hydrogen atom or a condensed aryl group;
   R³ and R⁴ may not form together with their bridging nitrogen atom a triazole group;
   if R² represents a hydrogen atom;
   and R¹ represents a hydrogen atom; a substituted C₁₋₂ alkyl group; a condensed aryl group; a benzyl group;
   R³ and R⁴ may not form together with their bridging nitrogen atom a substituted, unsaturated, condensed ring system containing at least 4 nitrogen atoms as ring members; an unsaturated, (C=O)-substituted, uncondensed, 6-membered ring system in which one additonal carbon atom is replaced by nitrogen.
   if R² represents a hydrogen atom;
   and R³ and R⁴ form together with their bridging nitrogen atom a pyrrolidine ring, a piperidine ring, a piperazine ring or a morpholine ring;
   R¹ may not represent a hydrogen atom; a methyl group; an unsaturated tert-butyl group; a (C=O)-substituted alkyl-heteroaryl group;
   if R² represents a hydrogen atom;
   and R¹ does not represent a benzyl group;
   and either R³ or R⁴ represents a hydrogen atom;
   either R³ or R⁴ may not represent a substituted alkyl-heteroaryl group; an alkyl-aryl group; a substituted heteroaryl group; an unsubstituted methyl group; a substituted alkyl group; an aryl group; a cyclohexyl group; an alkyl-cycloalkyl group wherein the cycloalkyl group containins at least one heteroatom;
   if R² represents a hydrogen atom;
   and R¹ represents an alkyl group;
   and either R³ or R⁴ represents a hydrogen atom;
   the other of R³ or R⁴ may not represent a substituted or unsubstituted C₁₋₂ alkyl group; a branched C₁₋₄ alkyl group; a substituted or unsubstituted C₃₋₆ cycloalkyl group in which optionally at least one carbon atom is optionally replaced by N, S or O; a cycloheptyl group;
   if R² represents a hydrogen atom;
   and R¹ represents an alkyl group;
   either R³ or R⁴ may not represent a cyclohexyl group while the other represents at the same time an alkyl-aryl group; or either R³ or R⁴ may not represent an alkyl group while the other represents at the same time an alkyl-aryl group;
   if R² represents a hydrogen atom;
   and R¹ represents a benzhydryl group;
   and either R³ or R⁴ represents a hydrogen atom;
   either R³ or R⁴ may not represent a a (C=O)-substituted alkyl-aryl group; a (C=O)-substituted aryl group;
   if R² represents a hydrogen atom;
   and R¹ represents benzyl;
   and either R³ or R⁴ represents a hydrogen atom;
   the other of R³ or R⁴ may not represent a substituted or unsubstituted C₁₋₂ alkyl group; an unsubstituted benzyl group; a C₃₋₈ substituted alkyl group;
   a substituted alkyl-aryl group;
   or
   R³ or R⁴, together with their bridging nitrogen atom may not form a a tetrahydropyrimidine ring; a triazolo pyrimidine ring;
   if R² represents a benzyl group;
   and either R³ or R⁴ represents a hydrogen atom
   either R³ or R⁴ may not represent a (C=O)-substituted alkyl-aryl group;
   if R² represents a C₁₋₃ alkyl group;
   and either R³ or R⁴ represents a hydrogen atom
   either R³ or R⁴ may not represent an unsubstituted C₁₋₂ alkyl group; a substituted alkyl group; a substituted or unsubstituted aryl group; a substituted heteroaryl group; a (C=O)-substituted alkyl-aryl group;
   optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

These compounds show binding to the Sigma receptor and/or show promise to be useful for the treatment of Alzheimer's disease.

As a general remark the claim to the compounds will cover as well any prodrug of the claimed and invented compounds as well as any use thereof, especially including their esters and ethers. Examples of well known methods of producing a prodrug of a given acting compound are known to those skilled in the art and can be found e.g. in Krogsgaard-Larsen et al., Textbook of Drug design and Discovery, Taylor & Francis (April 2002).

The term "condensed" according to the present invention means that a ring or ring-system is attached to another ring or ring-system, whereby the terms "annulated" or "annelated" are also used by those skilled in the art to designate this kind of attachment.

The term "ring" or "ring system" according to the present invention refers to ring sytems comprising saturated, unsaturated or aromatic carbocyclic ring sytems which contain optionally at least one heteroatom as ring member and which are optionally at least mono-substituted. Said ring systems may be condensed to other carbocyclic ring systems. Also, preferably included in the definition of "ring system" are spiro ring systems. Rings or ring systems, as defined above include, but are not restricted to aryl, heteroaryl, cycloalkyl, heterocyclyl, cyclyl, or spiro groups.

According to the present invention, R³ and R⁴ together with their bridging nitrogen atom form a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group. For a better understanding, some illustrative examples of ring system A, thereby not limiting the scope of said ring system A, is given below:

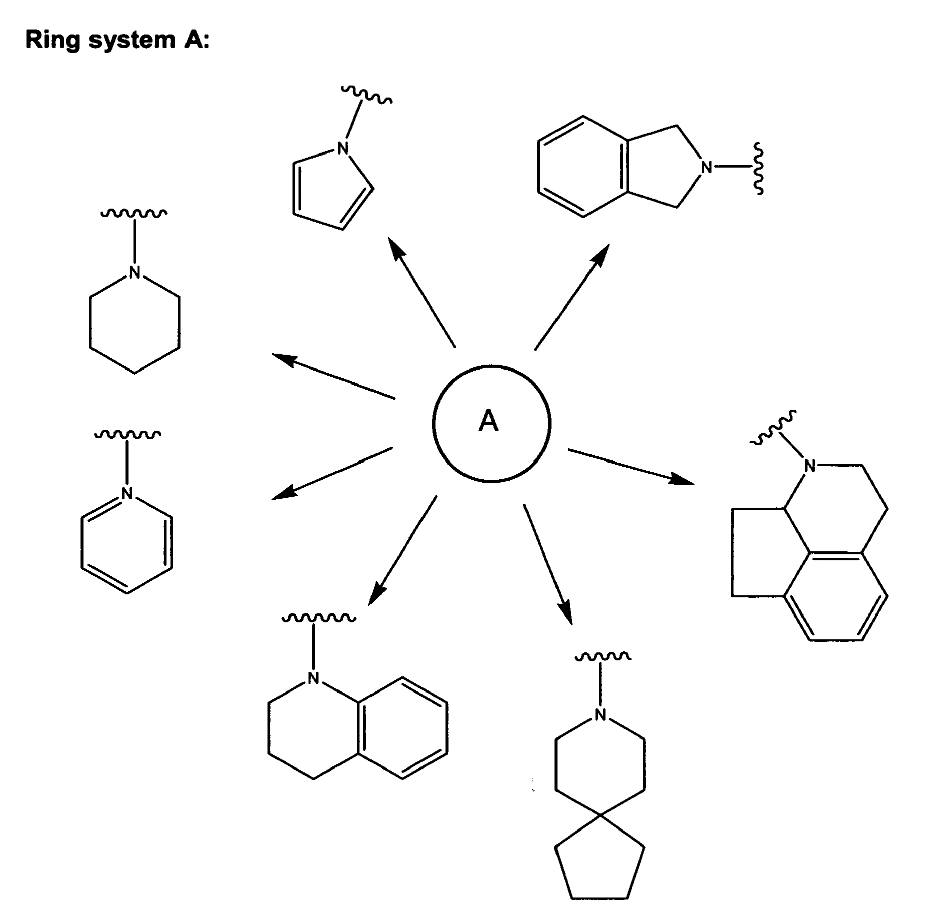

According to the present invention, ring system A may be connected via X with another ring system B, which represents a saturated or unsaturated ring system comprising 1 to 2 or 3 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group, wherein X preferably represents a bond, a -NH- group, a -(C=O)- group, a branched or linear, optionally substituted C₁₋₆-aliphatic chain, -O-, or a -SO₂- group.

Some illustrative examples for A-X-B, thereby not limiting the scope of said group, are given below:

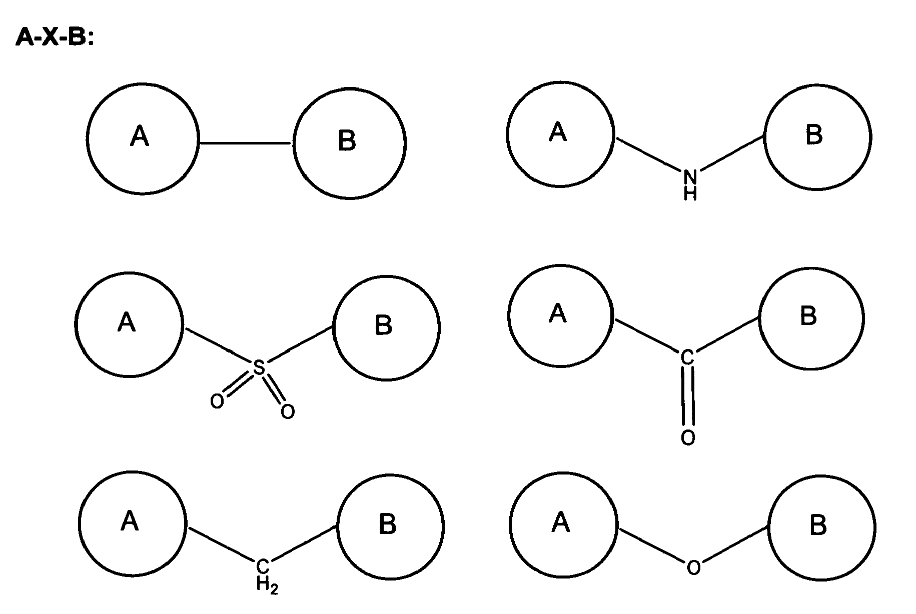

Similarly, an A-X-B-Y-C group, according to the present invention, refers to a ring system A, which is connected via X to a ring system B, with A, X and B as defined above, via Y to a ring system C. Said connector Y may represent a bond, a -NH- group, a -(C=O)- group; a branched or linear, optionally substituted C₁₋₆-aliphatic chain, -O-, or a -SO₂- group. Said ring system C ring system preferably represents a saturated or unsaturated ring system comprising 1 to 2 or 3 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;

Some illustrative examples for A-X-B-Y-C, thereby not limiting the scope of said group, are given below:

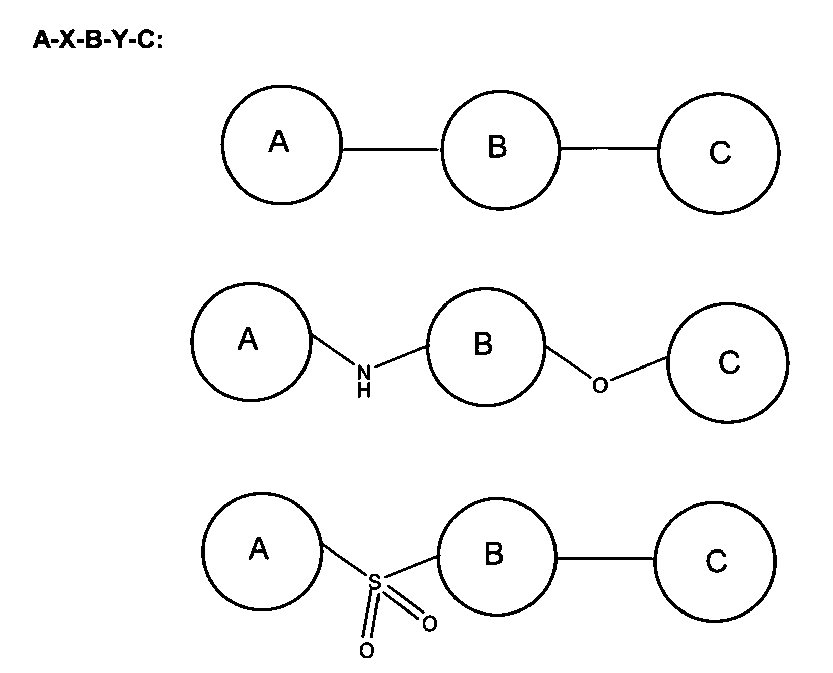

In an alternative embodiment of the present invention A represents a 4 to 18 membered ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;

Cycloalkyl radicals, as referred to in the present invention, are understood as meaning saturated and unsaturated (but not aromatic), cyclic hydrocarbons, which can optionally be unsubstituted, mono- or polysubstituted. In these radicals, for example C₃₋₄-cycloalkyl represents C₃- or C₄-cycloalkyl, C₃₋₅-cycloalkyl represents C₃-, C₄- or C₅-cycloalkyl, etc. With respect to cycloalkyl, the term also includes saturated cycloalkyls in which optionally at least one carbon atom may be replaced by a heteroatom, preferably S, N, P or O. However, mono-or polyunsaturated, preferably monounsaturated, cycloalkyls without a heteroatom in the ring also in particular fall under the term cycloalkyl as long as the cycloalkyl is not an aromatic system.

Examples for cycloalkyl radicals preferably include, but are not restricted to cyclopropyl, 2-methylcyclopropyl, cyclopropylmethyl, cyclobutyl, cyclopentyl, cyclopentylmethyl, cyclohexyl, cycloheptyl, cyclooctyl, acetyl, tert-butyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine.

Preferably, cycloalkyl radicals, as defined in the present invention may optionally be mono-or polysubstituted by F, Cl, Br, I, NH₂, SH, OH, SO₂, CF₃, carboxy, amido, cyano, carbamyl, nitro, -SO₂NH₂, C₁₋₆ alkyl or C₁₋₆-alkoxy.

Aliphatic radicals/groups, as referred to in the present invention, are optionally mono- or polysubstituted and may be branched or unbranched, saturated or unsaturated. Unsaturated aliphatic groups, as defined in the present invention, include alkenyl and alkinyl radicals. Saturated aliphatic groups, as defined in the present invention, include alkyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl. Preferred substituents for aliphatic radicals, according to the present invention, are F, Cl, Br, I, NH₂, SH, OH, SO₂, CF₃, carboxy, amido, cyano, carbamyl, nitro, phenyl, benzyl, -SO₂NH₂, C₁₋₆ alkyl and/or C₁₋₆-alkoxy.

Preferably, R³ and R⁴, according to the present invention, may not be represent aliphatic radicals which are substituted by a (C=O) group.

In alternative embodiment of the present invention, R³ and R⁴ may present at the same moment a hydrogen atom.

Preferably, with respect to the present invention, (C=O)-O groups, if not otherwise defined, do not represent substituents for aliphatic groups, cycloalkyl groups, aryl groups, heteroaryl groups, alky-cycloalkyl groups, alkyl-aryl groups or alkyl-heteroaryl groups.

The term (CH₂)₃₋₆ is to be understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-; (CH₂)₁₋₄ is to be understood as meaning -CH₂-, -CH₂-CH₂-, -CH₂-CH₂-CH₂- and -CH₂-CH₂-CH₂-CH₂-; (CH₂)₄₋₅ is to be understood as meaning -CH₂-CH₂-CH₂-CH₂- and -CH₂-CH₂-CH₂-CH₂-CH₂-, etc.

An aryl radical, as referred to in the present invention, is understood as meaning ring systems with at least one aromatic ring but without heteroatoms even in only one of the rings. These aryl radicals may optionally be mono-or polysubstituted with for example F, CI, Br, I, NH₂, SH, OH, SO₂, oxo, carboxy, amido, cyano, carbamyl, nitro, phenyl, benzyl, -SO₂NH₂, C₁₋₆ alkyl or C₁₋₆-alkoxy. Preferred examples of aryl radicals include but are not restricted to phenyl, naphthyl, fluoranthenyl, fluorenyl, tetralinyl or indanyl or anthracenyl radicals, which may optionally be mono- or polysubstituted.

A heteroaryl radical is understood as meaning heterocyclic ring systems which have at least one aromatic ring and may optionally contain one or more heteroatoms from the group consisting of nitrogen, oxygen and/or sulfur and may optionally be unsubstituted, mono- or polysubstituted by for example F, Cl, Br, I, NH₂, SH, OH, SO₂, oxo, carboxy, amido, cyano, carbamyl, nitro, henyl, benzyl, -SO₂NH₂, C₁₋₆ alkyl or C₁₋₆-alkoxy. Preferred examples of heteroaryls include but are not restricted to furan, benzofuran, thiophene, benzothiophene, pyrrole, pyridine, pyrimidine, pyridazine, pyrazine, quinoline, isoquinoline, phthalazine, benzo-1,2,5-thiadiazole, benzothiazole, indole, benzotriazole, benzodioxolane, benzodioxane, benzimidzole, carbazole and quinazoline.

An alkyl-aryl radical, as defined in the present invention, comprises a linear or branched, optionally at least mono-substituted alkyl chain which is bonded to an aryl group, as defined above. A preferred alkyl-aryl radical is a benzyl group, wherein the alkyl chain is optionally branched or substituted. Preferred substituents for alky-aryl radicals, according to the present invention, are F, Cl, Br, I, NH₂, SH, OH, SO₂, CF₃, carboxy, amido, cyano, carbamyl, nitro, phenyl, benzyl, -SO₂NH₂, C₁₋₆ alkyl and/or C₁₋₆-alkoxy.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH₄, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

The term "pharmacological tool" refers to the property of compounds of the invention through which they are particularly selective ligands for Sigma receptors which implies that compound of formula (I), described in this invention, can be used as a model for testing other compounds as sigma ligands, ex. a radiactive ligands being replaced, and can also be used for modeling physiological actions related to sigma receptors.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

A preferred embodiment of the present invention are compounds of general formula (I) as defined above,
wherein
R¹ represents a hydrogen atom, an unbranched or branched, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom; an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
   or
   R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

A preferred embodiment of the present invention are compounds of general formula (I) as defined above,
wherein
R¹ represents a hydrogen atom, an unbranched or branched, saturated, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl group; or a benzyl group which is optionally substituted by one or more substituents independently selected from the group consisting of F, Cl, Br, I, NH₂, SH, OH;
R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -0-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆₋alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

A preferred embodiment of the present invention are compounds of general formula (I) as defined above,
wherein
R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group; a branched or unbranched, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl, group; or a benzyl group which is optionally substituted by one or more substituents independently selected from the group consisting of F, Cl, Br, I, NH₂, SH, OH;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group; an unsubstituted heteroaryl group; a saturated or unsaturated, optionally at least mono-substituted alkyl-cycloalkyl group; an unsubstituted alkyl-aryl group; a saturated or unsaturated, optionally at least mono-substituted alkyl-heteroaryl group; with the condition that R³ and
R⁴ may not at the same time represent a hydrogen atom;
R⁵, R⁶, R⁷ and R^{7a} have the meaning as defined above;

A preferred embodiment of the present invention are compounds of general formula (I) as defined above,
wherein
R¹ represents an unbranched or branched, optionally at least mono-substituted C₂₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated unsubstituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; a (C=O)-NR⁷R^{7a} group;
R² represents a hydrogen atom;
R³ and R⁴ together with their bridging nitrogen atom form
   1) a ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a ring system in which the ring containing the nitrogen atom is saturated, but may be condensed with an unsaturated ring, comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -0-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -0-, or a -S0₂- group;
      C represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=0)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

A preferred embodiment of the present invention are compounds of general formula (I) as defined above,
wherein
R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
R⁵, R⁶, R⁷ and R^{7a} have the meaning as defined above;

A preferred embodiment of the present invention are compounds of general formula (I) as defined above,
wherein
R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cydoalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom, an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group; a branched or unbranched, optionally at least mono-substituted, optionally at least one heteroatom as ring member containing C₁₋₆-alkyl-cycloalkyl group; an optionally at least mono-substituted aryl group; an optionally at least mono-substituted heteroaryl group; a branched or unbranched, optionally at least mono-substituted C₁₋₆-alkyl-aryl group; a branched or unbranched, saturated or unsaturated, optionally at least mono-substituted C₁₋₆-alkyl-heteroaryl group; with the condition that R³ and R⁴ may at the same time not represent a hydrogen atom;
   or
   R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

A preferred embodiment of the present invention are compounds of general formula (I) as defined above,
wherein
R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a group; a (SO₂)-R⁶ group; or a (C=O)-NR⁷R^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

A preferred embodiment of the present invention are compounds of general formula (I) as defined above,
wherein
R¹ represents an optionally at least one heteroatom as ring member containing C₁₋₆-alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆-alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C_{1- 6-}alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a C₁₋₆ alkyl, group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; an optionally at least mono-substituted C₁₋₆ alkyl-cycloalkyl group; an unsubstituted C₁₋₆ alkyl-aryl group; an unsubstituted C₁₋₆ alkyl-heteroaryl group; with the condition that R³ and R⁴ may not at the same time represent a hydrogen atom;
R⁵, R⁶, R⁷ and R^{7a} have the meaning as defined above;

A preferred embodiment of the present invention are compounds of general formula (I) as defined above,
wherein
R¹ represents an unbranched or branched, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a hydrogen atom;
R³ and R⁴ together with their bridging nitrogen atom form
   1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R ^{12a} have the meaning as defined above;

A preferred embodiment of the present invention are compounds of general formula (I) as defined above,
wherein
R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, S0₂, CF₃, CN, nitro,-SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
R⁵, R⁶, R⁷ and R^{7a} have the meaning as defined above;

A preferred embodiment of the present invention are compounds of general formula (I) as defined above,
wherein
R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆-alkyl-heteroaryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, S0₂, CF₃, CN, nitro, -S0₂NH₂ or C_{I}-₆-alkoxy; a (SO₂)-R⁶ group;
R² represents a (C=O)-R⁸ group, with R⁸ representing a hydrogen atom; a methyl group; an ethyl group; a propyl group; a butyl group; a pentyl group; a hexyl group;
R³ and R⁴, equal or different, represent a hydrogen atom, an unbranched or branched C₁₋₈-alkyl group, which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a saturated or unsaturated, optionally at least one heteroatom as ring member containing cycloalkyl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, optionally at least one heteroatom as ring member containing C₁₋₆-alkyl-cycloalkyl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-heteroaryl group which is optionally substituted by one or more substituents independently selected from the group consisting of F, Cl, Br, I, NH₂, SH, OH, SO₂, CF₃, CN, nitro,-S0₂NH₂ or C₁₋₆-alkoxy; with the condition that R³ and R⁴ may at the same time not represent a hydrogen atom;
   or
   R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   2) an A-X-B group,
      wherein
      A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

A preferred embodiment of the present invention are compounds of general formula (I) as defined above,
wherein
R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro,-S0₂NH₂ or C₁-₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a group; a (SO₂)-R⁶ group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   2) an A-X-B group,
      wherein
      A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, CI, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, CI, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

A preferred embodiment of the present invention are compounds of general formula (I) as defined above,
wherein
R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, CI, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂₋R⁶ group;
R² represents a methyl, ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a C₁₋₆ alkyl-cycloalkyl group which is optionally substituted by one or more substituents independently selected from the group consisting of F, CI, Br, I, NH₂, SH, OH, CF₃; an unsubstituted C₁₋₆ alkyl-aryl group; an unsubstituted C₁₋₆ alkyl-heteroaryl group; with the condition that R³ and R⁴ may not at the same time represent a hydrogen atom;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

A preferred embodiment of the present invention are compounds of general formula (I) as defined above,
wherein
R¹ represents an unbranched or branched, saturated, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, CI, Br, I, NH₂, SH, OH, SO₂, CF₃, CN, nitro, -SO₂NH₂ or C₁₋₆-alkoxy; a branched or unbranched, saturated C₁₋₆-alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ and R⁴ together with their bridging nitrogen atom form
   1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
   2) an A-X-B group,
      wherein
      A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   3) an A-X-B-Y-C group,
      wherein
      A represents a 4 to 18 membered ring system in which the ring containing the nitrogen atom is saturated, but may be condensed with an unsaturated ring comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 6membered ring system, in which at least 1 carbon atom is optionally replaced by N or O and which is optionally at least mono-substituted by F, Cl, Br, I, NH₂, SH, OH, oxo, or CF₃;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetra hyd ro pyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

A preferred embodiment of the present invention are compounds of general formula (I) as defined above,
wherein
R¹ represents an optionally at least one heteroatom as ring member containing C₁₋₆-alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

A preferred embodiment of the present invention are compounds of general formula (I) as defined above,
wherein
R¹ represents a branched or unbranched C₁₋₆₋alkyl-aryl group wherein the C₁₋₆-alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a hydrogen atom; a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN; with the condition that R³ and R⁴ may not at the same time represent a hydrogen atom;
   or
   R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a saturated or unsaturated 4 to 18 membered ring system comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;

A preferred embodiment of the present invention are compounds of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a saturated or unsaturated 4 to 18 membered ring system comprising 1 to 3 optionally condensed rings, in which at least 1 carbon atom is optionally replaced by N or 0 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
      Y represents a bond, a -NH- group, a -(C=O)- group, a branched or linear, optionally substituted C₁₋₆-aliphatic chain, -O-, or a -S0₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;

A preferred embodiment of the present invention are compounds of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, optionally at least mono-substituted C₃₋₈ aliphatic group with substituents independently selected from the group consisting of F, Cl, Br, I, NH₂, SH, OH, CF₃, CN, nitro; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a saturated or unsaturated, optionally at least mono-substituted C₁₋₆ alkyl-cycloalkyl group; a saturated or unsaturated, unsubstituted C₁₋₆ alkyl-aryl group; a saturated or unsaturated, unsubstituted C₁₋₆ alkyl-heteroaryl group; with the condition that R³ and R⁴ may not at the same time represent a hydrogen atom;

A preferred embodiment of the present invention are compounds of general formula (I) as defined above,
wherein
R¹ represents a branched or unbranched C₁₋₆-alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆-alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ and R⁴ together with their bridging nitrogen atom form
   1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
   3) an A-X-B-Y-C group,
      wherein
      A represents a 4 to 18 membered ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;

A preferred embodiment of the present invention are compounds of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;

A preferred embodiment of the present invention are compounds of general formula (I) as defined above,
wherein
R¹ represents a benzyl group or a (SO₂)-R⁶ group with R⁶ being an aryl group;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN;
   or
   R³ and R⁴ together with their bridging nitrogen atom form
   1) a ring system A, comprising: 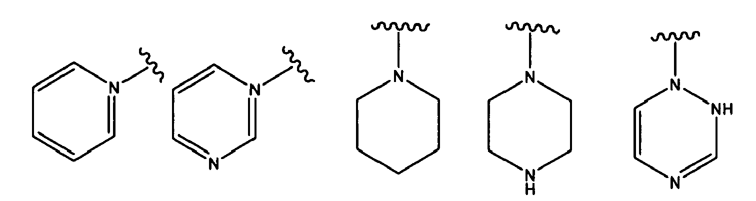 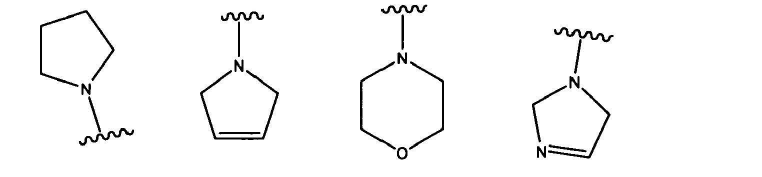 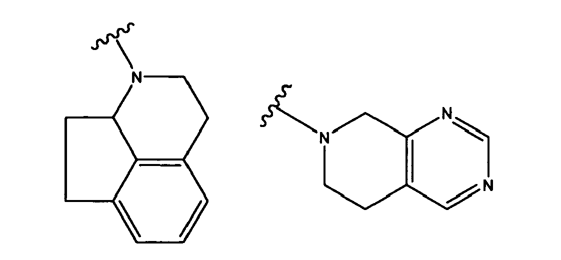 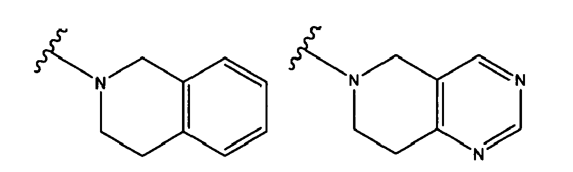 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a ring system comprising: 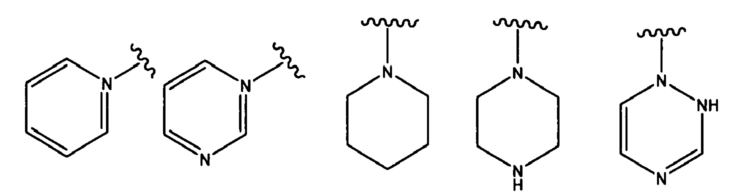 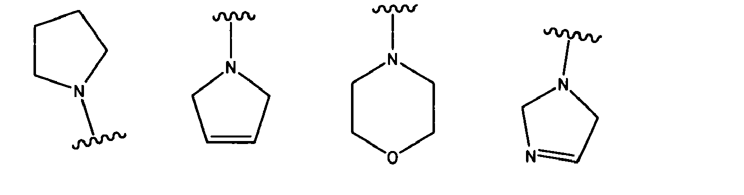 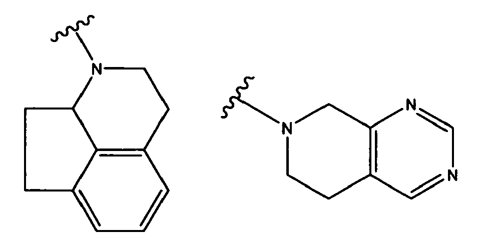 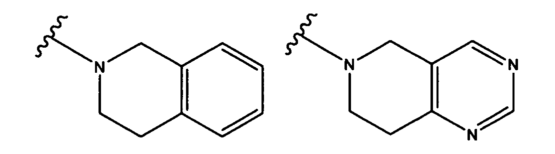 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear optionally substituted C₁₋₂ alkyl chain, -O-, or a -SO₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 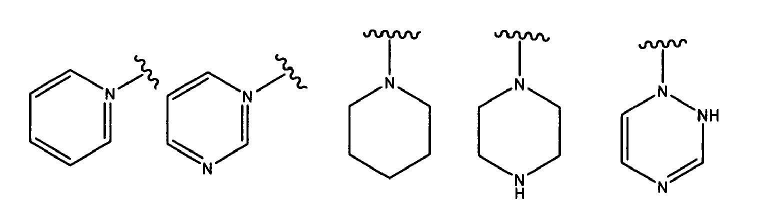 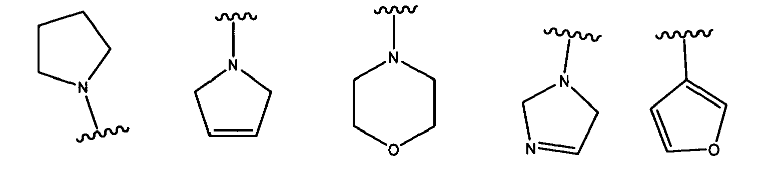 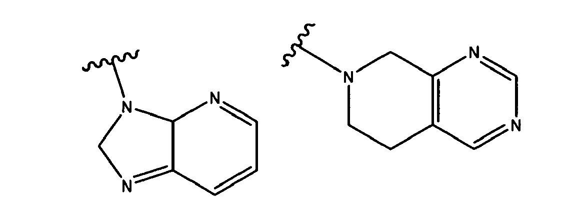 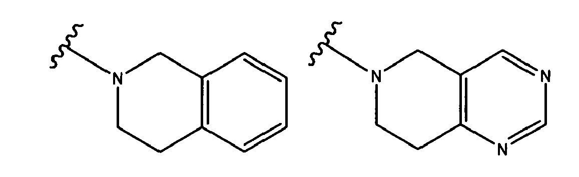 which is optionally at least mono-substituted by F, Cl, Br, I, NH₂, SH, OH, CF₃, a C₁₋₆-aliphatic group; a C₁₋₆-alkoxy group;
   3) an A-X-B-Y-C group,
      wherein
      A represents a ring system comprising: 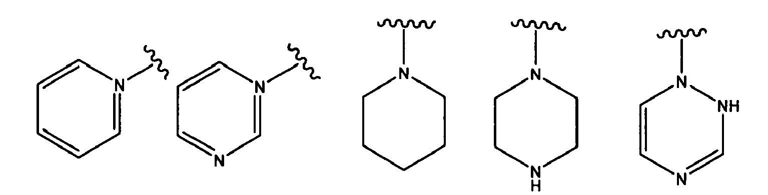 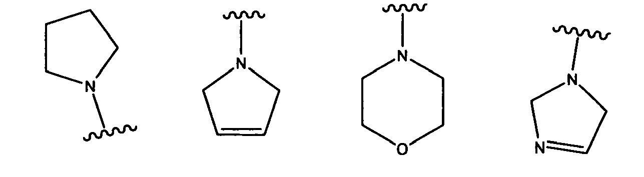 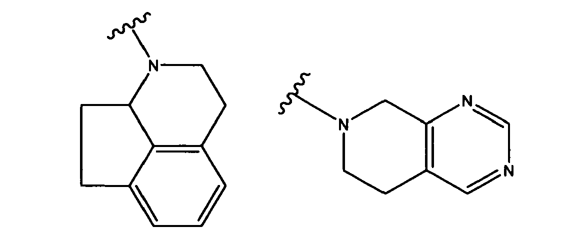 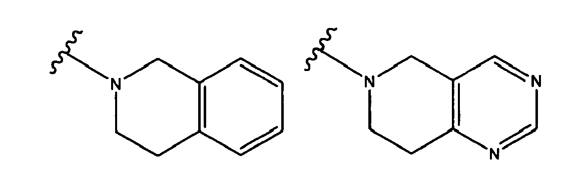 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear C₁₋₂ alkyl chain, -0-, or a -S0₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 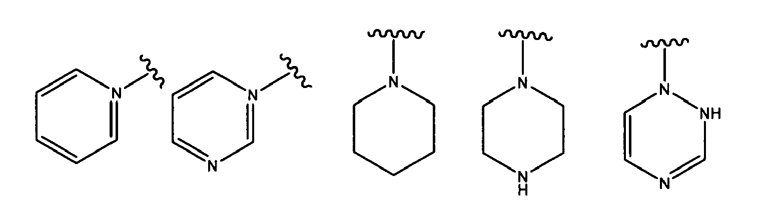 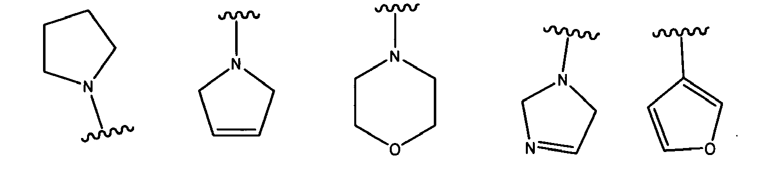 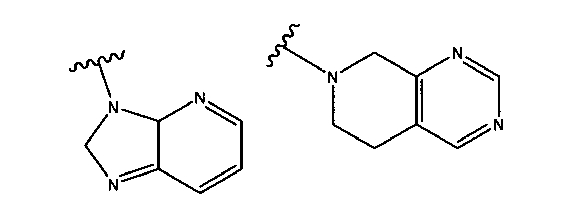 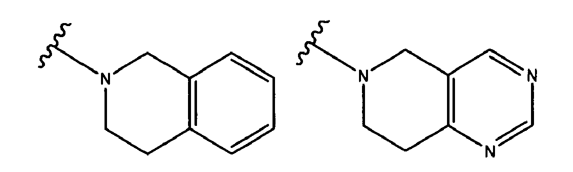 which is optionally at least mono-substituted by F, Cl, Br, I, NH₂, SH, OH, CF₃, a C₁₋₆-aliphatic group; a C₁₋₆-alkoxy group;
      Y represents a bond, a -NH- group, a linear C₁₋₂-aliphatic group;
      C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;

A preferred embodiment of the present invention are compounds of general formula (I) as defined above,
wherein
R¹ represents a benzyl group or a (SO₂)-R⁶ group with R⁶ being an aryl group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   a ring system A, comprising: 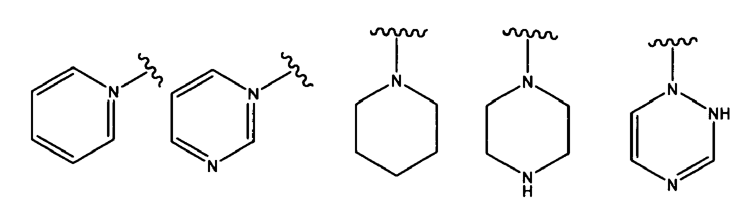 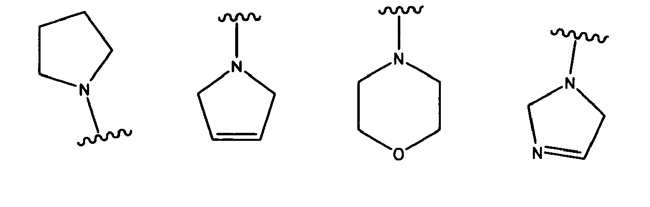 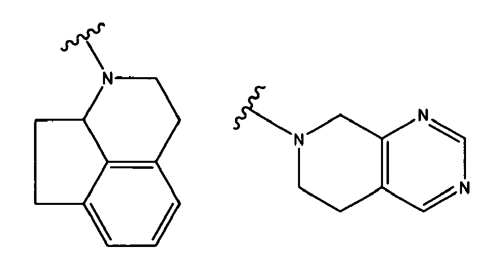 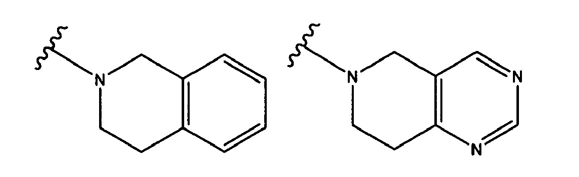 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   an A-X-B group,
   wherein
   A represents a ring system comprising: 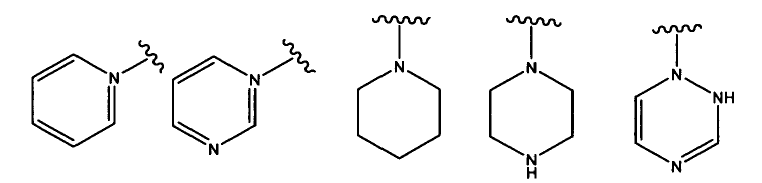 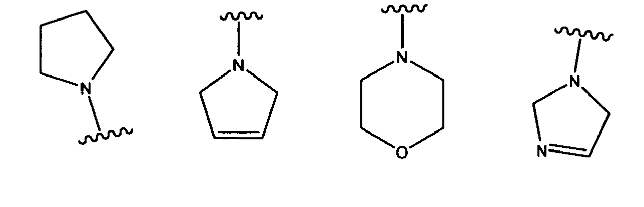 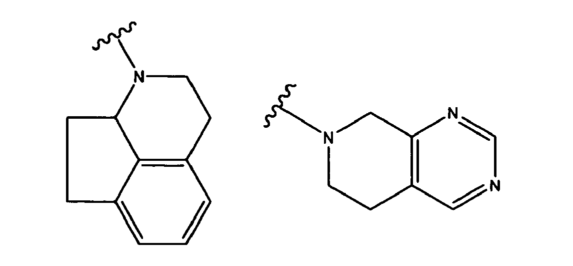 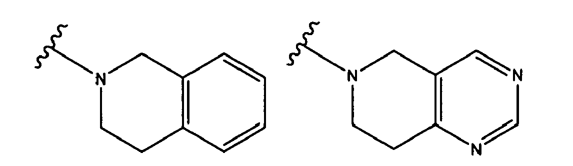 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   X represents a bond, a -NH- group, a -(C=O)- group, a linear C₁₋₂ alkyl chain, -0-, or a -SO₂- group;
   B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 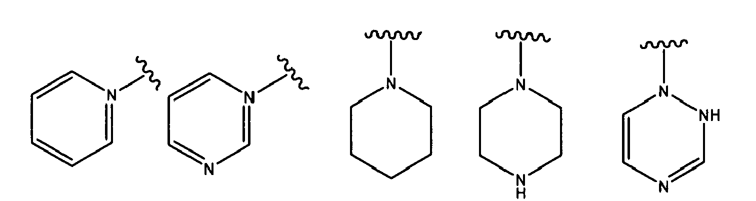 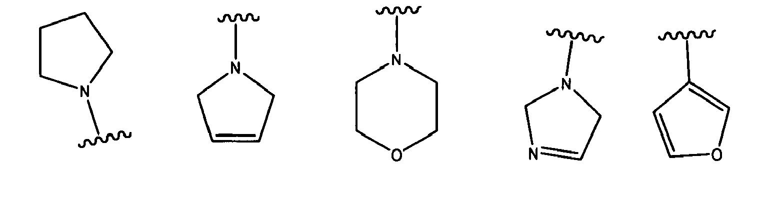 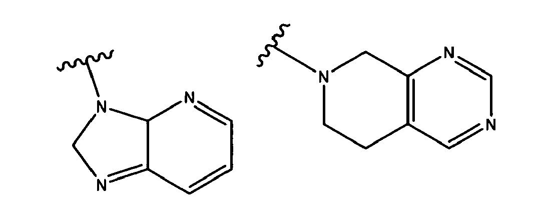 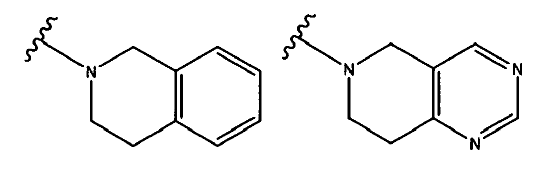 which is optionally at least mono-substituted by F, Cl, Br, I, NH₂, SH, OH, CF₃, a C₁₋₆-aliphatic group; a C₁₋₆-alkoxy group;
   an A-X-B-Y-C group,
   wherein
   A represents a ring system comprising: 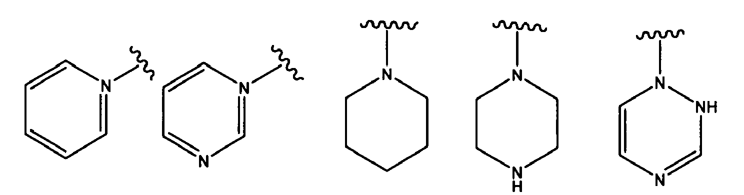 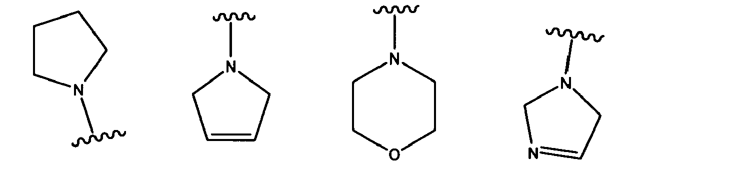 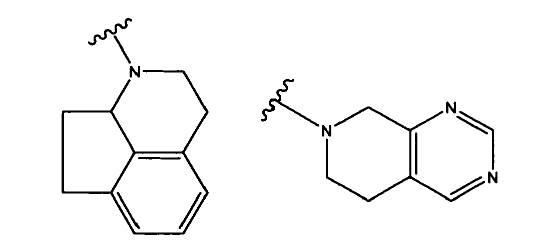 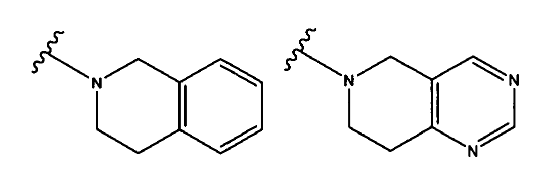 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   X represents a bond, a -NH- group, a -(C=O)- group, a linear C₁₋₂ alkyl chain, -O-, or a -SO₂- group;
   B represents an aryl group which is optionally at least mono-substituted by F, CI, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 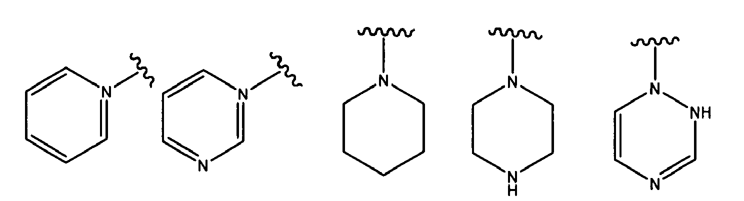 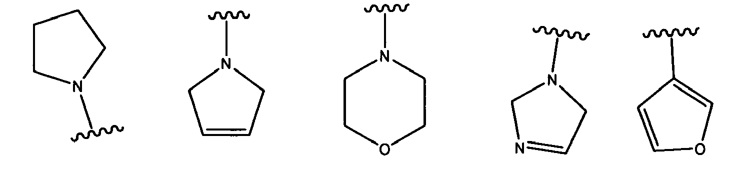 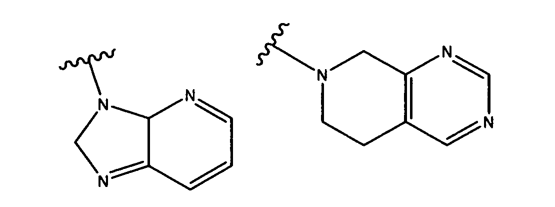 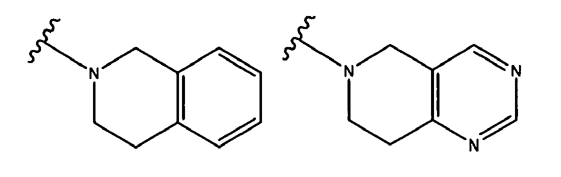 which is optionally at least mono-substituted by F, Cl, Br, I, NH₂, SH, OH, CF₃, a C₁₋₆-aliphatic group; a C₁₋₆-alkoxy group;
   Y represents a bond, a -NH- group, a linear C₁₋₂ alkyl group;
   C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;

A preferred embodiment of the present invention are compounds of general formula (I) as defined above,
wherein
R¹ represents a benzyl group or a (SO₂)-R⁶ group with R⁶ being an aryl group;
R² represents a hydrogen atom;
R³ and R⁴ together with their bridging nitrogen atom form
   a ring system A, comprising: 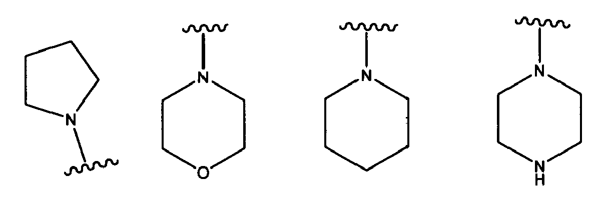 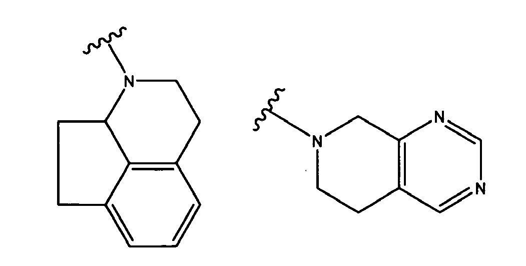 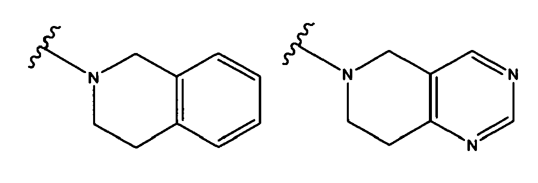 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   an A-X-B group,
   wherein
   A represents a ring system comprising: 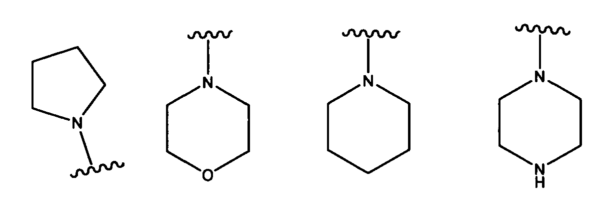 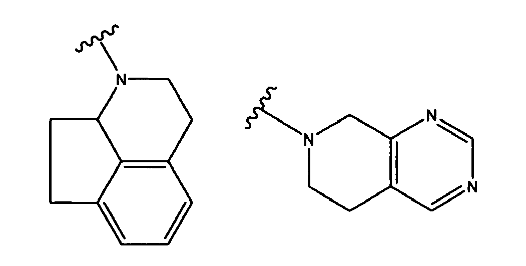 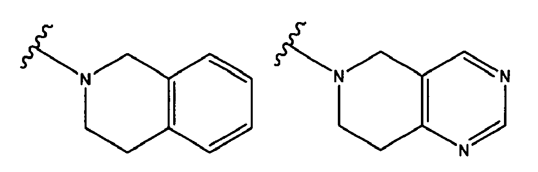 which is optionally at least mono-substituted by F, Cl, Br, I, NH₂, SH, OH, CF₃; a C₁₋₆-aliphatic group; a C₁₋₆-alkoxy group; a (C=O)-OR^{1o} group with R¹⁰ being a linear or branched C₁₋₆ alkyl group;
   X represents a bond, a -NH- group, a -(C=O)- group, a linear C₁₋₂ alkyl chain, -O-, or a -SO₂- group;
   B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 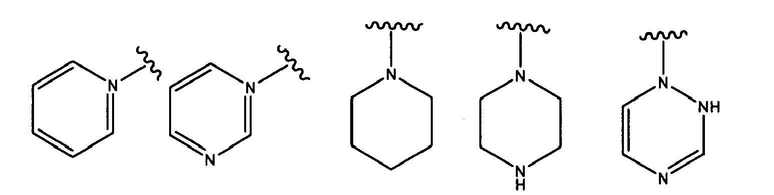 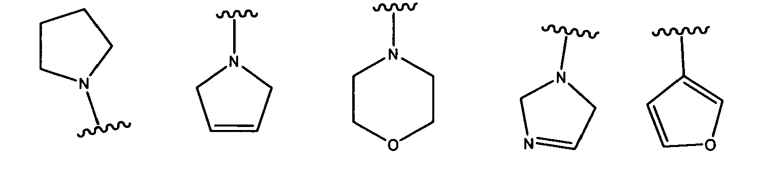 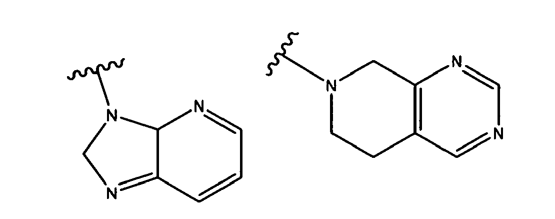 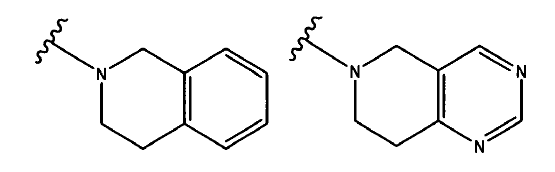 which is optionally at least mono-substituted by F, Cl, Br, I, NH₂, SH, OH, CF₃, a C₁₋₆-aliphatic group; a C₁₋₆-alkoxy group;
   an A-X-B-Y-C group,
   wherein
   A represents a ring system comprising: 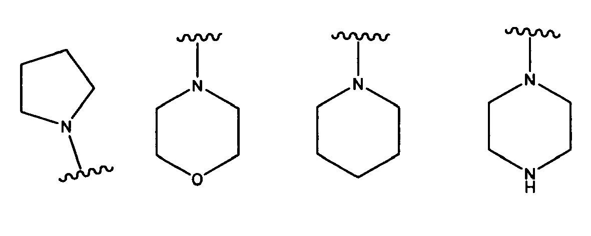 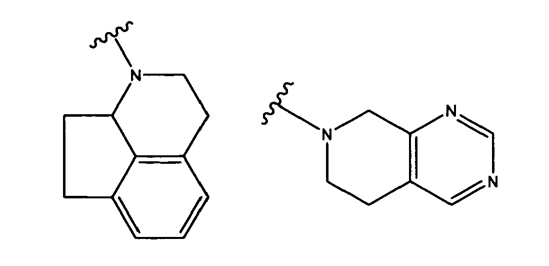 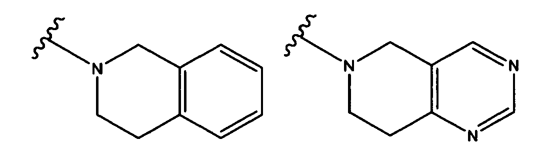 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   X represents a bond, a -NH- group, a -(C=O)- group, a linear C₁₋₂ alkyl chain, -O-, or a -SO₂- group;
   B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 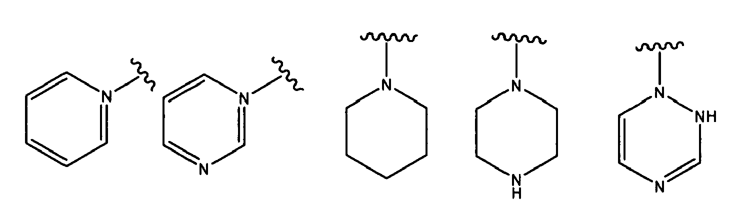 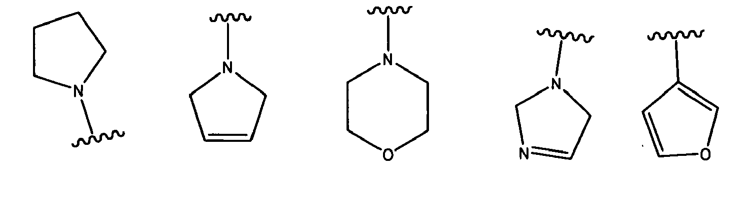 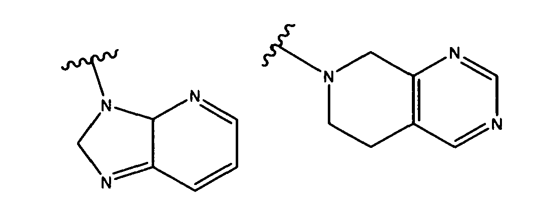 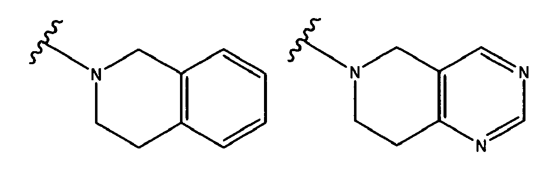 which is optionally at least mono-substituted by F, Cl, Br, I, NH₂, SH, OH, CF₃, a C₁₋₆-aliphatic group; a C₁₋₆ alkoxy group;
   Y represents a bond, a -NH- group, a linear C₁₋₂-alkyl group;
   C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;

In a highly preferred embodiment of the present invention the substituted pyrrolidine compounds are selected from the group consisting of:
[1] (3,4-trans)-1-benzyl-4-(3,4-dihydroisoquinolin-2(1H)-yl)pyrrolidine-3-ol,
[2] (3,4-trans)-1-benzyl-4-morpholinopyrrolidine-3-ol,
[3] (3,4-trans)-1-benzyl-4-(4-(2-methyl-3H-imidazo[4,5-b]pyridin-3-yl)piperidin-1-yl)pyrrolidine-3-ol,
[4] (3,4-trans)-1-benzyl-4-(3-(6,7-dimethoxy-3,4-dihydroisoquinolin-2(1H)-yl)propyl amino)pyrrolidine-3-ol,
[5] (3,4-trans)-1-benzyl-4-(4-((3,4-dihydroisoquinolin-2(1H)-yl)methyl)piperidin-1-yl)pyrrolidine-3-ol,
[6] 1-(4-(6-((3,4-trans)-1-benzyl-4-hydroxypyrrolidine-3-yl)-5,6,7,8-tetrahydropyrido [4,3-d]pyrimidin-2-ylamino)phenyl)ethanone,
[7] 1-(1-((3,4-trans)-1-benzyl-4-hydroxypyrrolidine-3-yl)piperidin-4-yl)-1H-benzo[d] [1,3] oxazin-2(4H)-one,
[8] (3,4-trans)-1-benzyl-4-(2-(4-phenoxyphenylamino)-7,8-dihydropyrido[4,3-d]pyrimidin-6(5H)-yl)pyrrolidine-3-ol,
[9] (3,4-trans)-1-benzyl-4-(3-(3,4-dihydroisoquinolin-2(1H)-yl)propylamino)pyrrolidine -3-ol,
[10] (3,4-trans)-1-benzyl-4-(4-(4-methoxypyrimidin-2-yl)piperazin-1-yl)pyrrolidine-3-ol,
[11] (3,4-trans)-1-benzyl-4-(2,3,8,8a-tetrahydrocyclopenta[ij]isoquinolin-1(7H)-yl) pyrrolidine-3-ol,
[12] tert-butyl-4-((3,4-trans)-1-benzyl-4-hydroxypyrrolidine-3-yl)piperazine-1-carboxylate,
[13] tert-butyl 4-((3,4-trans)-4-hydroxy-1-(phenylsulfonyl)pyrrolidine-3-yl)piperazine-1-carboxylate,
[14] (3,4-trans)-4-morpholino-1-(phenylsulfonyl)pyrrolidine-3-ol,
[15] (3,4-trans)-4-(3,4-dihydroisoquinolin-2(1H)-yl)-1-(phenylsulfonyl)pyrrolidine-3-ol,
[16] (3,4-trans)-4-(3,4-dihydroisoquinolin-2(1H)-yl)-1((S)-1-phenylethyl)pyrrolidine-3-ol,
[17] (3,4-trans)-1-benzyl-4-(4-benzylpiperazin-1-yl)pyrrolidine-3-ol,
[18] (3,4-trans)-1-benzyl-4-(4-phenylpiperazin-1-yl)pyrrolidine-3-ol,
[19] Ethyl 2-(4-((3,4-trans)-1-benzyl-4-hydroxypyrrolidine-3-yl)piperazin-1-yl)acetate,
[20] (4-((3,4-trans)-1-benzyl-4-hydroxypyrrolidine-3-yl)piperazin-1-yl)(phenyl) methanone,
[21] Ethyl 4-((3,4-trans)-1-benzyl-4-hydroxypyrrolidine-3-yl)piperazine-1-carboxilate,
[22] tert-butyl 4-((3,4-trans)-1-(4-fluorobenzyl)-4-hydroxypyrrolidine-3-yl)piperazine-1-carboxylate,
[23] (3,4-trans)-1-benzyl-4-(4-methylpiperazin-1-yl)pyrrolidine-3-ol,
[24] tert-butyl-4-(3,4-trans)-4-acetoxy-1-benzylpyrrolidine-3-yl)piperazine-1-carboxylate,
[25] (3,4-trans)-1-benzyl-4-(piperazin-1-yl)pyrrolidine-3-yl acetate,
[26] tert-butyl 4-((3,4-trans)-4-acetoxypyrrolidine-3-y)piperazine-1-carboxylate,
[27] trans 1-Benzyl-4-(4-(2-phenyl-phenylsulfonyl)piperazin-1-yl)pyrrolidin-3-yl acetate,
[28] trans 1-Benzyl-4-(4-(2-phenyl-phenylsulfonyl)piperazin-1-yl)pyrrolidin-3-ol dihydrochloride,
[29] (3,4-trans)-1-benzyl-4-(phenylamino)pyrrolidin-3-ol,
[30] (3,4-trans)-1-benzyl-4-(benzylamino)pyrrolidin-3-ol,
[31] (3R,4R)-1-benzyl-4-(octylamino)pyrrolidin-3-ol,
[32] (3,4-trans)-1-benzyl-4-(4-methoxybenzylamino)pyrrolidin-3-ol,
[33] (3,4-trans)-l -benzyl-4-(butylamino)pyrrolidin-3-ol,
[34] (3R,4R)-1-benzyl-4-(4-(4-chloro-2,5-dimethylphenylsulfonyl)piperazin-1-yl)pyrrolidin-3-yl acetate,
[35] (3R,4R)-1-benzyl-4-(4-tosylpiperazin-1-yl)pyn-olidin-3-yl acetate,
[36] (3,4-trans)-1-benzyl-4-(butylamino)pyrrolidin-3-ol,
[37] (3R,4R)-1-benzyl-4-(4-methoxyphenylamino)pyrrolidin-3-ol,
[38] Methyl 4-((3,4-trans)-1-benzyl-4-hydroxypyrrolidin-3-ylamino)benzoate,
[39] (3R,4R)-1-benzyl-4-(4-(naphthalen-1-ylsulfonyl)piperazin-1-yl)pyrrolidin-3-yl acetate,
[40] (3R,4R)-1-benzyl-4-(4-(2-chlorophenylsulfonyl)piperazin-1-yl)pyrrolidin-3-ol,
[41] (3R,4R)-1-benzyl-4-(4-(2-bromophenylsulfonyl)piperazin-1-yl)pyrrolidin-3-yl acetate,
[42] (3R,4R)-1-benzyl-4-(4-(2-bromophenylsulfonyl)piperazin-1-yl)pyrrolidin-3-ol,
[43] (3R,4R)-1-benzyl-4-(4-(5-fluoro-2-methylphenylsulfonyl)piperazin-1-yl)pyrrolidin-3-yl acetate,
[44] (3R,4R)-1-benzyl-4-(4-(5-fluoro-2-methylphenylsulfonyl)piperazin-1-yl)pyrrolidin-3-ol,
[45] (3R,4R)-1-benzyl-4-(4-(phenylsulfonyl)piperazin-1-yl)pyrrolidin-3-yl acetate,
[46] (3R,4R)-1-benzyl-4-( 4-(phenylsulfonyl)piperazin-1-yl)pyrrolidin-3-ol,
[47] (3R,4R)-1-benzyl-4-(4-(2-fluorophenylsulfonyl)piperazin-1-yl)pyrrolidin-3-yl acetate,
[48] (3R,4R)-1-benzyl-4-(4-(2-fluorophenylsulfonyl)piperazin-1-yl)pyrrolidin-3-ol,
[49] (3R,4R)-1-benzyl-4-(4-(naphthalen-1-ylsulfonyl)piperazin-1-yl)pyrrolidin-3-ol,
[50] (3R,4R)-1-benzyl-4-(4-(4-chloro-2,5-dimethylphenylsulfonyl)piperazin-1-yl)pyrrolidin-3-ol,
[51] (3R,4R)-1-benzyl-4-(4-tosylpiperazin-1-yl)pyrrolidin-3-ol,
[52] (3R,4R)-1-benzyl-4-(4-(2-chlorophenylsulfonyl)piperazin-1-yl)pyrrolidin-3-yl acetate,
optionally in form of a corresponding salt, or a corresponding solvate.

In another aspect the present invention also provides a process for the manufacture of substituted pyrrolidine compounds of general formula (I) given above, characterized in that at least one pyrroline compound of general formula (II), 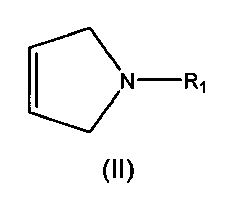 wherein R¹ has the meaning given above, is reacted with a per-acid, preferably MCPBA to form a compound of general formula (III), 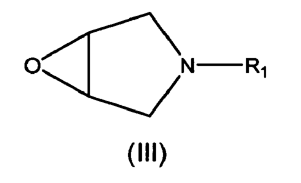 wherein R¹ has the meaning given above, followed by reacting the epoxyo-pyrroline compound of formula III with a compound of formula IV 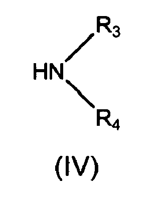 together with LiClO₄, ACN at preferably 90° C to give a compound of formula I, in which R² is H; alternatively followed by reacting the pyrrolidine compound of formula (I) with a base to give a compound of formula (I), in which R² has the meaning as defined above.

The process for obtaining the substituted pyrrolidine compounds of general formula given above is well known for those skilled in the art and is described in detail in e.g. EP 0985664, Synth. Commun, 20(2), 227-230 (1990), Synth. Commun., 34(23), 4421-4430 (2004), JP 11349565, JP-2000-80077, Tetrahedron: Asymmetry 12 (2001), 2989-2997, DE 4309964 and Org. Biomol. Chem. 2004, 2, 2418-2420.

The intermediates of general formula (I)I with R¹ being alkyl, cycloalkyl, aryl, alkylaryl, alkyl-heteroaryl are obtained by reacting an amine of general formula VI

R¹-NH₂ (VI)

with cis-1,4-dichloro-2-butene and dichloromethane at room temperature.

Intermediates of general formula (I)I with R¹ being (C=O)-R, (C=O)O-R or (SO₂)-R are obtained by reacting an amine of general formula VI

R¹-NH₂ (VI)

with cis-1 ,4-dichloro-2-butene, NaH, dimethylformamide (DMF) at room temperature.

Methods for obtaining epoxyo-pyrroline compound of formula III as given above, with R¹ being alkyl, cycloalkyl, aryl, alkylaryl, alkyl-heteroaryl, are described in EP 0985664.

Methods for obtaining epoxyo-pyrroline compound of formula III as given above, with R¹ being (C=O)-R, (C=O)O-R or (SO₂)-R, are described in DE 4309964 and Org. Biomol. Chem. 2004, 2, 2418-2420.

The process for obtaining substituted pyrrolidine compounds given above is also illustrated in scheme I given below:

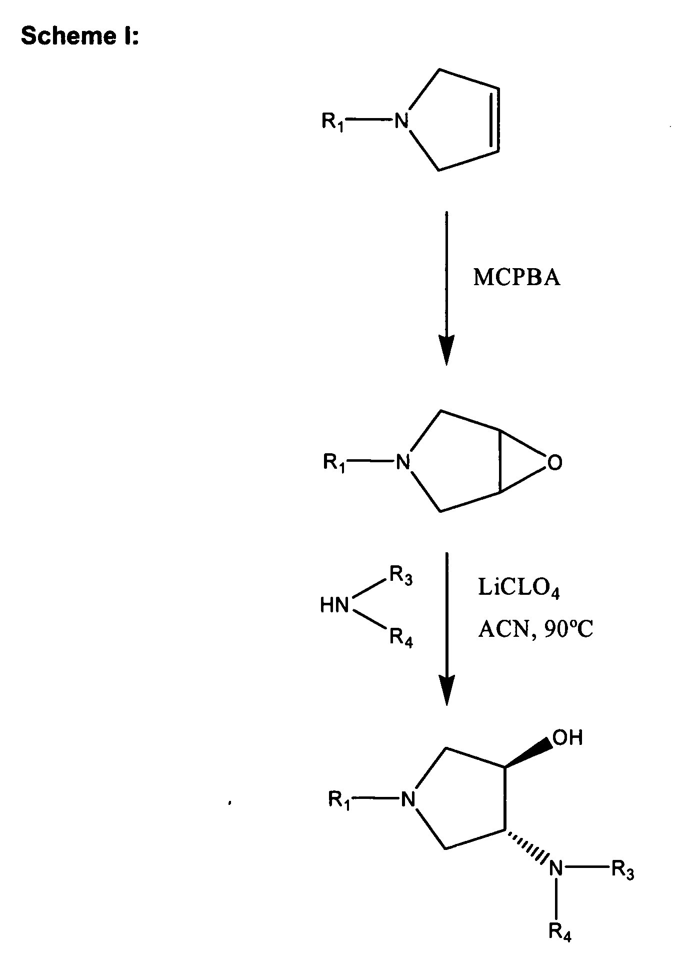

Alternatively, compounds of general formula (I) can be obtained by standard procedures known by those skilled in the art.

The alternative processes is shown in scheme 2 below. A) refers to non-cyclic substituents formed by R³ and R⁴, B) refers to cyclic substituents formed by R³ and R⁴, wherein R³ and R⁴ have the meaning as defined above and R^{x} represents F, Cl, Br, I, NH₂, SH, OH, CF₃, a substituted or unsubstituted C₁₋₆-aliphatic group; a substituted or unsubstituted C₁₋₆-alkyl-aryl group; a C₁₋₆-alkyl-heteroaryl group; a substituted or unsubstituted aryl group; a substituted or unsubstituted heteroaryl group; a C₁₋₆-alkoxy group; a (C=O)-R⁹ group; a (C=O)-OR¹⁰ group; a (SO₂)-R¹¹ group; a NH-R¹¹ group; or a (C=O)-NR¹²R^{12a} group

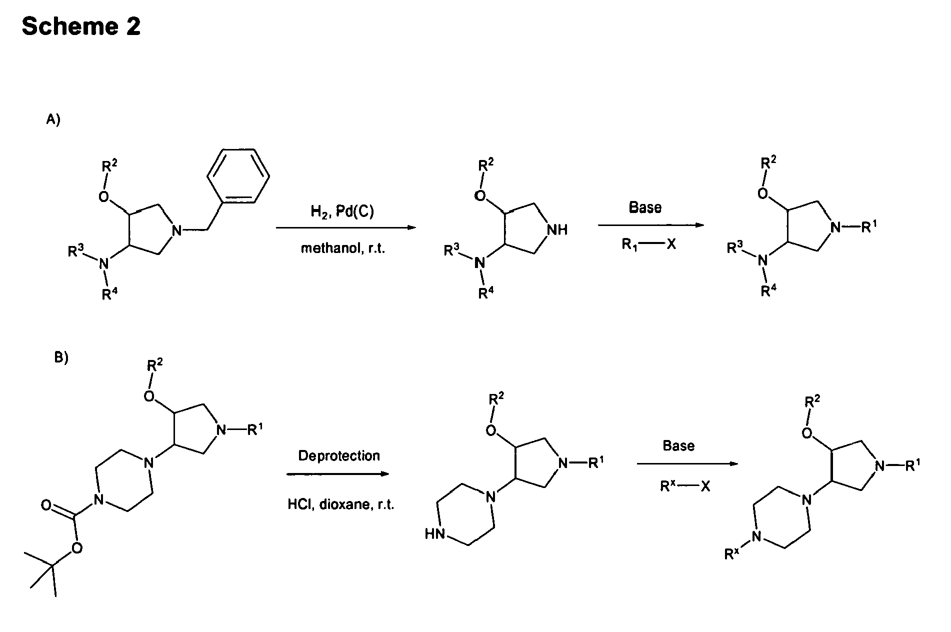

### General procedure for the epoxide opening with amines.

A mixture of epoxide (1 eq), amine (1.5 eq) and LiClO₄ (2 eq) in acetonitrile was heated at 90°C until starting epoxide is not present (4-48 hours). The reaction mixture was cooled at room temperature and the solvent was evaporated under vacuum. The residue was suspended in dichloromethane and washed with water and saturated solution of NaCl, dried over Na₂S0₄, filtered and concentrated and the residue was purified by flash chromatography.

During the processes described above the protection of sensitive groups or of reagents may be necessary and/or desirable. The introduction of conventional protective groups as well as their removal may be performed by methods well-known to those skilled in the art.

If the substituted pyrrolidine compounds of general formula (I) themselves are obtained in form of a mixture of stereoisomers, particularly enantiomers or diastereomers, said mixtures may be separated by standard procedures known to those skilled in the art, e.g. chromatographic methods or fractionalized crystallization with chiral reagents. It is also possible to obtain pure stereoisomers via stereoselective synthesis.

Solvates, preferably hydrates, of the substituted pyrrolidine compounds of general formula (I), of corresponding stereoisomers, or of corresponding salts thereof may also be obtained by standard procedures known to those skilled in the art.

The purification and isolation of the inventive substituted pyrrolidine compounds of general formula (I), of a corresponding stereoisomer, or salt, or solvate or any intermediate thereof may, if required, be carried out by conventional methods known to those skilled in the art, e.g. chromatographic methods or recrystallization.

It has been found that the substituted pyrrolidine compounds of general formula (I) and given below, stereoisomers thereof, corresponding salts and corresponding solvates have high affinity to sigma receptors, i.e. they are selective ligands for the sigma receptor and act as modulators, e.g. antagonists, inverse agonists or agonists, on these receptors.

The substituted pyrrolidine compounds of general formula (I) given below, their stereoisomers, corresponding salts thereof and corresponding solvates are toxicologically acceptable and are therefore suitable as pharmaceutical active substances for the preparation of medicaments.

Another aspect of the invention is a medicament comprising at least one combination of compounds according to the invention and optionally one or more pharmaceutically acceptable excipients.

Another aspect of the present invention relates to a medicament comprising at least one substituted pyrazoline compound of general formula (I), 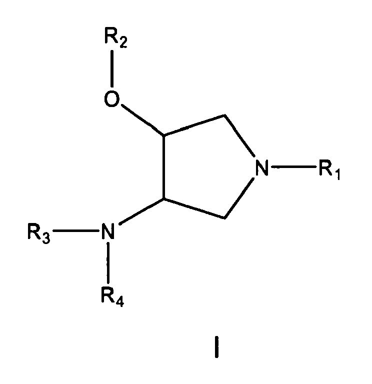 wherein
R¹ represents a hydrogen atom, an unbranched or branched, saturated, optionally at least mono-substituted C₁₋₆-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a hydrogen atom; a substituted or unsubstituted aliphatic group; a (C=O)-R⁸ group; a benzyl group which is optionally substituted by one or more substituents independently selected from the group consisting of F, Cl, Br, I, NH₂, SH, OH;
R³ and R⁴, equal or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
   or
   R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵ and R⁶ represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁷ and R^{7a}, identical or different, represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁸ represents a hydrogen atom; a branched or unbranched, saturated or unsaturated, unsubstituted aliphatic group;
R⁹, R¹⁰ and R¹¹ represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R¹² and R^{12a}, identical or different, represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
   with the provisos that
   if R² represents a hydrogen atom;
   and R¹ represents a hydrogen atom or a condensed aryl group;
   R³ and R⁴ may not form together with their bridging nitrogen atom a triazole group;
   if R² represents a hydrogen atom;
   and R¹ represents a hydrogen atom; a substituted C₁₋₂ alkyl group; a condensed aryl group; a benzyl group;
   R³ and R⁴ may not form together with their bridging nitrogen atom a substituted, unsaturated, condensed ring system containing at least 4 nitrogen atoms as ring members; an unsaturated, (C=O)-substituted, uncondensed, 6-membered ring system in which two carbon atoms are replaced by nitrogen.
   if R² represents a hydrogen atom;
   and R³ and R⁴ form together with their bridging nitrogen atom a pyrrolidine ring, a piperidine ring, a piperazine ring or a morpholine ring;
   R¹ may not represent a hydrogen atom; a methyl group; an unsaturated tert-butyl group; a (C=O)-substituted alkyl-heteroaryl group;
   if R² represents a hydrogen atom;
   and R¹ does not represent a benzyl group;
   and either R³ or R⁴ represents a hydrogen atom;
   either R³ or R⁴ may not represent a substituted alkyl-heteroaryl group; an alkyl-aryl group; a substituted heteroaryl group; an unsubstituted methyl group; a substituted alkyl group; an aryl group; a cyclohexyl group; an alkyl-cycloalkyl group wherein the cycloalkyl group containins at least one heteroatom;
   if R² represents a hydrogen atom;
   and R¹ represents an alkyl group;
   and either R³ or R⁴ represents a hydrogen atom;
   either R³ or R⁴ may not represent a substituted or unsubstituted C₁₋₂ alkyl group; a branched C₁₋₄ alkyl group; a substituted or unsubstituted C₃₋₆ cycloalkyl group in which optionally at least one carbon atom is optionally replaced by N, S or O; a cycloheptyl group;
   if R² represents a hydrogen atom;
   and R¹ represents an alkyl group;
   either R³ or R⁴ may not represent a cyclohexyl group while the other represents at the same time an alkyl-aryl group; or either R³ or R⁴ may not represent an alkyl group while the other represents at the same time an alkyl-aryl group;
   if R² represents a hydrogen atom;
   and R¹ represents a benzhydryl group;
   and either R³ or R⁴ represents a hydrogen atom;
   either R³ or R⁴ may not represent a a (C=O)-substituted alkyl-aryl group; a (C=O)-substituted aryl group;
   if R² represents a hydrogen atom;
   and R¹ represents benzyl;
   and either R³ or R⁴ represents a hydrogen atom;
   either R³ or R⁴ may not represent a substituted or unsubstituted C₁₋₂ alkyl group; a substituted C₃₋₈ alkyl group; a substituted aryl group; a substituted alkyl-aryl group;
   or
   R³ or R⁴, together with their bridging nitrogen atom may not form a a tetrahydropyrimidine ring; a triazolo pyrimidine ring;
   if R² represents a benzyl group;
   and either R³ or R⁴ represents a hydrogen atom;
   either R³ or R⁴ may not represent a (C=O)-substituted alkyl-aryl group;
   if R² represents a C₁₋₃ alkyl group;
   and either R³ or R⁴ represents a hydrogen atom
   either R³ or R⁴ may not represent an unsubstituted C₁₋₂ alkyl group; a substituted alkyl group; a substituted or unsubstituted aryl group; a substituted heteroaryl group; a (C=O)-substituted alkyl-aryl group;
   optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

Also preferred is a medicament comprising at least one substituted pyrazoline compound of general formula (I) as defined above,
wherein
R¹ represents a hydrogen atom, an unbranched or branched, saturated, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (S0₂)-R^{6 6} group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom; an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
   or
   R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-_{NR}¹²_{R}^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

Also preferred is a medicament comprising at least one substituted pyrazoline compound of general formula (I) as defined above,
wherein
R¹ represents a hydrogen atom, an unbranched or branched, saturated, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl group; or a benzyl group which is optionally substituted by one or more substituents independently selected from the group consisting of F, Cl, Br, I, NH₂, SH, OH;
R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, CI, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a}have the meaning as defined above;

Also preferred is a medicament comprising at least one substituted pyrazoline compound of general formula (I) as defined above,
wherein
R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group; a branched or unbranched, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl group; or a benzyl group which is optionally substituted by one or more substituents independently selected from the group consisting of F, Cl, Br, I, NH₂, SH, OH;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group; an unsubstituted heteroaryl group; a saturated or unsaturated, optionally at least mono-substituted alkyl-cycloalkyl group; an unsubstituted alkyl-aryl group; a saturated or unsaturated, optionally at least mono-substituted alkyl-heteroaryl group; with the condition that R³ and
R⁴ may not at the same time represent a hydrogen atom;
R⁵, R⁶, R⁷ and R^{7a} have the meaning as defined above;

Also preferred is a medicament comprising at least one substituted pyrazoline compound of general formula (I) as defined above,
wherein
R¹ represents an unbranched or branched, optionally at least mono-substituted C₂₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated unsubstituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; a (C=O)-NR⁷R^{7a} group;
R² represents a hydrogen atom;
R³ and R⁴ together with their bridging nitrogen atom form
   1) a ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, 1, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

Also preferred is a medicament comprising at least one substituted pyrazoline compound of general formula (I) as defined above,
wherein
R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; or a (C=O)-NR⁷R^{7a} group;
_{R}² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted alkyl-aryl group; an unsubstituted C₁₋₆ alkyl-heteroaryl group;
R⁵, R⁶, R⁷ and R^{7a} have the meaning as defined above;

Also preferred is a medicament comprising at least one substituted pyrazoline compound of general formula (I) as defined above,
wherein
R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, S0₂, CF₃, CN, nitro, -SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom, an unbranched or branched, saturated or unsaturated, optionally at least mono-substituted C_{1- 8}-aliphatic radical; a saturated or unsaturated, optionally at least mono-substituted, optionally at least one heteroatom as ring member containing cycloalkyl group; a branched or unbranched, optionally at least mono-substituted, optionally at least one heteroatom as ring member containing C₁₋₆-alkyl-cycloalkyl group; an optionally at least mono-substituted aryl group; an optionally at least mono-substituted heteroaryl group; a branched or unbranched, optionally at least mono-substituted C₁₋₆-alkyl-aryl group; a branched or unbranched, saturated or unsaturated, optionally at least mono-substituted C₁₋₆-alkyl-heteroaryl group; with the condition that R³ and R⁴ may at the same time not represent a hydrogen atom;
   or
   R³ and R⁴ together with their bridging nitrogen atom form
   1) a saturated or unsaturated ring system A, comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -0-, or a -S0₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=0)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

Also preferred is a medicament comprising at least one substituted pyrazoline compound of general formula (I) as defined above,
wherein
R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a group; a (SO₂)-R⁶ group; or a (C=O)-NR⁷R^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

Also preferred is a medicament comprising at least one substituted pyrazoline compound of general formula (I) as defined above,
wherein
R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, CI, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, S0₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃-₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; an optionally at least mono-substituted C₁₋₆ alkyl-cycloalkyl group; an unsubstituted C₁₋₆ alkyl-aryl group; an unsubstituted C₁₋₆ alkyl-heteroaryl group; with the condition that R³ and R⁴ may not at the same time represent a hydrogen atom;
R⁵, R⁶, R⁷ and R^{7a} have the meaning as defined above;

Also preferred is a medicament comprising at least one substituted pyrazoline compound of general formula (I) as defined above,
wherein
R¹ represents an unbranched or branched, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a hydrogen atom;
R³ and R⁴ together with their bridging nitrogen atom form
   1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   2) an A-X-B group,
      wherein
      A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
   3) an A-X-B-Y-C group,
      wherein
      A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning as defined above;

Also preferred is a medicament comprising at least one substituted pyrazoline compound of general formula (I) as defined above,
wherein
R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted allkyl-aryl group; an unsubstituted C₁₋₆ alkyl-heteroaryl group;
R⁵, R⁶, R⁷ and R^{7a} have the meaning as defined above;

Also preferred is a medicament comprising at least one substituted pyrazoline compound of general formula (I) as defined above,
wherein
R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆-alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a (C=O)-R⁸ group, with R⁸ representing a hydrogen atom; a methyl group; an ethyl group; a propyl group; a butyl group; a pentyl group; a hexyl group;
R³ and R⁴, equal or different, represent a hydrogen atom, an unbranched or branched C₁₋₈-alkyl group, which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a saturated or unsaturated, optionally at least one heteroatom as ring member containing cycloalkyl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, optionally at least one heteroatom as ring member containing C₁₋₆-alkyl-cycloalkyl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; with the condition that R³ and R⁴ may at the same time not represent a hydrogen atom;
   or
   R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹group; a NH-R¹¹ group;
   2) an A-X-B group,
      wherein
      A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, CI, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, CI, Br, I, NH₂, SH, OH, CF₃;

Also preferred is a medicament comprising at least one substituted pyrazoline compound of general formula (I) as defined above,
wherein
R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated, C₁₋₆-alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, - SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a group; a (SO₂)-R⁶ group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   2) an A-X-B group,
      wherein
      A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-,or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

Also preferred is a medicament comprising at least one substituted pyrazoline compound of general formula (I) as defined above,
wherein
R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a methyl, ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a C₁₋₆ alkyl-cycloalkyl group which is optionally substituted by one or more substituents independently selected from the group consisting of F, Cl, Br, I, NH₂, SH, OH, CF₃; an unsubstituted C₁₋₆ alkyl-aryl group; an unsubstituted C₁₋₆ alkyl-heteroaryl group; with the condition that R³ and R⁴ may not at the same time represent a hydrogen atom;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, CI, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

Also preferred is a medicament comprising at least one substituted pyrazoline compound of general formula (I) as defined above,
wherein
R¹ represents an unbranched or branched, saturated, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ and R⁴ together with their bridging nitrogen atom form
   1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
   2) an A-X-B group,
      wherein
      A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   3) an A-X-B-Y-C group,
   wherein
   A represents a 4 to 18 membered ring system in which the ring containing the nitrogen atom is saturated, but may be condensed with an unsaturated ring comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
   X represents a bond, a -NH- group, a -(C=O)- group, a branched or linear, optionally substituted C₁₋₆-aliphatic chain, -O-, or a -SO₂- group;
   B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
   Y represents a bond, a -NH- group, a -(C=O)- group, a branched or linear, optionally substituted C₁₋₆-aliphatic chain, -O-, or a -SO₂- group;
   C represents a saturated or unsaturated 4 to 6membered ring system, in which at least 1 carbon atom is optionally replaced by N or O and which is optionally at least mono-substituted by F, Cl, Br, I, NH₂, SH, OH, oxo, or CF₃;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;

Also preferred is a medicament comprising at least one substituted pyrazoline compound of general formula (I) as defined above,
wherein
R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted alkyl-aryl group; an unsubstituted C₁₋₆ alkyl-heteroaryl group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, CI, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, CI, Br, I, NH₂, SH, OH, CF₃;

Also preferred is a medicament comprising at least one substituted pyrazoline compound of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a hydrogen atom; a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN; with the condition that R³ and R⁴ may not at the same time represent a hydrogen atom;
   or
   R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a saturated or unsaturated 4 to 18 membered ring system comprising 1 to 3 optionally condensed rings, in which at least 1 carbon atom is optionally replaced by N or 0 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C_{1- 6}-alkoxy group; a (C=O)-OR¹⁰ group with R¹⁰ being a linear or branched C₁₋₆ alkyl group;

Also preferred is a medicament comprising at least one substituted pyrazoline compound of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a saturated or unsaturated 4 to 18 membered ring system comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -S0₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
   3) an A-X-B-Y-C group,
      wherein
      A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;

Also preferred is a medicament comprising at least one substituted pyrazoline compound of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, optionally at least mono-substituted C₃₋₈ aliphatic group with substituents independently selected from the group consisting of F, Cl, Br, I, NH₂, SH, OH, CF₃, CN, nitro; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a saturated or unsaturated, optionally at least mono-substituted C₁₋₆ alkyl-cycloalkyl group; a saturated or unsaturated, unsubstituted C₁₋₆ alkyl-aryl group; a saturated or unsaturated, unsubstituted C₁₋₆ alkyl-heteroaryl group; with the condition that R³ and R⁴ may not at the same time represent a hydrogen atom;

Also preferred is a medicament comprising at least one substituted pyrazoline compound of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆-alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ and R⁴ together with their bridging nitrogen atom form
   1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, CI, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
   3) an A-X-B-Y-C group,
      wherein
      A represents a 4 to 18 membered ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
      X represents a bond, a -NH- group, a -(C=O)- group, a branched or linear, optionally substituted C₁₋₆-aliphatic chain, -O-, or a -SO₂- group;
      B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
      Y represents a bond, a -NH- group, a -(C=O)- group, a branched or linear, optionally substituted C₁₋₆-aliphatic chain, -O-, or a -SO₂- group;
      C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;

Also preferred is a medicament comprising at least one substituted pyrazoline compound of general formula (I) as defined above,
wherein
R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;

Also preferred is a medicament comprising at least one substituted pyrazoline compound of general formula (I) as defined above,
wherein
R¹ represents a benzyl group or a (SO₂)-R⁶ group with R⁶ being an aryl group;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN;
   or
   R³ and R⁴ together with their bridging nitrogen atom form
   1) a ring system A, comprising: 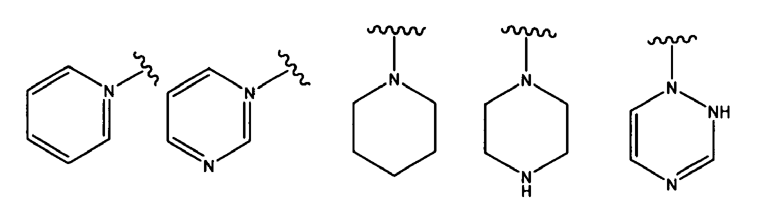 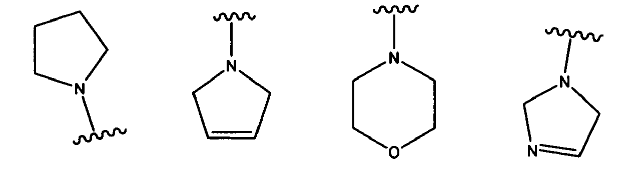 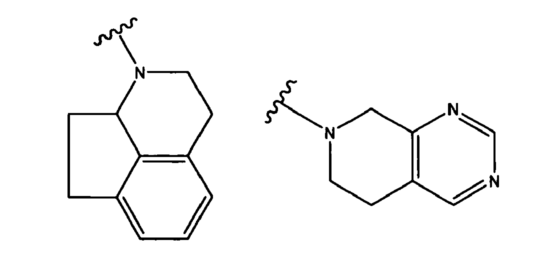 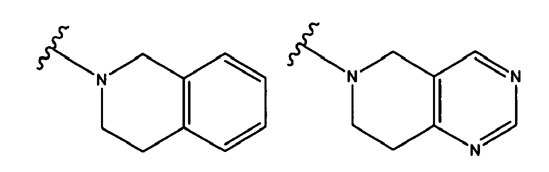 which is optionally at least mono-substituted by F, CI, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a ring system comprising: 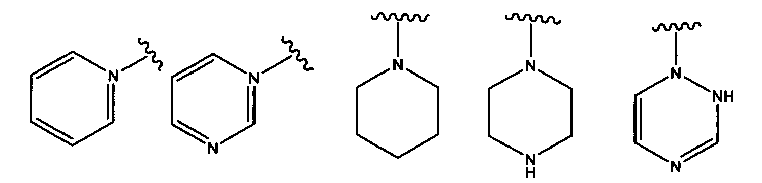 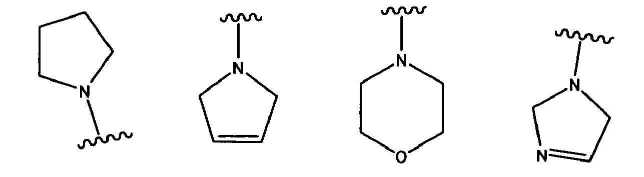 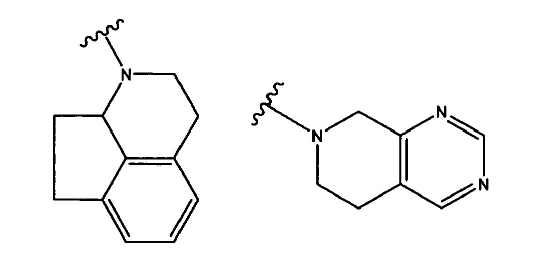 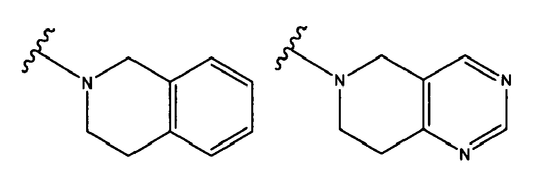 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear optionally substituted C₁₋₂ alkyl chain, -O-, or a -SO₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 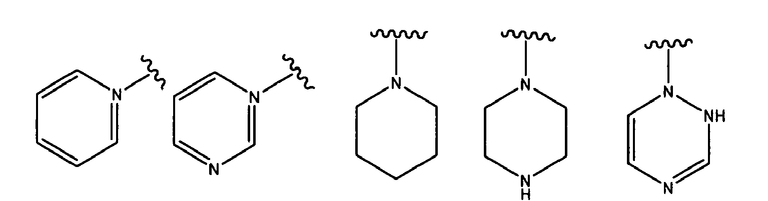 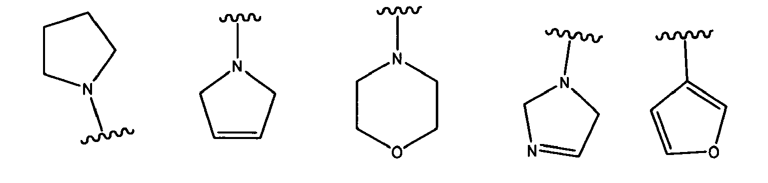 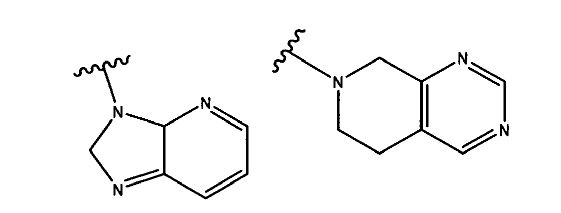 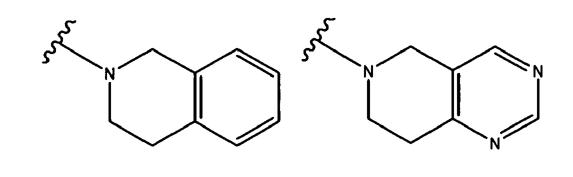 which is optionally at least mono-substituted by F, Cl, Br, I, NH₂, SH, OH, CF₃, a C₁₋₆-aliphatic group; a C₁₋₆-alkoxy group;
   3) an A-X-B-Y-C group,
      wherein
      A represents a ring system comprising: 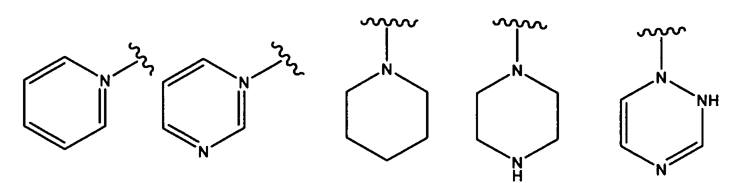 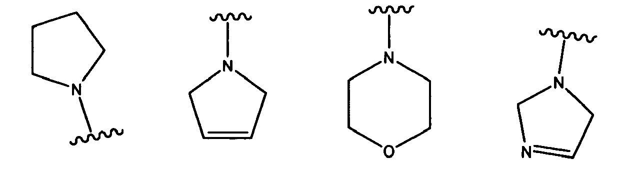 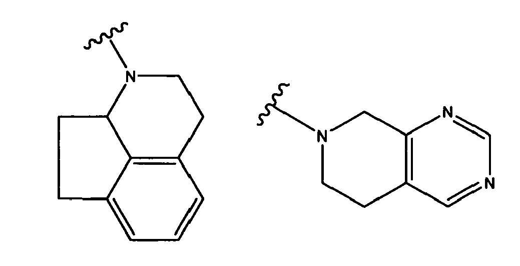 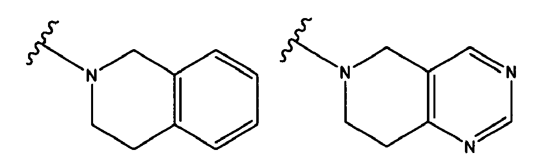 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear C₁₋₂ alkyl chain, -0-, or a -S0₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 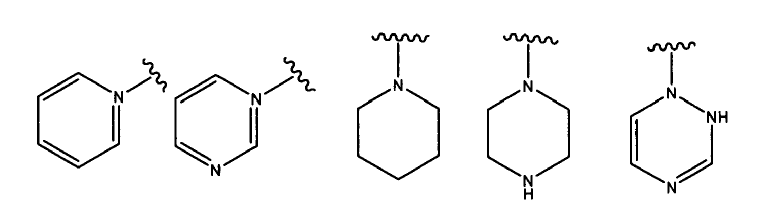 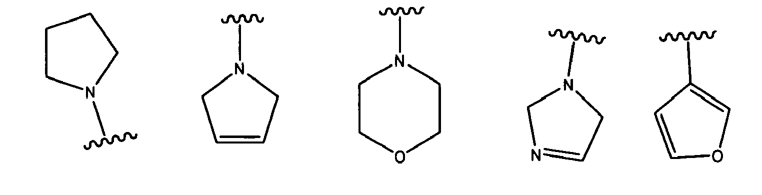 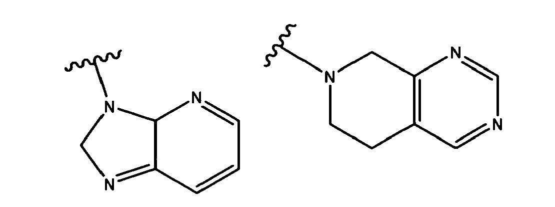 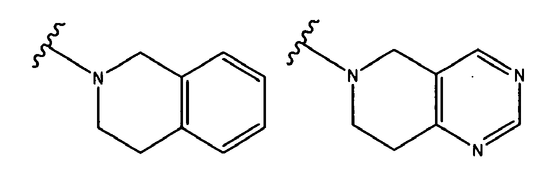 which is optionally at least mono-substituted by F, Cl, Br, I, NH₂, SH, OH, CF₃, a C₁₋₆-aliphatic group; a C₁₋₆-alkoxy group;
      Y represents a bond, a -NH- group, a linear C₁₋₂-aliphatic group;
      C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;

Also preferred is a medicament comprising at least one substituted pyrazoline compound of general formula (I) as defined above,
wherein
R¹ represents a benzyl group or a (SO₂)-R⁶ group with R⁶ being an aryl group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
   1) a ring system A, comprising: 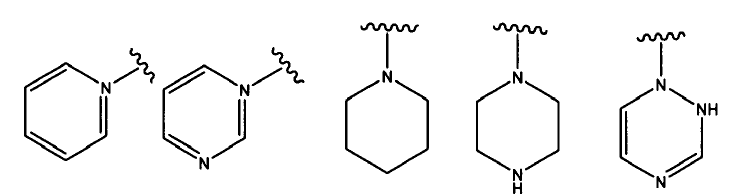 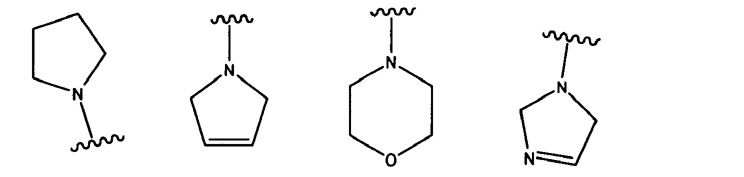 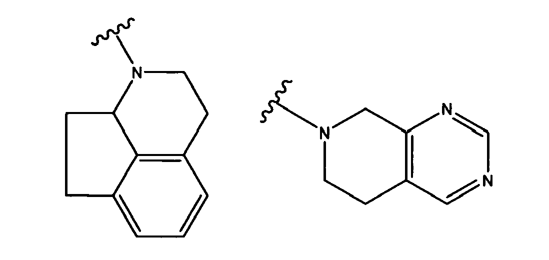 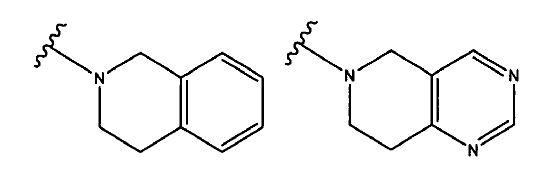 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a ring system comprising: 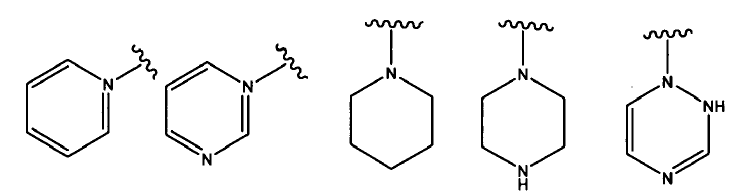 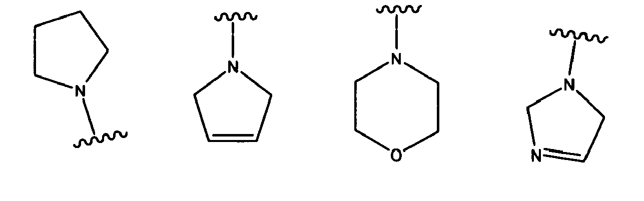 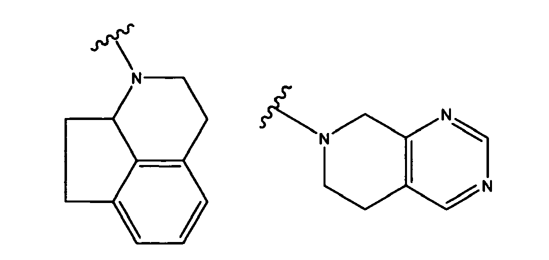 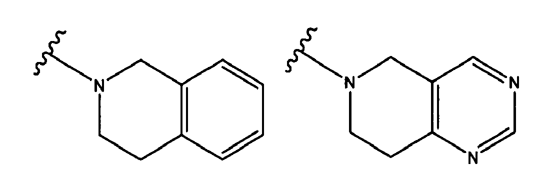 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear C₁₋₂ alkyl chain, -O-, or a -SO₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 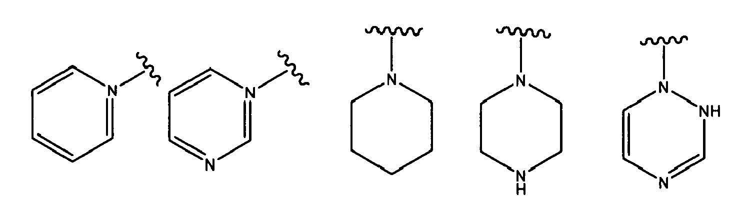 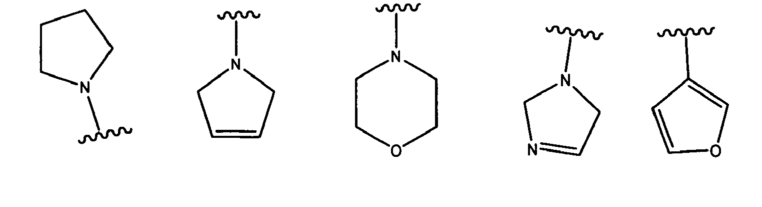 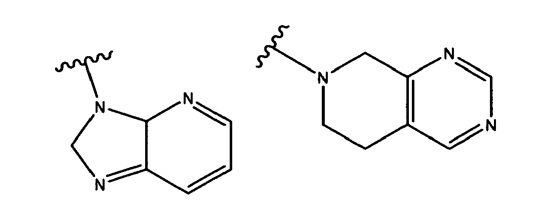 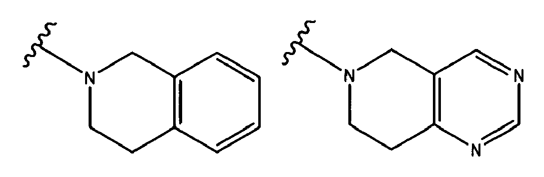 which is optionally at least mono-substituted by F, Cl, Br, I, NH₂, SH, OH, CF₃, a C₁₋₆-aliphatic group; a C₁₋₆-alkoxy group;
   3) an A-X-B-Y-C group,
      wherein
      A represents a ring system comprising: 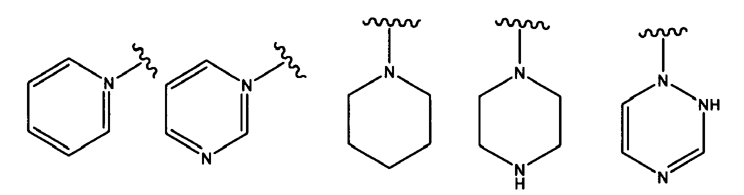 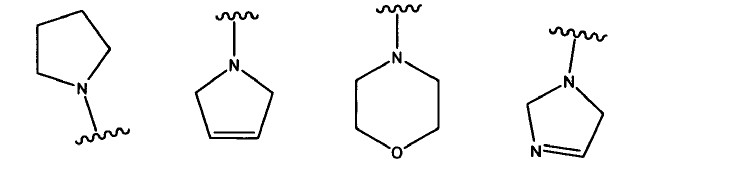 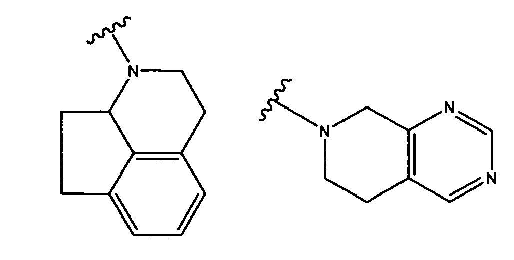 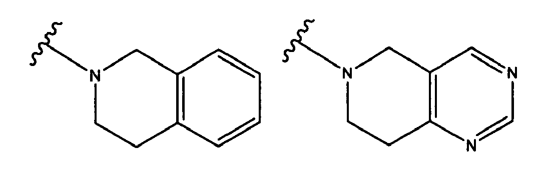 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear C₁₋₂ alkyl chain, -0-, or a -SO₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 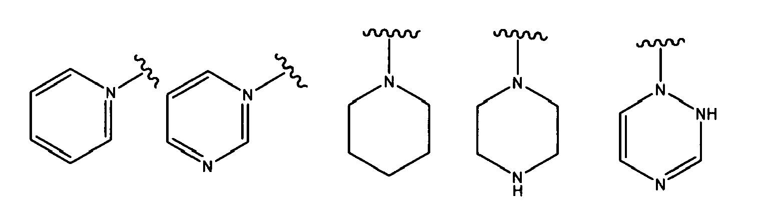 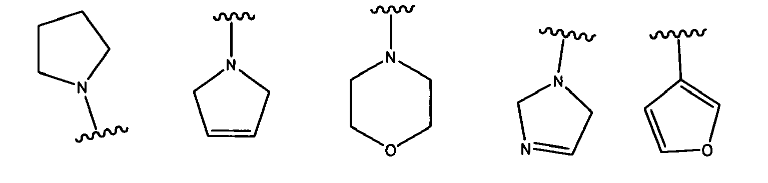 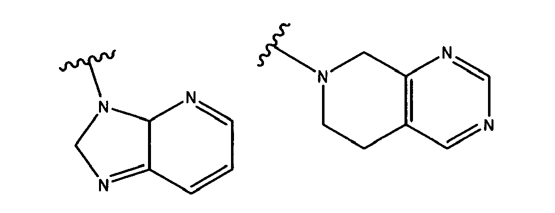 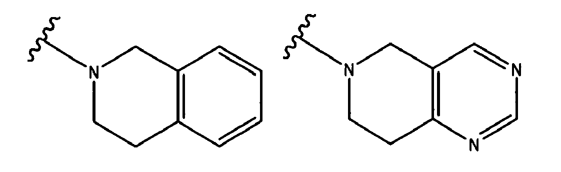 which is optionally at least mono-substituted by F, Cl, Br, I, NH₂, SH, OH, CF₃, a C₁₋₆-aliphatic group; a C₁₋₆-alkoxy group;
      Y represents a bond, a -NH- group, a linear C₁₋₂ alkyl group;
      C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;

Also preferred is a medicament comprising at least one substituted pyrazoline compound of general formula (I) as defined above,
wherein
R¹ represents a benzyl group or a (SO₂)-R⁶ group with R⁶ being an aryl group;
R² represents a hydrogen atom;
R³ and R⁴ together with their bridging nitrogen atom form
   1) a ring system A, comprising: 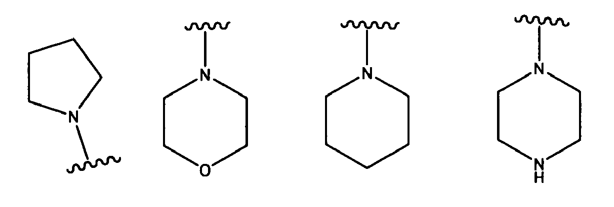 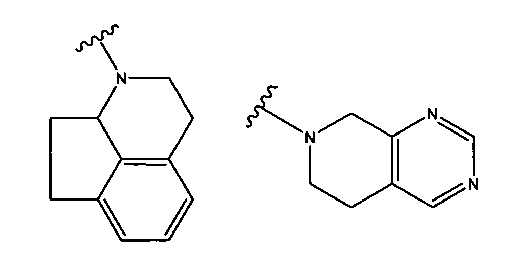 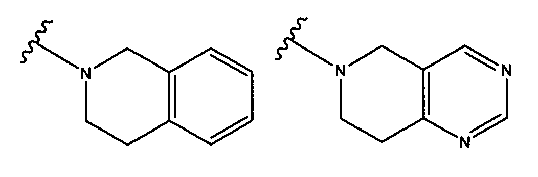 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
   2) an A-X-B group,
      wherein
      A represents a ring system comprising: 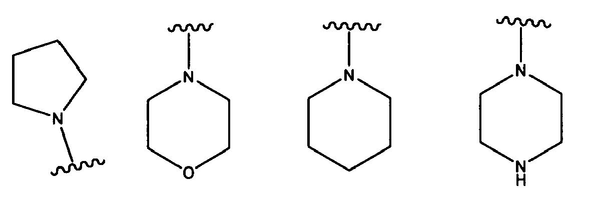 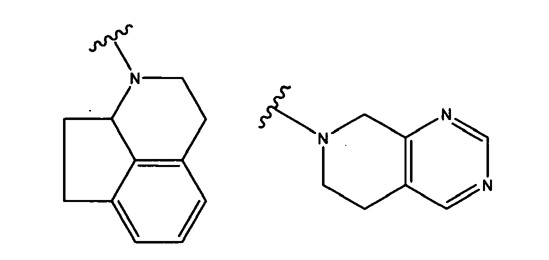 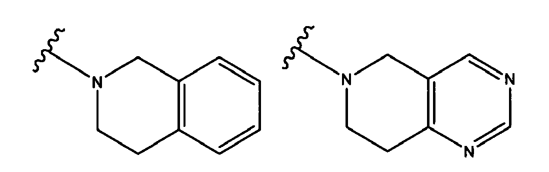 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear C₁₋₂ alkyl chain, -O-, or a -SO₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 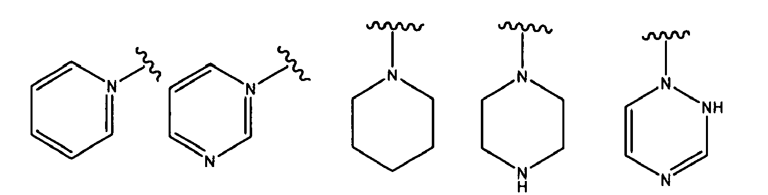 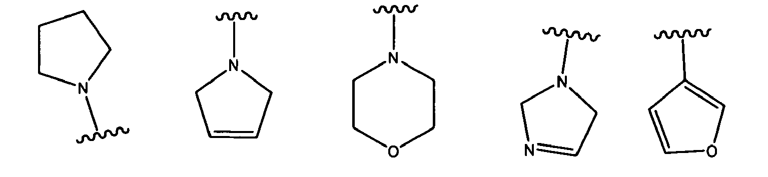 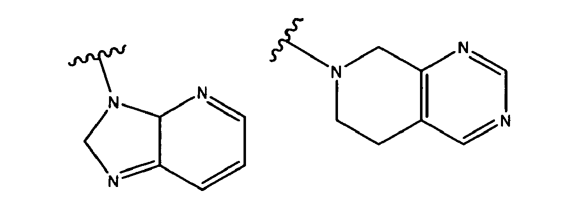 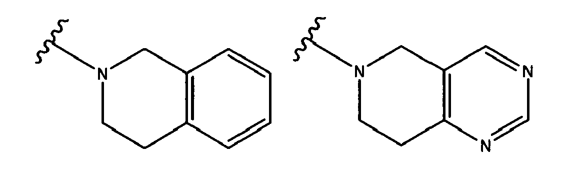 which is optionally at least mono-substituted by F, Cl, Br, I, NH₂, SH, OH, CF₃, a C₁₋₆-aliphatic group; a C₁₋₆-alkoxy group;
   3) an A-X-B-Y-C group,
      wherein
      A represents a ring system comprising: 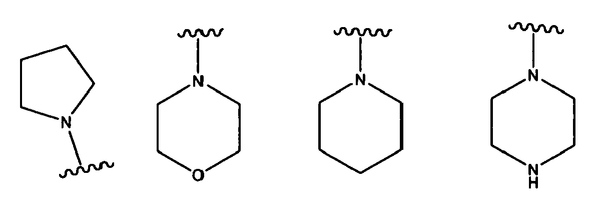 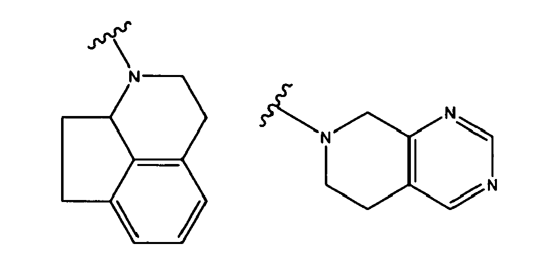 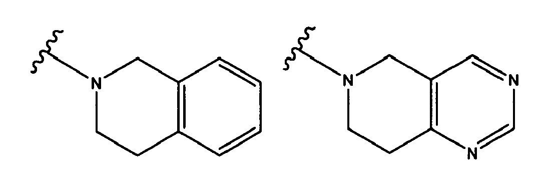 which is optionally at least mono-substituted by F, CI, Br, I, NH₂, SH, OH, CF₃; a C₁₋₆₋aliphatic group; a C₁₋₆-alkoxy group; a (C=O)-OR¹⁰ group with R¹⁰ being a linear or branched C₁₋₆ alkyl group;
      X represents a bond, a -NH- group, a -(C=O)- group, a linear C₁₋₂ alkyl chain, -O-, or a -SO₂- group;
      B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 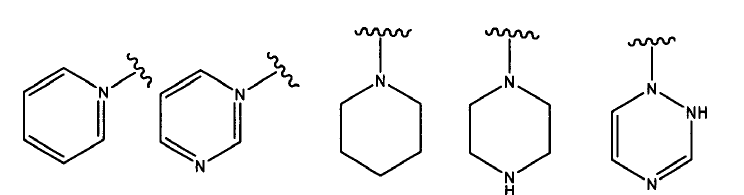 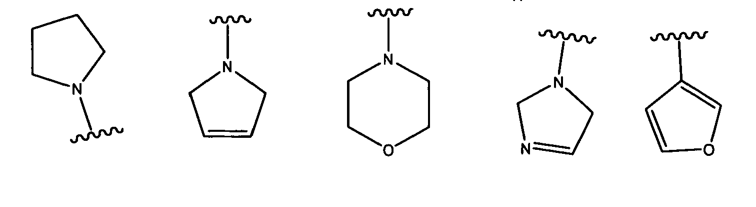 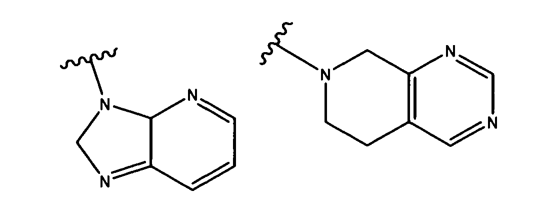 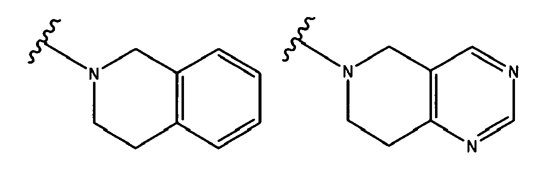 which is optionally at least mono-substituted by F, Cl, Br, I, NH₂, SH, OH, CF₃, a C₁₋₆-aliphatic group; a C₁₋₆ alkoxy group;
      Y represents a bond, a -NH- group, a linear C₁₋₂-alkyl group;
      C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;

Most particularly preferred is a medicament comprising at least one substituted pyrrolidine compound selected from the group consisting of:
[1] (3,4-trans)-1-benzyl-4-(3,4-dihydroisoquinolin-2(1H)-yl)pyrrolidine-3-ol,
[2] (3,4-trans)-1-benzyl-4-morpholinopyrrolidine-3-ol,
[3] (3,4-trans)-1-benzyl-4-(4-(2-methyl-3H-imidazo[4,5-b]pyridin-3-yl)piperidin-1-yl)pyrrolidine-3-ol,
[4] (3,4-trans)-1-benzyl-4-(3-(6,7-dimethoxy-3,4-dihydroisoquinolin-2(1H)-yl)propyl amino)pyrrolidine-3-ol,
[5] (3,4-trans)-1-benzyl-4-(4-((3,4-dihydroisoquinolin-2(1H)-yl)methyl)piperidin-1-yl)pyrrolidine-3-ol,
[6] 1-(4-(6-((3,4-trans)-1-benzyl-4-hydroxypyrrolidine-3-yl)-5,6,7,8-tetrahydropyrido [4,3-d]pyrimidin-2-ylamino)phenyl)ethanone,
[7] 1-(1-((3,4-trans)-1-benzyl-4-hydroxypyrrolidine-3-yl)piperidin-4-yl)-1H-benzo[d] [1,3] oxazin-2(4H)-one,
[8] (3,4-trans)-1-benzyl-4-(2-(4-phenoxyphenylamino)-7,8-dihydropyrido[4,3-d]pyrimidin-6(5H)-yl)pyrrolidine-3-ol,
[9] (3,4-trans)-1-benzyl-4-(3-(3,4-dihydroisoquino)in-2(1H)-yl)propylamino)pyrrolidine -3-ol,
[10](3,4-trans)-1-benzyl-4-(4-(4-methoxypyrimidin-2-y)piperazin-1-yl)pyrrolidine-3-ol,
[11] (3,4-trans)-1-benzyl-4-(2,3,8,8a-tetrahydrocyclopenta[ij]isoquinolin-1(7H)-yl) pyrrolidine-3-ol,
[12]tert-butyl-4-((3,4-trans)-1-benzyl-4-hydroxypyrrolidine-3-yl)piperazine-1-carboxylate,
[13]tert-butyl 4-((3,4-trans)-4-hydroxy-1-(phenylsulfonyl)pyrrolidine-3-yl)piperazine-1-carboxylate,
[14](3,4-trans)-4-morpholino-1-(phenylsulfonyl)pyrrolidine-3-ol,
[15] (3,4-trans)-4-(3,4-dihydroisoquinolin-2(1H)-yl)-1-(phenylsulfonyl)pyrrolidine-3-ol,
[16] (3,4-trans)-4-(3,4-dihydroisoquinolin-2(1H)-yl)-1((S)-1-phenylethyl)pyrrolidine-3-ol,
[17](3,4-trans)-1-benzyl-4-(4-benzylpiperazin-1-yl)pyrrolidine-3-ol,
[18] (3,4-trans)-1-benzyl-4-(4-phenylpiperazin-1-yl)pyrrolidine-3-ol,
[19] Ethyl 2-(4-((3,4-trans)-1-benzyl-4-hydroxypyrrolidine-3-yl)piperazin-1 - yl)acetate,
[20] (4-((3,4-trans)-1-benzyl-4-hydroxypyrrolidine-3-yl)piperazin-1-yl)(phenyl) methanone,
[21] Ethyl 4-((3,4-trans)-1-benzyl-4-hydroxypyrrolidine-3-yl)piperazine-1-carboxilate,
[22]tert-butyl 4-((3,4-trans)-1-(4-fluorobenzyl)-4-hydroxypyrrolidine-3-yl)piperazine-1-carboxylate,
[23] (3,4-trans)-1-benzyl-4-(4-methylpiperazin-1-yl)pyrrolidine-3-ol,
[24]tert-butyl-4-(3,4-trans)-4-acetoxy-1-benzylpyrrolidine-3-yl)piperazine-1-carboxylate,
[25](3,4-trans)-1-benzyl-4-(piperazin-1-yl)pyrrolidine-3-yl acetate,
[26] tert-butyl 4-((3,4-trans)-4-acetoxypyrrolidine-3-yl)piperazine-l-carboxylate,
[27]trans 1-Benzyl-4-(4-(2-phenyl-phenylsulfonyl)piperazin-1-yl)pyrrolidin-3-yl acetate,
[28]trans 1-Benzyl-4-(4-(2-phenyl-phenylsulfonyl)piperazin-1-yl)pyrrolidin-3-ol dihydrochloride,
[29] (3,4-trans)-1-benzyl-4-(phenylamino)pyrrolidin-3-ol,
[30] (3,4-trans)-1-benzyl-4-(benzylamino)pyrrolidin-3-ol,
[31] (3R,4R)-1-benzyl-4-(octylamino)pyrrolidin-3-ol,
[32](3,4-trans)-1-benzyl-4-(4-methoxybenzylamino)pyrrolidin-3-ol,
[33](3,4-trans)-1-benzyl-4-(butylamino)pyrrolidin-3-ol,
[34] (3R,4R)-1-benzyl-4-(4-(4-chloro-2,5-dimethylphenylsulfonyl)piperazin-1-yl)pyrrolidin-3-yl acetate,
[35](3R,4R)-1-benzyl-4-(4-tosylpiperazin-1-yl)pyrrolidin-3-yl acetate,
[36](3,4-trans)-1-benzyl-4-(butylamino)pyrrolidin-3-ol,
[37] (3R,4R)-1-benzyl-4-(4-methoxyphenylamino)pyrrolidin-3-ol,
[38] Methyl 4-((3,4-trans)-1-benzyl-4-hydroxypyrrolidin-3-ylamino)benzoate,
[39](3R,4R)-1-benzyl-4-(4-(naphthalen-1-ylsulfonyl)piperazin-1-yl)pyrrolidin-3-yl acetate,
[40](3R,4R)-1-benzyl-4-(4-(2-chlorophenylsulfonyl)piperazin-1-yl)pyrrolidin-3-ol,
[41](3R,4R)-1-benzyl-4-(4-(2-bromophenylsulfonyl)piperazin-1-yl)pyrrolidin-3-yl acetate,
[42](3R,4R)-1-benzyl-4-(4-(2-bromophenylsulfonyl)piperazin-1-yl)pyrrolidin-3-ol,
[43] (3R,4R)-1-benzyl-4-(4-(5-fluoro-2-methylphenylsulfonyl)piperazin-1-yl)pyrrolidin-3-yl acetate,
[44] (3R,4R)-1-benzyl-4-(4-(5-fluoro-2-methylphenylsulfonyl)piperazin-1-yl)pyrrolidin-3-ol,
[45](3R,4R)-1-benzyl-4-(4-(phenylsulfonyl)piperazin-1-yl)pyrrolidin-3-yl acetate,
[46] (3R,4R)-1-benzyl-4-(4-(phenylsulfonyl)piperazin-1-yl)pyrrolidin-3-ol,
[47] (3R,4R)-1-benzyl-4-(4-(2-fluorophenylsulfonyl)piperazin-1-yl)pyrrolidin-3-yl acetate,
[48] (3R,4R)-1-benzyl-4-(4-(2-fluorophenylsulfonyl)piperazin-1-yl)pyrrolidin-3-ol,
[49](3R,4R)-1-benzyl-4-(4-(naphthalen-1-ylsulfonyl)piperazin-1-yl)pyrrolidin-3-ol,
[50] (3R,4R)-1-benzyl-4-(4-(4-chloro-2,5-dimethylphenylsulfonyl)piperazin-1-yl)pyrrolidin-3-ol,
[51] (3R,4R)-1-benzyl-4-(4-tosylpiperazin-1-yl)pyrrolidin-3-ol,
[52] (3R,4R)-1-benzyl-4-(4-(2-chlorophenylsulfonyl)piperazin-1-yl)pyrrolidin-3-yl acetate,
optionally in form of a corresponding salt, or a corresponding solvate.

In an embodiment according to the invention the medicament is for the prophylaxis and/or treatment of a sigma receptor-mediated disease or condition.

In an embodiment according to the invention the medicament is for the prophylaxis and/or treatment of Alzheimer's disease (AD).

In an embodiment according to the invention the medicament is for the prophylaxis and/or treatment of one or more disorders selected from the group consisting of diarrhoea, lipoprotein disorders, migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety, psychosis or schizophrenia; inflammation, or autoimmune diseases.

In an embodiment according to the invention the medicament is for the prophylaxis and/or treatment of one or more disorders selected from the group consisting of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

In another embodiment according to the invention the medicament is for the prophylaxis and/or treatment of one or more disorders selected from the group consisting of pain, preferably neuropathic pain, inflammatory pain or other pain conditions involving allodynia and/or hyperalgesia.

Said medicaments may also comprise any combination of one or more of the substituted pyrrolidine compounds of general formula (I) given above, stereoisomers thereof, physiologically acceptable salts thereof or physiologically acceptable solvates thereof.

Another aspect of the present invention is the use of at least one substituted pyrazoline compound of general formula (I) given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, and optionally one or more pharmaceutically acceptable excipients, for the preparation of a medicament for the modulation of sigma receptors, preferably for the prophylaxis and/or treatment of Alzheimer's disease (AD).

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Solid oral compositions (which are preferred as are liquid ones) may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to the methods well known in normal pharmaceutical practice., in particular with an enteric coating.

The mentioned formulations will be prepared using standard methods such as those described or referred to in the Spanish and US Pharmacopeias and similar reference texts.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered forms suitable for reconstitution with water or other suitable liquid medium before use, for immediate or retarded release.

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Another aspect of the present invention refers to a method for the prophylaxis and/or treatment of a sigma receptor-mediated disease or condition, the method comprising administering to the subject at least one substituted pyrrolidine compound of general formula (I) as described above and optionally at least one further active substance and/or optionally at least one auxiliary substance to the subject.

Another aspect of the present invention refers to a method for the prophylaxis and/or treatment Alzheimer's disease, the method comprising administering to the subject at least one substituted pyrrolidine compound of general formula (I) as described above and optionally at least one further active substance and/or optionally at least one auxiliary substance to the subject.

Another aspect of the present invention refers to a method for the prophylaxis and/or treatment of diarrhoea, lipoprotein disorders, migraine, obesity, elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and dysbetalipoproteinemia, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety or schizophrenia; inflammation, or autoimmune diseases, the method comprising administering to the subject at least one compound of general formula (I) as described above and optionally at least one further active substance and/or optionally at least one auxiliary substance to the subject.

A preferred embodiment of the present invention refers to a method for the prophylaxis and/or treatment of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

The present invention is illustrated below with the aid of examples. These illustrations are given solely by way of example and do not limit the general spirit of the present invention.

### Examples:

### Obtention of intermediates

### Example 0-A: 1-Benryl-3-pyrroline

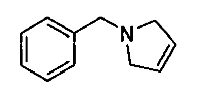

Was synthesized according to published methods with slight modifications: a) EP0985664, b) Synthetic Communications 13(13), 1117-1123 (1983).
To a solution of cis-1,4-dichloro-2-butene (0.76 g, 5.77 mmol) in anhydrous dichloromethane (4 ml) cooled at 5°C, benzylamine (3.75 g, 34.66 mol) was added dropwise. The mixture was stirred at 5°C for 10 min and after at r.t. 24 hours. The white solid was filtered and washed with dichloromethane. The filtrated was cooled at 0°C and HCl 37% (0.6 ml) was added. The resulting white solid was filtered and washed with dichloromethane. The filtrate was concentrated to give an orange oil that was purified by flash chromatography: silica gel, hexane:ethyl acetate (1:1) to afforded the product (0.76 g, 82%) as yellow oil.
¹**H NMR** (400 MHz, CDCI3): δ (ppm) 7.38-7.21 (m 5H), 5.78 (s, 2H), 3.80 (s, 3H), 3.81 (s, 2H), 3.48 (s, 4H). **¹³C NMR** (75 MHz, CDCl₃) δ (ppm) 139.67, 128.69, 128.34, 127.78, 126.96, 60.40, 59.70.

### Example 0-B:1-((S)-1-phenylethyl)-3-pyrroline

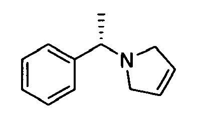

Was synthesized according to published methods with slight modifications: a) EP0985664, b) Synthetic Communications 34(23), 4421-4430 (2004).
To a solution of 1,4-dimethylsulphonyl-2-butene (0.52 g, 2.86 mmol) in dichloromethane (10 ml), (S)-alpha-methylbenzylamine (1.31 g, 10.87 mmol) was added and the solution was stirred 20 hours at r.t. The solid obtained was filtrated and the filtrate was washed with water (2x30ml), dried over Na₂SO₄, filtered and concentrated. The crude was purified by flash chromatography: silica gel, gradient hexane to hexane:ethyl acetate 1:1 to afforded the product (0.45 g, 90%) as yellow oil.
**¹H NMR** (400 MHz, CDCl3): δ (ppm) 7.42-7.25 (m, 5H), 5.80 (s, 2H), 3.53 (q, J=6.5Hz, 1H), 3.44 (m, 2H), 3.36 (m, 2H), 1.44 (d, J=6.5 Hz, 3H). **¹³C NMR** (75 MHz, CDCl₃) δ (ppm) 145.90, 128.47, 127.67, 127.28, 127.07, 65.30, 58.55, 23.50.

### Example 0-C: 1-tert-Butoxycarbonyl-3-pyrroline

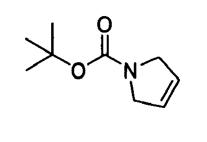

Was synthesized according to published methods: a) JP11349565. b) Tetrahedron: Asymmetry 12 (2001) 2989-2997. c) Organic & Biomolecular Chemistry 2004, 2 (17), 2418-2420.
To a solution of *tert*-butyl carbamate (0.55 g, 4.65 mmol) in DMF (5 ml) cooled at 0°C, NaH (0.41 g, 10.23 mmol) was added in portions. The mixture was stirred at 0°C for 10 min. Cis-1,4-dichloro-2-butene (0.73 g, 5.58 mmol) was added and the mixture was stirred for 15 min at r.t. and then heated at 65°C for 20 h. Water was added and the solution was extracted with hexane:ethyl acetate (4:1). The organic phase was washed with water and saturated solution of NaCl, dried over Na₂SO₄, filtered and concentrated to afford pure product (0.40 g, 51%) as yellow oil.
**¹H NMR** (400 MHz, CDCl3): δ (ppm) 5.70 (m 2H), 4.15 (m, 4H), 1.48 (s, 9H).

### Example 0-D: 1-Phenylsulfonyl-3-pyrroline

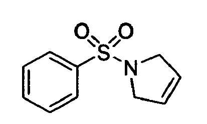

Was synthesized according to published methods: JP2000080077.
To a solution of benzenesulfonamide (0.25 g, 1.55 mmol) in DMF (4 ml), NaH (140 mg, 3.51 mmol) was added in portions and the solution was stirred 10 min at r.t. Cis-1,4-dichloro-2-butene (0.22 g, 1.71 mmol) was added and the mixture was stirred for 20 h. at r.t. Water was added and the solution was extracted with hexane:ethyl acetate (4:1). The organic phase was washed with water and saturated solution of NaCI, dried over Na₂SO₄, filtered and concentrated to afford pure product (0.32 g, quant. yield) as white solid.
**¹H NMR** (400 MHz, CDCI3): δ (ppm) 7.84 (m, 2H), 7.59 (m, 1H), 7.52 (m, 2H).

### Example 0-E: 1-(-fluorobenzyl)-3-pyrroline

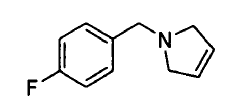

To a solution of cis-1,4-dichloro-2-butene (0.50 g, 3.8 mmol) in anhydrous dichloromethane (4 ml) cooled at 0°C, 4-fluorobenzylamine (2.94 g, 22.8 mol) was added dropwise. The mixture was stirred at 0°C for 10 min and then at r.t. 24 hours. The white solid was filtered and washed with dichloromethane. The filtrated was cooled at 0°C and HCl 10% was added until slightly acid pH. The resulting white solid was filtered and washed with dichloromethane. The filtrate was concentrated and the residue was purified by flash chromatography: silica gel, hexane:ethyl acetate (1:1) to afforded the product (353 m g, 52%) as yellow oil.
**¹H NMR** (400 MHz, CDCl3): δ (ppm) 7.31 (m, 2H), 6.99 (m, 2H), 5.78 (s, 2H), 3.77 (s, 2H), 3.46 (s, 4H). **¹³C NMR** (75 MHz, CDCl₃) δ (ppm) 161.91 (d, J_{cF}=243Hz), 135.41, 130.14 (d, J_{CF}=8Hz), 127.78 , 115.05 (d, J_{CF}=21Hz), 59.59, 59.52. **MS (ES+**) m/z: 178.1 (M+H⁺).

### Example 0-F: 1-Benzyl-3,4-epoxypyrroline

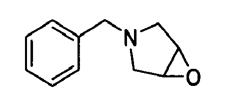

Was synthesized according to published methods: EP0985664.
To a solution of 1-benzyl-3-pyrroline (5.0 g, 31.40 mmol) in methanol (20 ml) cooled at 0°C, water was added (5 ml) and H₂S0₄ 96% (2 ml). The solution was stirred 5 min. and 3-chloroperoxybenzoic acid (10.0 g, 40.56 mmol) was added in portions. The suspension was stirred at r.t. for 18 h. Methanol was evaporated and the aquose solution was neutralized with aq. NaOH 10 % until pH=7. The suspension was extracted with dichloromethane and the organic phase was washed with water and saturated solution of NaCl, dried over Na₂SO₄, filtered and concentrated to afford pure product (4.35 g, 80%) as yellow oil.
**¹H NMR** (400 MHz, CDCl3): δ (ppm) 7.49-7.23 (m, 5H), 3.81 (s, 2H), 3.66 (s, 2H), 3.23 (d, J=12Hz, 2H), 2.72 (d, J=12Hz, 2H).

### Example 0-G: 1-((S)-1-phenylethyl)-3,4-epoxypyrroline

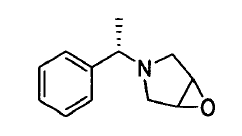

Was synthesized according to published methods with slight modifications: a) EP0985664, b) Synthetic Communications 34 (23), 4421-4430, 2004.
To a solution of 1-((S)-alpha-methylbenzyl)-3-pyn-oline (0.10 g, 0.57 mmol) in methanol (0.35 ml) cooled at 0°C, water was added (0.046 ml) and H₂SO₄ 96% (0.038 ml). The solution was stirred 5 min. and 3-chloroperoxybenzoic acid (0.18 g, 0.75 mmol) was added in portions. The suspension was stirred at r.t. for 18 h. Methanol was evaporated and the aquose solution was neutralized with aq. NaOH 10 % until pH=7. The suspension was extracted with dichloromethane and the organic phase was washed with water and saturated solution of NaCl, dried over Na₂SO₄, filtered and concentrated to afford pure product (0.10 g, 91%) as colourless oil.
**¹H NMR** (400 MHz, CDCl3): δ (ppm) 7.32-7.20 (m, 5H), 3.63 (dd, J1=1.5, J2=3.5Hz, 1H), 3.54 (dd, J1=1.5, J2=3.5Hz, 1H), 3.42 (d, J=11Hz, 1H), 3.40 (q, J=6.5Hz, 1H), 3.01 (d, J=11Hz, 1H), 2.47 (dd, J1=1.5, J2=11Hz, 1H), 2.27 (dd, J1=1.5, J2=11Hz, 1H), 1.35 (d, J=6.5Hz, 3H).

### Example 0-H: 1-(tert-butoxycarbonyl)-3,4-epoxypyrroline.

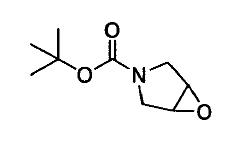

Was synthesized according to published methods: Organic & Biomolecular Chemistry 2004, 2 (17), 2418-2420.
To a solution of 1-*tert*-butoxycarbonyl-3-pyrroline (0.16 g, 0.94 mmol) in dichloromethane (3 ml), 3-chloroperoxybenzoic acid (0.46 g, 1.89 mmol) was added in portions. The suspension was stirred at r.t. for 20 h. Dichloromethane was evaporated and the resulting white solid was suspended in diethylether and washed with saturated solution of NaHCO₃, saturated solution of NaCl, dried over Na₂SO₄, filtered and concentrated. The crude was purified by flash chromatography: silica gel, hexane:ethyl acetate (2:1), to give the product (65 mg, 28%) as yellow oil.
**¹H NMR** (400 MHz, CDCl3): δ (ppm) 3.78 (d, J=12.7Hz, 1H), 3.71 (d, J1=12.7Hz, 1H), 3.63 (m, 2H), 3.30 (dd, J1=1.5Hz, J2=6.5Hz, 1H), 3.27 (dd, J1=1.5Hz, J2=6.5Hz, 1H), 1.41 (s, 9H).

### Example 0-I: 1-(phenylsulfonyl)-3,4-epoxypyrroline.

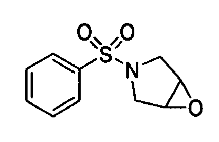

To a solution of 1-phenylsulfonyl-3-pyrroline (4.54 g, 21.69 mmol) in dichloromethane (100 ml), 3-chloroperoxybenzoic acid (10.7 g, 43.46 mmol) was added in portions. The suspension was stirred at r.t. for 16 h. Dichloromethane was added and the solution was washed with saturated solution of NaHCO₃, saturated solution of NaCl, dried over Na₂SO₄, filtered and concentrated. The crude was purified by flash chromatography: silica gel, gradient dichloromethane to dichloromethane:ethyl acetate (9:1), to give the product (3.52 g, 72%) as white solid.
**¹H NMR** (400 MHz, CDCl3): δ (ppm) 7.79 (m, 2H), 7.58 (m, 1H), 7.52 (m, 2H), 3.72 (d, J=12.7Hz, 1H), 3.58 (s, 2H), 3.38 (d, J1=12.7Hz, 2H).

### Example 0-J: 1-(4-fluorobenzyl)-3,4-epoxypyrroline

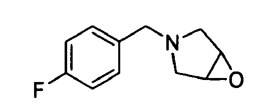

To a solution of 1-4-fluorobenzyl-3-pyrroline (105 mg, 0.59 mmol) in methanol (0.5 ml) cooled at 0°C, water was added (0.1 ml) and H₂SO₄ 96% (0.040 ml). The solution was stirred 5 min. and 3-chloroperoxybenzoic acid (173 m g, 0.77 mmol) was added in portions. The suspension was stirred at r.t. for 20 h. Methanol was evaporated and the aquose solution was neutralized with aq. NaOH 10 % until pH=7. The suspension was extracted with dichloromethane and the organic phase was washed with water and saturated solution of NaCl, dried over Na₂SO₄, filtered and concentrated. The residue was purified by flash chromatography: silica gel, gradient dichloromethane to dichloromethane:methanol 8% to afford pure product (71 mg, 62%) as yellow oil.
**¹H NMR** (400 MHz, CDCl3): δ (ppm) 7.26 (m, 2H), 6.98 (m, 2H), 3.67 (s, 2H), 3.62 (s, 2H), 3.16 (d, J=11Hz, 2H), 2.53 (d, J=11Hz, 2H). **MS (ES+)** m/z: 194.1 (M+H⁺).

### Example 1: (3,4-trans)-1-benzyl-4-(3,4-dihydroisoquinolin-2(1H)-yl)pyrrolidine-3-ol

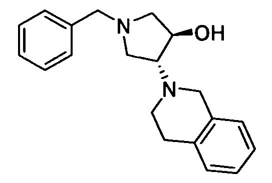

From 1-benzyl-3,4-epoxypyrroline (90 mg, 0.51 mmol), 1,2,3,4-tetrahydroisoquinoline (102 mg, 0.77 mmol), LiClO₄ (111 mg, 1.02 mmol) and acetonitrile (4 ml). Reaction time: 4 hours. Purification: silica gel, gradient dichloromethane to dichlorometane:methanol 5%, afforded the product (76 mg, 48%) as yellow oil.
**¹H NMR** (400 MHz, CDCl3): δ (ppm) 7.32 (m, 4H), 7.26 (m, 1H), 7.09 (m, 3H), 6.99 (m, 1H), 4.25 (m, 1H), 3.66 (AB system, 2H), 3.59 (AB system, 2H), 3.13 (t, J=8Hz, 1H), 2.94-2.81 (m, 4H), 2.79 (d, J=10 Hz, 1H), 2.72-2.61 (m, 2H), 2.60 (bs, 1H), 2.28 (t, J=8Hz, 1H). **¹³C NMR** (75 MHz, CDCl₃): δ (ppm) 137.93, 134.14, 128.86, 128.53, 128.24, 127.11, 126.53, 126.17, 125.58, 74.58, 74.07, 62.21, 60.17, 57.26, 54.56, 48.92, 28.75. **MS (El+)** m/z: 308.1 (M⁺).

### Example 2: (3,4-trans)-1-benzyl-4-morpholinopyrrolidine-3-ol

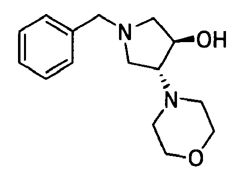

From 1-benzyl-3,4-epoxypyrroline (90 mg, 0.51 mmol), 1,2,3,4-tetrahydroisoquinoline (67 mg, 0.77 mmol), LiClO₄ (111 mg, 1.02 mmol) and acetonitrile (4 ml). Reaction time: 4 hours. Purification: silica gel, gradient dichloromethane to dichlorometane:methanol 5%, afforded the product (55 mg, 41 %) as yellow oil.
**¹H NMR** (400 MHz, CDCI3): δ (ppm) 7.28 (m, 5H), 4.15 (m, 1H), 3.71 (m, 4H), 3.57 (AB system, 2H), 3.02 (t, J=8Hz, 1 H), 2.77 (d, J=10 Hz, 1 H), 2.65 (m, 2H), 2.57 (m, 3H), 2.43 (m, 2H), 2.19 (t, J=8Hz, 1 H). **¹³C NMR** (75 MHz, CDCl₃): δ (ppm) 137.73, 128.85, 128.27, 127.19, 74.79, 74.04, 66.65, 62.21, 60.15, 56.92, 52.14. **MS (El+)** m/z: 262.1 (M⁺).

### Example 3: (3,4-trans)-1-benzyl-4-(4-(2-methyl-3H-imidazo[4,5-b]pyridin-3-yl)piperidin-1-yl)pyrrolidine-3-ol

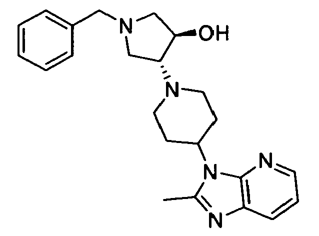

From 1-benzyl-3,4-epoxypyrroline (60 mg, 0.34 mmol), 2-methyl-3-(piperidin-4-yl)-3H-imidazo[4,5-b]pyridine (128 mg, 0.44 mmol), LiClO₄ (74 mg, 0.68 mmol) and acetonitrile (3 ml). Reaction time: 18 hours. Purification: silica gel, gradient dichloromethane to dichlorometane:2-propanol 30%, afforded the product (30 mg, 22%) as white solid.
**¹H NMR** (400 MHz, CDCl3): δ (ppm) 8.27 (dd, J₁=1.5Hz, J₂=4.5Hz, 1H), 7.89 (dd, J₁=1.5Hz, J₂=8Hz, 1H), 7.28 (m, 5H), 7.14 (dd, Jₗ=4.5Hz, J₂=8Hz, 1H), 4.42 (m, 1H), 4.25 (m, 1H), 3.59 (AB system, 2H), 3.35 (d, J=10Hz, 1H), 2.90-2.76 (m, 4H), 2.70 (m, 1H), 2.67 (s, 3H), 2.38-2.14 (m, 3H), 1.83 (m, 2H). **¹³C NMR** (75 MHz, CDCl₃): δ (ppm) 152.40, 148.21, 142.54, 137.85, 134.67, 128.87, 128.31, 127.21, 126.08, 117.73, 74.58, 74.33, 62.25, 60.18, 57.24, 53.93, 52.11, 51.16, 29.99, 15.78. **MS (EI+)** m/z: 392.2 (M+H⁺).

### Example 4: (3,4-trans)-1-benzyl-4-(3-(6,7-dimethoxy-3,4-dihydroisoquinolin-2(1 H)-yl)propyl amino)pyrrolidine-3-ol

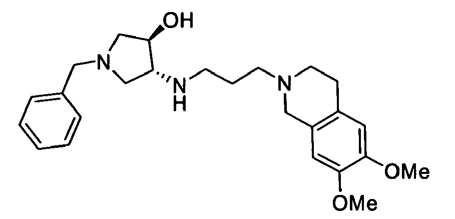

From 1-benzyl-3,4-epoxypyrroline (90 mg, 0.51 mmol), 3-(6,7-dimethoxy-3,4-dihydroisoquinolin-2(1H)-yl)propan-1-amine (153 mg, 0.61 mmol), LiClO₄ (111 mg, 1.02 mmol) and acetonitrile (6 ml). Reaction time: 5 hours. Purification: silica gel, gradient dichloromethane to dichlorometane:2-propanol 30%, afforded the product (100 mg, 45%) as colourless oil.
**¹H NMR** (400 MHz, CDCl3): δ (ppm) 7.28 (m, 5H), 6.58 (s, 1H), 6.50 (s, 1H), 3.95 (m, 1H), 3.83 (s, 3H), 3.82 (s, 3H), 3.56 (AB system, 2H), 3.53 (s, 2H), 3.07 (m, 1H), 3.01 (m, 1H), 2.80 (m, 2H), 2.69 (m, 5H), 2.59 (m, 1H), 2.54 (m, 2H), 2.14 (m, 1H), 1.76 (m, 2H). **¹³C NMR** (75 MHz, CDCl₃): δ (ppm) 147.49, 147.14, 138.35, 128.70, 128.18, 126.98, 126.08, 111.36, 109.43, 76.6, 66.91, 61.11, 60.10, 59.31, 56.37, 55.86, 55.69, 50.89, 46.84, 28.53, 27.28. **MS (El+)** m/z: 426.2 (M+H⁺).

### Example 5: (3,4-trans)-1-benzyl-4-(4-((3,4-dihydroisoquinolin-2(1 H)-yl)methyl)piperidin-1-yl)pyrrolidine-3-ol

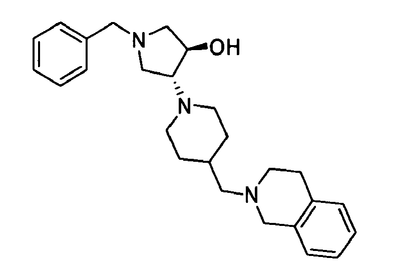

From 1-benzyl-3,4-epoxypyrroline (260 mg, 1.48 mmol), 2-(piperidin-4-ylmethyl)-1,2,3,4-tetrahydroisoquinoline (444 mg, 1.92 mmol), LiClO₄(317 mg, 2.92 mmol) and acetonitrile (20 ml). Reaction time: 6 hours. Purification: silica gel, gradient dichloromethane to dichlorometane:methanol 8%, afforded the product (430 mg, 71%) as yellow foam.
**¹H NMR** (400 MHz, CDCl3): δ (ppm) 7.30 (m, 5H), 7.12 (m, 3H), 7.02 (m, 1H), 4.25 (m, 1H), 3.61 (s, 2H), 3.58 (AB system, 2H), 3.20 (d, J=10Hz, 1H), 3.02 (t, J=8z, 1H), 2.90 (m, 3H), 2.80-2.65 (m, 5H), 2.36 (d, J=7Hz, 2H), 2.29 (t, J=9Hz, 1H), 2.09 (m, 2H), 1.85 (m, 2H), 1.67 (m, 1H), 1.35 (m, 2H). ¹³**C NMR** (75 MHz, CDCl₃): δ (ppm) 137.87, 134.84, 134.32, 128.79, 128.52, 128.19, 127.07, 126.42, 125.94, 125.44, 74.77, 74.01, 64.22, 62.03, 60.06, 56.60, 52.52, 51.41, 51.17, 33.21, 30.29, 28.98. **MS (El+)** m/z: 406.2 (M+H⁺).

### Example 6: 1-(4-(6-((3,4-trans)-1-benzyl-4-hydroxypyrrolidine-3-yl)-5,6,7,8-tetrahydropyrido [4,3-d]pyrimidin-2-ylamino)phenyl)ethanone

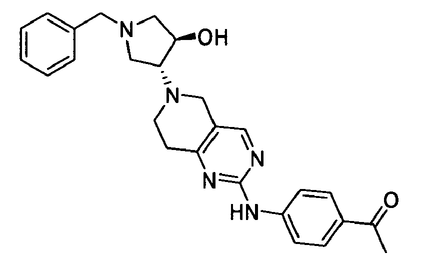

From 1-benzyl-3,4-epoxypyrroline (125 mg, 0.71 mmol), 1-(4-(5,6,7,8-tetrahydropyrido[4,3-d]pyrimidin-2-ylamino)phenyl)ethanone (140 mg, 0.52 mmol), LiClO₄ (151 mg, 1.42 mmol) and acetonitrile (5 ml). Reaction time: 6 hours. Purification: silica gel, gradient dichloromethane to dichlorometane:methanol 8%, afforded the product (180 mg, 62%) as yellow solid.
**¹H NMR** (400 MHz, CDCl3): δ (ppm) 8.11 (s, 1H), 7.93 (d, J=8.5Hz, 2H), 7.82 (s, 1H), 7.71 (d, J=8.5Hz, 2H), 7.30 (m, 5H), 4.27 (m, 1H), 3.87 (bs, 1H), 3.62 (AB system, 2H), 3.85 (AB system, 2H), 3.12 (t, J=8Hz, 1H), 2.92 (m, 4H), 2.81 (m, 1H), 2.72 (m, 2H), 2.55 (s, 3H), 2.33 (t, J=8Hz, 1H). **¹³C NMR** (75 MHz, CDCl₃): δ (ppm) 196.78, 164.54, 158.04, 155.47, 144.53, 137.63, 130.63, 129.84, 128.95, 128.26, 127.28, 119.88, 117.42, 74.68, 73.68, 62.27, 60.12, 57.21, 50.78, 48.10, 31.71, 26.27. **MS (EI+)** m/z: 444.2 (M+H⁺).

### Example 7: 1-(1-((3,4-trans)-1-benzyl-4-hydroxypyrrolidine-3-yl)piperidin-4-yl)-1H-benzo[d] [1,3] oxazin-2(4H)-one

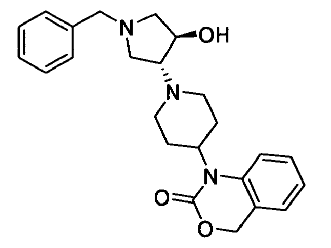

From 1-benzyl-3,4-epoxypyrroline (787 mg, 4.49 mmol), 1-(piperidin-4-yl)-1H-benzo[d][1,3]oxazin-2(4H)-one (820 mg, 3.53 mmol), LiClO₄ (947 mg, 8.90 mmol) and acetonitrile (20 ml). Reaction time: 6 hours. Purification: silica gel, gradient dichloromethane to dichlorometane:methanol 5%, afforded the product (1.2 g, 83%) as white solid.
**¹H NMR** (400 MHz, CDCl3): δ (ppm) 7.31 (m, 5H), 7.27 (m, 1H), 7.14 (d, J=7.5Hz, 2H), 7.07 (t, J=7.5Hz, 1H), 5.06 (s, 2H), 4.20 (m, 1H), 3.96 (m, 1H), 3.59 (AB system, 2H), 3.29 (m, 1H), 3.04 (t, J=8Hz, 1H), 2.95 (m, 1H), 2.80-2.63 (m, 5H), 2.33 (bs, 1H), 2.25 (t, 8Hz, 1H), 2.16 (m, 2H), 1.82 (m, 2H).
**¹³C NMR** (75 MHz, CDCl₃): δ (ppm) 152.97, 138.01, 129.00, 128.89, 128.30, 127.19, 124.70, 123.06, 122.62, 114.31, 74.66, 74.40, 67.02, 62.24, 60.20, 57.24, 56.80, 52.35, 51.41, 28.16. **MS (EI+)** m/z: 408.2 (M+H⁺).

### Example 8: (3,4-trans)-1-benzyl-4-(2-(4-phenoxyphenylamino)-7,8-dihydropyrido[4,3-d]pyrimidin-6(5H)-yl)pyrrolidine-3-ol

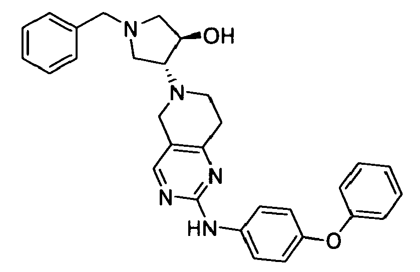

From 1-benzyl-3,4-epoxypyrroline (41 mg, 0.23 mmol), N-(4-phenoxyphenyl)-5,6,7,8-tetrahydropyrido[4,3-d]pyrimidin-2-amine (82 mg, 0.25 mmol), LiClO₄ (50 mg, 0.46 mmol) and acetonitrile (6 ml). Reaction time: 6 hours. Purification: silica gel, gradient dichloromethane to dichlorometane:methanol 5%, afforded the product (78 mg, 68%) as yellow oil.
**¹H NMR** (400 MHz, CDCl3): δ (ppm) 8.07 (s, 1H), 7.56 (d, J=9Hz, 2H), 7.31 (m, 7H), 7.18 (s, 1H), 7.06 (t, J=7.5Hz, 1H), 6.99 (d, J=9Hz, 4H), 4.26 (m, 1H), 3.62 (AB system, 2H), 3.56 (AB system, 2H), 3.14 (t, J=8Hz, 1H), 3.07 (bs, 1H), 3.00-2.79 (m, 5H), 2.72 (m, 2H), 2.31 (t, 8Hz, 1 H). **¹³C NMR** (75 MHz, CDCl₃): δ (ppm) 164.47, 158.87, 158.05, 155.71, 151.79, 137.68, 135.54, 129.63, 128.92, 128.41, 127.34, 122.75, 120.72, 119.90, 118.58, 118.12, 74.95, 73.95, 62.30, 60.23, 57.51, 50.83, 48.41, 31.86. **MS (EI+)** m/z: 494.2 (M+H⁺).

### Example 9: (3,4-trans)-1-benzyl-4-(3-(3,4-dihydroisoquinolin-2(1 H)-yl)propylamino)pyrrolidine -3-ol

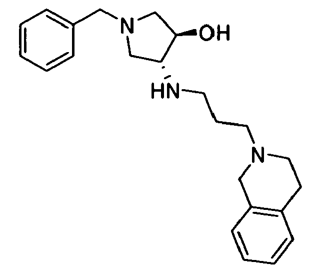

From 1-benzyl-3,4-epoxypyrroline (253 mg, 1.44 mmol), 3-(3,4-dihydroisoquinolin-2(1H)-yl)propan-1-amine (302 mg, 1.58 mmol), LiClO₄ (307 mg, 2.88 mmol) and acetonitrile (12 ml). Reaction time: 6 hours. Purification: silica gel, gradient dichloromethane to dichlorometane:methanol 10%, afforded the product (260 mg, 49%) as yellow oil.
**¹H NMR** (400 MHz, CDCl3): δ (ppm) 7.27 (m, 5H), 7.10 (s, 3H), 7.00 (m, 1H), 3.94 (m, 1H), 3.61 (s, 2H), 3.55 (AB system, 2H), 3.06 (m, 1H), 3.01 (m, 1H), 2.88 (t, J=6Hz, 2H), 2.70 (m, 5H), 2.56 (m, 3H), 2.37 (bs, 1H), 2.14 (m, 1H), 1.77 (m, 2H). **¹³C NMR** (75 MHz, CDCl₃): δ (ppm) 138.46, 134.72, 134.30, 128.90, 128.65, 128.25, 127.06, 126.59, 126.16, 125.63, 76.7, 67.00, 61.18, 60.23, 59.23, 56.63, 56.27, 50.89, 46.99, 29.14, 27.25. **MS (El+)** m/z: 366.2 (M+H⁺).

### Example 10: (3,4-trans)-1-benzyl-4-(4-(4-methoxypyrimidin-2-yl)piperazin-1-yl)pyrrolidine-3-ol

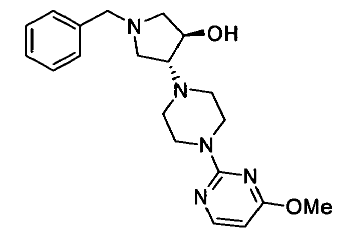

From 1-benzyl-3,4-epoxypyrroline (284 mg, 1.62 mmol), 4-methoxy-2-(piperazin-1-yl)pyrimidine (346 mg, 1.78 mmol), LiClO₄ (345 mg, 3.24 mmol) and acetonitrile (18 ml). Reaction time: 6 hours. Purification: silica gel, gradient dichloromethane to dichlorometane:methanol 8%, afforded the product (377 mg, 63%) as white solid.
**¹H NMR** (400 MHz, CDCl3): δ (ppm) 8.04 (d, J=5.5Hz, 1H), 7.31 (m, 5H), 5.98 (d, J=5.5Hz, 1H), 4.22 (m, 1H), 3.88 (s, 3H), 3.81 (t, J=4.5 Hz, 4H), 3.59 (AB system, 2H), 3.06 (t, J=8Hz, 1H), 2.97 (bs, 1H), 2.78 (m, 1H), 2.72 (m, 1H), 2.65 (m, 3H), 2.48 (m, 2H), 2.27 (t, J=8Hz, 1H). **¹³C NMR** (75 MHz, CDCl₃): δ (ppm) 169.87, 161.64, 157.84, 137.93, 128.91, 128.31, 127.28, 96.67, 74.79, 74.55, 62.32, 60.23, 57.34, 52.91, 51.68, 43.51. **MS (EI+)** m/z: 370.2 (M+H⁺).

### Example 11 : (3,4-trans)-1-benzyl-4-(2,3,8,8a-tetrahydrocyclopenta[ij]isoquinolin-1(7H)-yl)pyrrolidine-3-ol

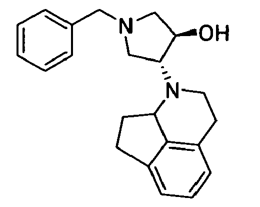

From 1-benzyl-3,4-epoxypyrroline (85 mg, 0.48 mmol), 1,2,3,7,8,8a-hexahydrocyclopenta[*ij*]isoquinoline (85 mg, 0.53 mmol), LiCl0₄ (103 mg, 0.97 mmol) and acetonitrile (6 ml). Reaction time: 6 hours. Purification: silica gel, gradient dichloromethane to dichlorometane:methanol 8%, afforded the product (87 mg, 54%) as brown oil (mixture of two diastereomers).
**¹H NMR** (400 MHz, CDCl3): δ (ppm) 7.31 (m, 10H), 7.08 (m, 2H), 7.00 (m, 2H), 6.91 (m, 2H), 4.42 (m, 1H), 4.36 (m, 1H), 3.78 (m, 1H), 3.61 (AB system, 2H), 3.59 (AB system, 2H), 3.58 (m, 1H), 3.36 (m, 3H), 3.19 (m, 1H), 3.04 (m, 3H), 2.97-2.73 (m, 8H), 2.72-2.44 (m, 9H), 1.79 (m, 2H). **¹³C NMR** (75 MHz, CDCl₃): δ (ppm) 141.91, 141.76, 141.74, 141.64, 138.38, 137.65, 131.79, 131.62, 128.94, 128.74, 128.36, 128.35, 127.58, 127.38, 127.25, 127.14, 124.68, 124.67, 121.77, 121.65, 73.97, 70.68, 70.11, 69.93, 65.93, 65.48, 62.25, 60.86, 60.35, 60.10, 57.12, 51.17, 48.07, 46.99, 35.40, 34.77, 30.46, 30.39, 27.80, 27.70. **MS (EI+)** m/z: 335.2 (M+H⁺).

### Example 12 : tert-butyl-4-((3,4-trans)-1-benzyl-4-hydroxypyrrolidine-3-yl)piperazine-1-carboxylate

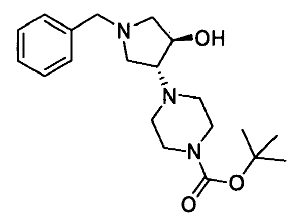

From 1-benzyl-3,4-epoxypyrroline (90 mg, 0.51 mmol), *tert*-butyl-1-piperazine carboxylate (124 mg, 0.66 mmol), LiClO₄ (109 mg, 1.02 mmol) and acetonitrile (5 ml). Reaction time: 5 hours. Purification: silica gel, gradient dichloromethane to dichlorometane:methanol 8%, afforded the product (115 mg, 62%) as yellow oil.
**¹H NMR** (400 MHz, CDCl3): δ (ppm) 7.30 (m, 5H), 4.14 (m, 1H), 3.56 (AB system, 2H), 3.42 (m, 4H), 3.00 (t, J=8z, 1H), 2.89 (bs, 1H), 2.73 (m, 1H), 2.68 (m, 1H), 2.63 (m, 1H), 2.51 (m, 2H), 2.36 (m, 2H), 2.20 (t, J=8Hz, 1H), 1.46 (s, 9H). **¹³C NMR** (75 MHz, CDCl₃): δ (ppm) 154.54,137.75,128.78,128.17,127.07, 79.56, 74.24, 74.09, 62.13, 60.11, 56.92, 51.28, 43.25, 28.28. **MS (EI+)** m/z: 362.2 (M+H⁺).

### Example 13 : tert-butyl 4-((3,4-trans)-4-hydroxy-1-(phenylsulfonyl)pyrrolidine-3-yl)piperazine-1-carboxylate

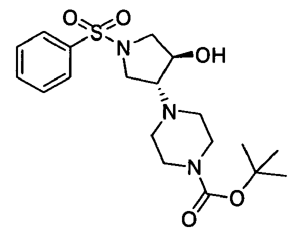

From 1-phenylsulfonyl-3,4-epoxypyrroline (100 mg, 0.44 mmol), *tert*-butyl-1-piperazine carboxylate (107 mg, 0.57 mmol), LiClO₄ (94 mg, 0.88 mmol) and acetonitrile (6 ml). Reaction time: 5 hours. Purification: silica gel, gradient hexane:ethyl acetate 1:1 to 1:3, afforded the product (84 mg, 46%) as colourless oil.
**¹H NMR** (400 MHz, CDCI3): δ (ppm) 7.77 (m, 2H), 7.60 (m, 1H), 7.52 (m, 2H), 4.18 (m, 1H), 3.45 (m, 2H), 3.31 (t, J=5Hz, 4H), 3.09 (dd, J₁=5Hz, J₂=10Hz, 1H), 3.00 (dd, J₁=7Hz, J₂=10Hz, 1H), 2.76 (m, 1H), 2.46 (m, 2H), 2.32 (m, 2H), 1.41 (s, 3H). **¹³C NMR** (75 MHz, CDCl₃): δ (ppm) 154.66, 135.39, 133.11, 129.23, 127.73, 80.01, 72.09, 71.06, 54.31, 50.63, 48.91, 43.05, 28.41. **MS (EI+)** m/z: 412.2 (M+H⁺).

### Example 14 : (3,4-trans)-4-morpholino-1-(phenylsulfonyl)pyrrolidine-3-ol

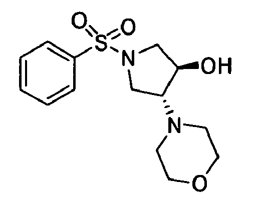

From 1-phenylsulfonyl-3,4-epoxypyrroline (40 mg, 0.17 mmol), morpholine (20 mg, 0.23 mmol), LiClO₄ (38 mg, 0.35 mmol) and acetonitrile (4 ml). Reaction time: 5 hours. Purification: silica gel, gradient dichloromethane to dichlorometane:methanol 5%, afforded the product (26 mg, 47%) as colourless oil.
**¹H NMR** (400 MHz, CDCl3): δ (ppm) 7.82 (d, J=7.5Hz, 2H), 7.63 (t, J=7.5Hz, 1H), 7.55 (t, J=7.5Hz, 2H), 4.22 (m, 1H), 3.63 (t, J=4Hz, 4H), 3.50 (m, 2H), 3.16 (dd, J₁=4.5Hz, J₂=10Hz, 1H), 3.06 (dd, J₁=7.3Hz, J₂=10Hz, 1H), 2.76 (m, 1H), 2.54 (m, 2H), 2.41 (m, 2H), 2.25 (bs, 1H). ¹³**C NMR** (75 MHz, CDCl₃): δ (ppm) 135.73, 133.08, 129.24, 127.87, 72.24, 71.55, 66.73, 54.35, 51.40, 48.65. **MS (El+)** m/z: 313.1 (M+H⁺).

### Example 15 : (3,4-trans)-4-(3,4-dihydroisoquinolin-2(1H)-yl)-1-(phenylsulfonyl)pyrrolidine-3-ol

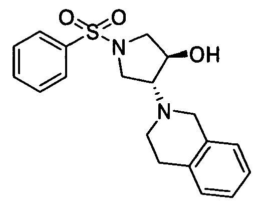

From 1-phenylsulfonyl-3,4-epoxypyrroline (100 mg, 0.44 mmol), 1,2,3,4-tetrahydroisoquinoline (77 mg, 0.57 mmol), LiClO₄ (94 mg, 0.88 mmol) and acetonitrile (6 ml). Reaction time: 5 hours. Purification: silica gel, gradient dichloromethane to dichlorometane:methanol 8%, afforded the product (115 mg, 72%) as light brown oil.
**¹H NMR** (400 MHz, CDCI3): δ (ppm) 7.79 (d, J=7.5Hz, 2H), 7.58 (t, J=7.5Hz, 1H), 7.51 (t, J=7.5Hz, 2H), 7.09 (m, 2H), 7.03 (m, 1H), 6.95 (m, 1H), 4.21 (m, 1H), 3.61 (AB system, 2H), 3.53 (dd, J₁=7.5Hz, J₂=10Hz, 1H), 3.40 (dd, J₁=7Hz, J₂=10Hz, 1H), 3.08 (m, 2H), 2.88 (m, 1H), 2.78 (m, 3H), 2.62 (m, H). **¹³C NMR** (75 MHz, CDCl₃): δ (ppm) 135.32, 133.82, 133.08, 129.20, 128.68, 127.78, 126.65, 126.48, 125.84, 72.30, 70.84, 54.32, 53.77, 48.88, 48.22, 28.83. **MS (EI+)** m/z: 359.1 (M+H⁺).

### Example 16 : (3,4-trans)-4-(3,4-dihydroisoquinolin-2(1H)-yl)-1((S)-1-phenylethyl)pyrrolidine-3-ol

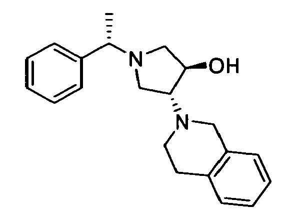

From 1-((S)-1-phenylethyl)-3,4-epoxypyrroline (100 mg, 0.52 mmol), 1,2,3,4-tetrahydroisoquinoline (110 mg, 0.79 mmol), LiClO₄ (114 mg, 1.05 mmol) and acetonitrile (5 ml). Reaction time: 4 hours. Purification: silica gel, gradient dichloromethane to dichlorometane:methanol 5%, afforded the product (90 mg, 53%) as white solid (mixture of two diastereomers).
**¹H NMR** (400 MHz, CDCl3): δ (ppm) 7.33 (m, 8H), 7.26 (m, 2H), 7.10 (m, 6H), 7.01 (m, 1H), 6.97 (m, 1H), 4.26 (m, 1H), 4.22 (m, 1H), 3.70 (AB system, 2H), 3.62 (AB system, 2H), 3.22 (m, 3H), 2.99 (m, 2H), 2.90 (m, 6H), 2.75 (m, 3H), 2.61 (m, 3H), 2.51 (m, 1H), 2.31 (t, J=8Hz, 1H), 2.18 (t, J=8Hz, 1H), 1.41 (d, J=6.5Hz, 3H), 1.37 (d, J=6.5Hz, 3H). **¹³C NMR** (75 MHz, CDCl₃): δ (ppm) 144.04, 143.77, 134.12, 134.11, 134.00, 133.95, 128.57, 128.53, 128.42, 128.39, 127.19, 127.15, 126.57, 126.55, 126.24, 126.20, 125.66, 125.61, 74.63, 74.17, 73.78, 73.65, 65.41, 65.26, 60.97, 60.75, 56.48, 55.64, 54.65, 54.49, 49.08, 48.85, 28.80, 22.22, 22.04. **MS (EI+)** m/z: 322.2 **(M⁺).**

### Example 17 : (3,4-trans)-1-benzyl-4-(4-benzylpiperazin-1-yl)pyrrolidine-3-ol

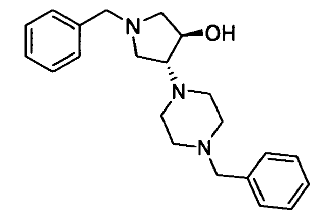

From 1-benzyl-3,4-epoxypyrroline (103 mg, 0.59 mmol), 1-benzylpiperazine (128 mg, 0.70 mmol), LiC10₄ (125 mg, 1.18 mmol) and acetonitrile (5 ml). Reaction time: 3 hours. Purification: silica gel, gradient dichloromethane to dichlorometane:methanol 8%, afforded the product (107 mg, 52%) as brown solid. M.p. 102-103°C.
**¹H NMR** (400 MH**z**, CDCl3): δ (ppm) 7.30 (m, 8H), 7.25 (m, 2H), 4.16 (m, 1H), 3.56 (AB system, 2H), 3.51 (s, 2H), 3.03 (t, J=8z, 1H), 2.76 (d, J=10Hz, 1H), 2.69 (td, J₁=3Hz, J₂=8Hz, 1H), 2.62 (dd, J₁=6Hz, J₂=10Hz, 2H), 2.48 (bs, 8H), 2.19 (t, J=8Hz, 1H). **¹³C NMR** (75 MHz, CDCl₃): δ (ppm) 137.91, 137.74, 129.11, 128.82, 128.20, 128.10, 127.07, 126.96, 74.55, 74.18, 62.90, 62.27, 60.19, 57.21, 52.66, 51.53. **MS (El+)** m/z: 352.2 (M+H⁺).

### Example 18 : (3,4-trans)-1-benzyl-4-(4-phenylpiperazin-1-yl)pyrrolidine-3-ol

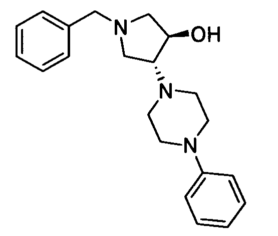

From 1-benzyl-3,4-epoxypyrroline (106 mg, 0.60 mmol), 1-phenylpiperazine (119 mg, 0.72 mmol), LiClO₄ (129 mg, 1.21 mmol) and acetonitrile (5 ml). Reaction time: 3 hours. Purification: silica gel, gradient dichloromethane to dichlorometane:methanol 8%, afforded the product (74 mg, 36%) as brown solid.
M.p. 120-123°C.
**¹H NMR** (400 MHz, CDCl3): δ (ppm) 7.32 (m, 4H), 7.27 (m, 3H), 6.92 (m, 2H), 6.85 (m, 1H), 4.21 (m, 1H), 3.59 (AB system, 2H), 3.20 (t, J=5z, 4H), 3.08 (t, J=8Hz, 1H), 2.80 (m, 1H), 2.74 (m, 3H), 2.68-2.56 (m, 3H), 2.22 (t, J=8Hz, 1H). **¹³C NMR** (75 MHz, CDCl₃): δ (ppm) 151.38, 138.24, 129.15, 128.84, 128.34, 127.21, 119.82, 116.02, 74.93, 74.79, 62.45, 60.19, 57.54, 51.79, 48.97. **MS (EI+)** m/z: 338.2 (M+H⁺).

### Example 19: Ethyl 2-(4-((3,4-trans)-1-benzyl-4-hydroxypyrrolidine-3-yl)piperazin-1-yl)acetate

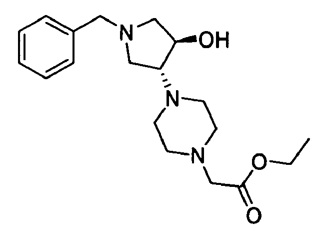

From 1-benzyl-3,4-epoxypyrroline (113 mg, 0.64 mmol), 1-ethylpyperazinoacetate (140 mg, 0.77 mmol), LiClO₄ (140 mg, 1.28 mmol) and acetonitrile (5 ml). Reaction time: 4 hours. Purification: silica gel, gradient dichloromethane to dichlorometane:methanol 8%, afforded the product (84 mg, 37%) as brown oil.
**¹H NMR** (400 MHz, CDCl3): δ (ppm) 7.30 (m, 5H), 4.18 (q, J=7Hz, 2H), 4.16 (m, 1H), 3.57 (AB system, 2H), 3.19 (s, 2H), 3.02 (t, J=8z, 4H), 2.76 (d, J=10Hz, 1H), 2.70 (td, J₁=3Hz, J₂=7.5Hz, 1H), 2.62 (m, 6H), 2.51 (m, 4H), 2.19 (t, J=8Hz, 1H), 1.26 (t, J=7Hz, 3H). **¹³C NMR** (75 MHz, CDCl₃): δ (ppm) 138.06, 128.85, 128.35, 127.19, 74.76, 74.55, 62.32, 60.89, 60.18, 59.45, 57.31, 52.85, 51.40, 14.24. **MS (EI+)** m/z: 348.2 (M+H⁺).

### Example 20 : (4-((3,4-trans)-1-benzyl-4-hydroxypyrrolidine-3-yl)piperazin-1-yl)(phenyl)methanone

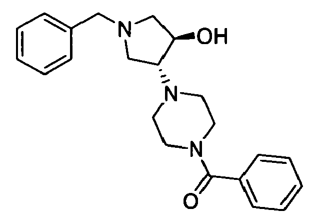

From 1-benzyl-3,4-epoxypyrroline (253 mg, 1.44 mmol), 1-benzoylpiperazine (330 mg, 1.73 mmol), LiClO₄ (308 mg, 2.89 mmol) and acetonitrile (10 ml). Reaction time: 5 hours. Purification: silica gel, gradient dichloromethane to dichlorometane:methanol 8%, afforded the product (317 mg, 60%) as brown solid. M.p. 130-134°C.
**¹H NMR** (400 MHz, CDCl3): δ (ppm) 7.38 (m, 5H), 7.30 (m, 5H), 4.14 (m, 1H), 3.84 (bs, 1H), 3.71 (bs, 1H), 3.56 (AB system, 2H), 3.42 (bs, 2H), 3.00 (t, J=8Hz, 1H), 2.71 (bs, 1H), 2.66-2.54 (m, 4H), 2.33 (m, 1H), 2.19 (t, J=8Hz, 1H). **¹³C NMR** (75 MHz, CDCl₃): δ (ppm) 170.29, 138.00, 135.60, 129.72, 128.82, 128.47, 128.35, 127.24, 127.06, 74.47, 74.45, 62.27, 60.12, 57.16, 47.50, 42.00. **MS (El+)** m/z: 366.2 (M+H⁺).

### Example 21 :Ethyl 4-((3,4-trans)-1-benzyl-4-hydroxypyrrolidine-3-yl)piperazine-1-carboxilate

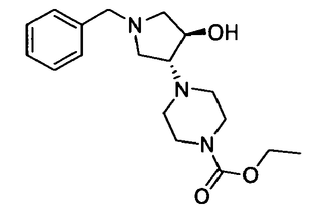

From 1-benzyl-3,4-epoxypyrroline (122 mg, 0.69 mmol), ethyl-1-piperazinecarboxilate (133 mg, 0.83 mmol), LiClO₄ (151 mg, 1.39 mmol) and acetonitrile (5 ml). Reaction time: 4 hours. Purification: silica gel, gradient dichloromethane to dichlorometane:methanol 8%, afforded the product (90 mg, 39%) as brown oil.
**¹H NMR** (400 MHz, CDCl3): δ (ppm) 7.30 (m, 5H), 4.14 (m, 1 H), 4.12 (q, J=7Hz, 2H), 3.56 (AB system, 2H), 3.46 (m, 4H), 3.00 (t, J=8Hz, 1H), 2.75 (m, 1H), 2.67 (td, J₁=3Hz, J₂=8Hz, 1H), 2.62 (m, 1H), 2.52 (m, 2H), 2.36 (m, 2H), 2.18 (t, J=8Hz, 1H), 1.24 (t, J=7Hz, 3H). **¹³C NMR** (75 MHz, CDCl₃):δ (ppm) 137.99, 128.90, 128.41, 127.28, 74.59, 74.55, 62.35, 61.42, 60.20, 57.35, 51.41, 43.45, 14.65. **MS (EI+)** m/z: 334.2 (M+H⁺).

### Example 22 :tert-butyl 4-((3,4-trans)-1-(4-fluorobenzyl)-4-hydroxypyrrolidine-3-yl)piperazine-1-carboxylate

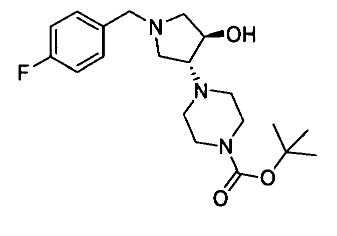

From 4-fluoro-1-benzyl-3,4-epoxypyrroline (55 mg, 0.28 mmol), *tert-*butyl-1-piperazinecarboxilate (69 mg, 0.37 mmol), LiClO₄ (61 mg, 0.57 mmol) and acetonitrile (3 ml). Reaction time: 8 hours. Purification by crystallization of dichloromethane:hexane afforded the product (58 mg, 53%) as white solid.
**¹H NMR** (400 MHz, CDCl3): δ (ppm) 7.25 (m, 2H), 6.99 (m, 2H), 4.16 (m, 1H), 3.52 (AB system, 2H), 3.42 (m, 4H), 2.99 (t, J=8Hz, 1H), 2.73 (m, 1H), 2.66 (td, J₁=3Hz, J₂=8Hz, 1H), 2.60 (m, 1H), 2.52 (m, 2H), 2.36 (m, 2H), 2.17 (t, J=8Hz, 1H), 1.45 (s, 9H). **¹³C NMR** (75 MHz, CDCl₃): δ (ppm) 162.40 (d, J_{CF}=244Hz), 154.66, 133.95, 130.24 (d, J_{CF}=8H_{Z}), 115.10 (d, J_{CF}=21Hz), 79.69, 74.69, 74.59, 64.64, 62.19, 59.33, 57.29, 51.48, 44.02, 28.37. **MS (El+)** m/z: 380.2 (M+H⁺).

### Example 23 : (3,4-trans)-1-benzyl-4-(4-methylpiperazin-1-yl)pyrrolidine-3-ol

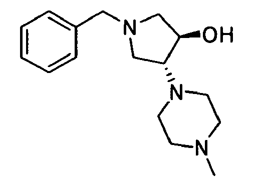

From 1-benzyl-3,4-epoxypyrroline (116 mg, 0.66 mmol), 1-methylpiperazine (80 mg, 0.79 mmol), LiCl0₄ (141 mg, 1.33 mmol) and acetonitrile (5 ml). Reaction time: 4 hours. Purification: silica gel, gradient dichloromethane to dichlorometane:methanol 8%, afforded the product (103 mg, 56%) as brown oil.
**¹H NMR** (400 MHz, CDCl3): δ (ppm) 7.30 (m, 5H), 4.16 (m, 1H), 3.57 (AB system, 2H), 3.04 (t, J=8Hz, 1H), 2.78 (m, 1H), 2.67 (td, J₁=3Hz, J₂=8Hz, 1H), 2.61 (m, 1H), 2.46 (bs, 8H), 2.27 (s, 3H), 2.17 (t, J=8Hz, 1H). **¹³C NMR** (75 MHz, CDCl₃): 5 (ppm) 137.74, 128.83, 128.30, 127.13, 74.96, 74.74, 62.45, 60.17, 57.61, 54.95, 51.72, 46.01. MS (EI+) m/z: 276.2

### Example 24 : tert-butyl-4-(3,4-trans)-4-acetoxy-1-benzylpyrrolidine-3-yl)piperazine-1-carboxylate

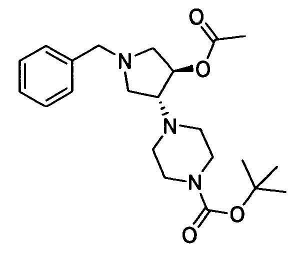

To a solution of *tert*-butyl-4-((3,4-trans)-1-benzyl-4-hydroxypyrrolidine-3-yl)piperazine-1-carboxylate (5.07 g, 14.02 mmol), 4-dimethylaminopyridine (171 mg, 1.40 mmol) and N,N-diisopropylethylamine (5.43 g, 42 mmol) in dichloromethane (200 ml) cooled at 0°C, was added acetyl chloride (1.68 g, 21.03 mmol). The resulting mixture was stirred for 1.5 hours raising the temperature slowly from 0°C to r.t. Additional dichloromethane was added and the solution was washed with water and saturated solution of NaCl, dried over NaSO₄, filtered and concentrated. Purification by flash chromatography: silica gel, gradient dichloromethane to dichloromethane:methanol 5%, afforded the product (5.4 g, 95%) as light brown oil.
**¹H NMR** (400 MHz, CDCl3): δ (ppm) 7.31 (m, 5H), 5.12 (m, 1H), 3.57 (AB system, 2H), 3.41 (bs, 4H), 3.05 (t, J=8Hz, 1H), 2.97 (td, J₁=3Hz, J₂=8Hz, 1H), 2.74 (m, 2H), 2.43 (m, 2H), 2.34 (m, 2H), 2.25 (t, J=8Hz, 1 H), 2.04 (s, 3H), 1.44 (s, 9H). **MS (El+)** m/z: 404.2 (M+H⁺).

### Example 25 : (3,4-trans)-1-benzyl-4-(piperazin-1-yl)pyrrolidine-3-yl acetate

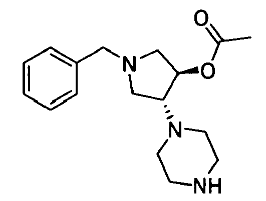

To a solution of *tert*-butyl-4-(3,4-trans)-4-acetoxy-1-benzylpyrrolidine-3-yl)piperazine-1-carboxylate (2.5 g, 6.19 mmol) in dioxane (18 ml) was added a solution of HCl 4.0M in dioxane (30 ml, 120 mmol) and the mixture was stirred at r.t. for 5 hours. The suspension obtained was concentrated to dryness and the residue was triturated with dichloromthane. The yellow solid obtained was filtered to afforded the product (2,3 g, 93%) as tri-hydrochloride.
**¹H NMR** (400 MHz, CD₃OD): δ (ppm) 7.67 (m, 2H), 7.53 (m, 3H), 5.70 (m, 1H), 4.60 (AB system, 2H), 4.13 (m, 1H), 4.05 (m, 2H), 3.84 (m, 1H), 3.63 (m, 1H), 3.56 (m, 6H), 3.42 (m, 2H), 2.15 (s, 3H). **¹³C NMR** (75 MHz, CD₃OD): δ (ppm) 171.40, 132.07, 131.43, 130.84, 130.51, 72.38, 68.34, 60.14, 53.73, 43.28, 20.77. **MS (EI+)** m/z: 304.1 (M+H⁺).

### Example 26 : tert-butyl 4-((3,4-trans)-4-acetoxypyrrolidine-3-yl)piperazine-1-carboxylate

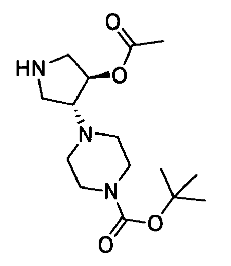

A solution of *tert*-butyl-4-(3,4-trans)-4-acetoxy-1-benzylpyrrolidine-3-yl)piperazine-1-carboxylate (2.9 g, 7.18 mmol) in methanol (110 ml) was purgued with argon, and Pd/C 10% was added (1.2 g). The mixture was purgued again with argon and after with hydrogen. The mixture was stirred at r.t. under hydrogen atmosphere for 24 hours. The suspension was purgued with argon, filtered over celite and washed with dichloromethane. The filtrate was concentrated to dryness to afforded the product (2.1 g, 93%) as brown oil.
**¹H NMR** (400 MHz, CDCl3): δ (ppm) 5.18 (m, 1H), 3.42 (m, 4H), 3.30 (m, 1H), 3.24 (m, 1H), 3.00 (m, 1H), 2.85 (m, 2H), 2.64 (bs, 1H), 2.50 (m, 2H), 2.42 (m, 2H), 2.06 (s, 3H), 1.45 (s, 9H). **¹³C NMR** (75 MHz, CDCl₃): δ (ppm) 170.35, 154.56, 79.67, 77.55, 72.10, 53.34, 51.43, 50.29, 43.31, 28.37, 21.13.

### Synthesis of piperazine sulfonamides

### A) trans 1-Benzyl-4-(4-(aryl sulfonyl)piperazin-1-yl)pyrrolidin-3-yl acetate (dihydrochloride).

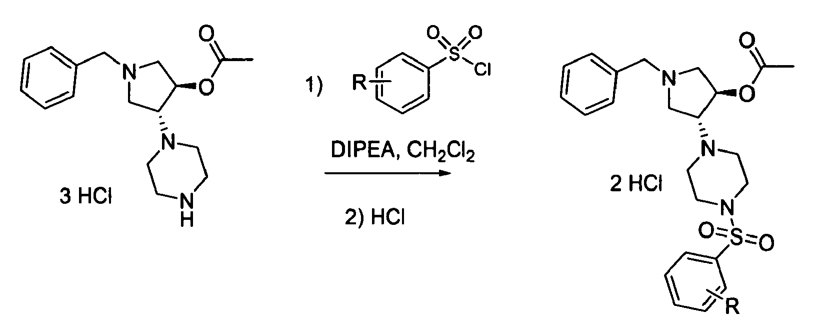

*trans*-1-Benzyl-4-(piperazin-1-yl)pyrrolidin-3-yl acetate trihydrochloride (0.25 mmol, 1 eq.), arylsulfonyl chloride (0.28 mmol, 1.1 eq) and N,N'-diisopropylethylamine (1.5 mmol, 6 eq)) are dissolved in CH₂Cl₂ (10 mL) and the mixture was stirred overnight at room temperature. The resulting solution was washed with water (3 x 10 mL), dried over Na₂SO₄ and evaporated to dryness. The base was crystallized in ethyl acetate (1-2 ml) or was crystallized as a hydrochloride with a mixture of ethyl acetate and a 2,8 M solution of hydrogen chloride in ethanol. Once crystallized, the product was collected by filtration, and vacuum dried to give a white solid (yield: 70-80%).

### Example 27: trans 1-Benzyl-4-(4-(2-phenyl-phenylsulfonyl)piperazin-1-yl)pyrrolidin-3-yl acetate.

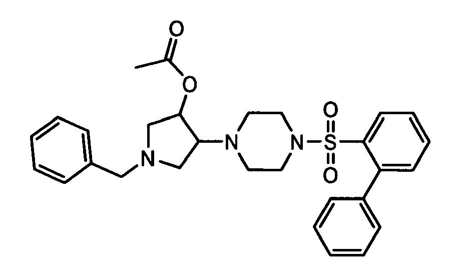

**¹H NMR** (300 MHz, DMSO-D6) δ ppm 1.98 (s, 3 H) 2.13 (m, 6 H) 2.57 - 2.72 (m, 6 H) 2.81 (m, 1 H) 3.48 (m, 2 H) 4.81 (m, 1 H) 7.26 (m, 5 H) 7.37 (m, 6 H) 7.61 (t, *J*=7.62 Hz, 1 H) 7.71 (t, J=7.47 Hz, 1 H) 7.99 (d, *J*=7.76 Hz, 1 H).
**MS** (APCI (M+H)⁺): 520

### B) trans-1-Benzyl-4-(4-(arylsulfonyl)piperazin-1-yl)pyrrolidin-3-ol.

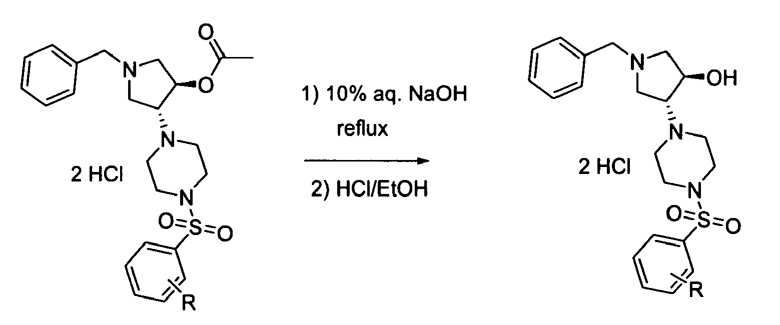

*trans-*1-Benzyl-4-(4-(arylsulfonyl)piperazin-1-yl)pyrrolidin-3-yl acetate (0.24 mmol, 1 eq.) was suspended in 10% aq. NaOH (24 mmol, 100 eq.) and the mixture was heated at reflux overnight. The product was extracted with ethyl acetate (3 x 10 ml), washed with water and brine and dried over Na₂SO₄. After the evaporation of the solvent, the oil obtained was treated with 2.8 M of hydrogen chloride in ethanol, crystallized, collected by filtration and vacuum dried to give a white solid identified as the corresponding pyrrolidin-3-ol (yield: 60-80%).

### Example 28: trans 1-Benzyl-4-(4-(2-phenyl-phenylsulfonyl)piperazin-1-yl)pyrrolidin-3-ol dihydrochloride

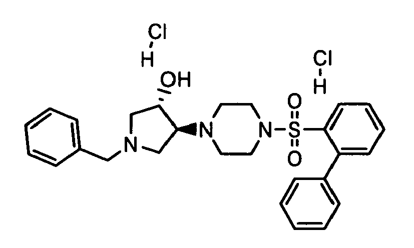

**¹H NMR** (400 MHz, METHANOL-*d*₄) δ ppm 3.08 (m., 2 H) 3.21 (m, 2 H) 3.54 (m, 2 H) 3.54 (m, 4 H) 3.65 (m, 2 H) 3.86 (m, 1 H) 4.46 (d, J=4.30 Hz, 2 H) 4.75 (m, 1 H) 7.48 - 7.56 (m, 6 H) 7.65 (m, 2 H) 8.07 (d, J=7.42 Hz, 1 H).
**MS** (APCI (M+H)⁺): 436

### Example 29: (3,4-trans)-1-benzyl-4-(phenylamino)pyrrolidin-3-ol

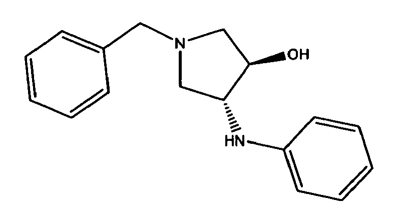

From 1-benzyl-3,4-epoxypyrroline (168 mg, 0.95 mmol), aniline (135 mg, 1.43 mmol) and LiC104 (204 mg, 1.91 mmol), no solvent was employed. Reaction time: 24 hours. Purification: silica gel, gradient dichloromethane to dichloromethane:methanol 10%, afforded the product (77 mg, 30%) as yellow solid
**1H NMR** (400 MHz, CDC13): 8 (ppm) 7.31 (m, 5H), 7.18 (t, J=7.5Hz, 2H), 6.72 (t, J=7.5Hz, 1H), 6.66 (t, J=7.5Hz, 2H), 4.06 (m, 1H), 3.79 (m, 2H), 3.67 (AB system, 2H), 3.25 (m, 1H), 2.83 (m, 1H), 2.63 (m, 1H), 2.28 (m, 1H). **¹³C NMR** (75 MHz, CDC1₃): 3 (ppm) 147.05, 138.04, 129.37, 128.88, 128.49, 127.32, 117.87, 113.54, 76.65, 61.80, 61.20, 59.88, 59.48. MS (EI⁺) m/z: 269.1 (M+H⁺).

### Example 30: (3,4-trans)-1-benzyl-4-(benzylamino)pyrrolidin-3-ol

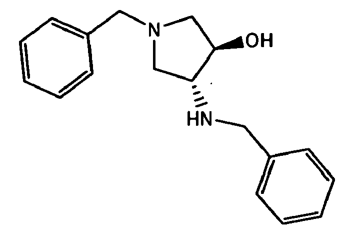

From 1-benzyl-3,4-epoxypyrroline (108 mg, 0.61 mmol), benzylamine (100 mg, 0.92 mmol), LiC104 (131 mg, 1.23 mmol) and acetonitrile (5 ml). Reaction time: 6 hours. Purification: silica gel, gradient dichloromethane to dichloromethane:methanol 10%, afforded the product (85 mg, 49%) as yellow solid.
**1H NMR** (400 MHz, CDC13): 8 (ppm) 7.30 (m, 10H), 4.02 (m, 1H), 3.76 (s, 2H), 3.60 (AB system, 2H), 3.15 (td, J₁=2.5Hz, J_{Z}= 7Hz, 11-1), 3.08 (m, 1H), 2.74 (m, 1H), 2.65 (m, 1H), 2.27 (bs, 2H), 2.18 (m, 1H). **¹³C NMR** (75 MHz, CDCl₃): S (ppm) 139.76, 138.20, 128.87, 128.52, 128.29, 127.19, 77.06, 66.40, 61.12, 60.12, 59.41, 52.41. MS (El+) m/z: 283.2 (M+H⁺).

### Example 31: (3R,4R)-1-benzyl-4-(octylamino)pyrrolidin-3-ol

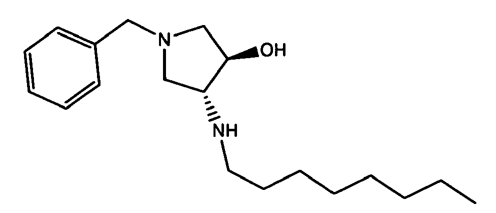

From 1-benzyl-3,4-epoxypyrroline (100 mg, 0.57 mmol), octylamine (112 mg, 0.85 mmol), LiC104 (121 mg, 1.14 mmol) and acetonitrile (5 ml). Reaction time: 7 hours. Purification: silica gel, gradient dichloromethane to dichloromethane:methanol 10%, afforded the product (100 mg, 57%) as yellow solid.
**1H NMR** (400 MHz, CDC13): 3 (ppm) 7.31 (m, 5H), 4.04 (m, 1H), 3.60 (AB system, 2H), 3.11 (td, J,=2.5Hz, JZ= 7Hz, 1H), 3.03 (m, lH), 2.97 (bs, 2H), 2.76 (m, 1H), 2.60 (m, 3H), 2.22 (m, 1H), 1.48 (m, 2H), 1.26 (m, 12H), 0.87 (t, J=7Hz, 3H). **³C NMR** (75 MHz, CDC1₃): 8 (ppm) 138.23, 128.82, 128.27, 127.10, 76.61, 67.05, 60.96, 60.11, 58.93, 48.19, 31.82, 29.83, 29.41, 29.20, 27.30, 22.66, 14.10.

### Example 32: (3,4-trans)-1-benzyl-4-(4-methoxybenzylamino)pyrrolidin-3-ol

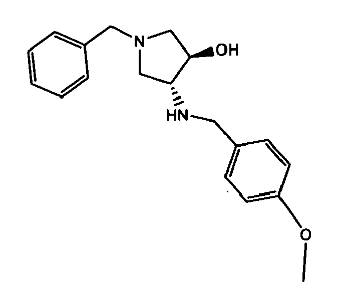

From 1-benzyl-3,4-epoxypyrroline (304 mg, 1.73 mmol), 4-methoxybenzylamine (285 mg, 2.08 mmol), LiC104 (369 mg, 3.47 mmol) and acetonitrile (15 ml).
Reaction time: 10 hours. Purifcation: silica gel, gradient dichloromethane to dichloromethane:methanol
111 NMR (400 MHz, CDC13): S (ppm) 7.30 (m, 5H), 7.21 (d AB, 2H), 6.83 (d AB, 2H), 4.03 (m, 1H), 3.77 (s, 3H), 3.78 (s, 2H), 3.61 (AB system, 2H), 3.16 (td, J₁=2.5Hz, J_{z}= 7Hz, 1H), 3.07 (m, 1H), 2.76 (m, 3H), 2.66 (m, 1H), 2.22 (m, 1H). ¹³C NMR (75 MHz, CDC1₃): 8 (ppm) 158.82, 137.95, 131.48, 129.49, 128.86, 128.29, 127.17, 113.88, 76.77, 66.17, 60.93, 60.03, 59.04, 55.26, 51.63.

### Example 33: (3,4-trans)-1-benzyl-4-(butylamino)pyrrolidin-3-ol

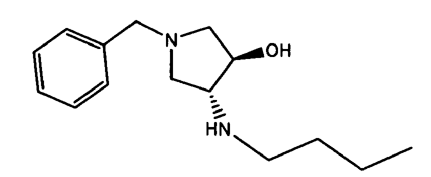

From 1 -benzyl-3,4-epoxypyrroline (307 mg, 1.75 mmol), butylamine (154 mg, 2.10 mmol), LiC104 (372 mg, 3.50 mmol) and acetonitrile (15 ml). Reaction time: 4 hours. Purification: silica gel, gradient dichloromethane to dichloromethane:methanol 10%, afforded the product (76 mg, 17%) as yellow solid.
**1H NMR** (400 MHz, CDC13): 8 (ppm) 7.31 (m, 5H), 3.98 (m, 1H), 3.59 (AB system, 2H), 3.08 (td, Jₗ=2.5Hz, J_{z}= 7Hz, 1H), 3.02 (m, 1H), 2.74 (m, 1H), 2.58 (m, 4H), 2.18 (m, 1H), 1.44 (m, 2H), 1.31 (m, 2H), 0.89 (t, J=7Hz, 3H). **³C NMR** (75 MHz, CDC1₃): 8 (ppm) 138.36, 128.88, 128.28, 127.11, 76.88, 67.11, 61.09, 60.11, 59.29, 48.05, 32.20, 20.45, 13.93.

### Example 34: (3R,4R)-1-benzyl-4-(4-(4-chloro-2,5-dimethylphenylsulfonyl)piperazin-1-yl)pyrrolidin-3-yl acetate

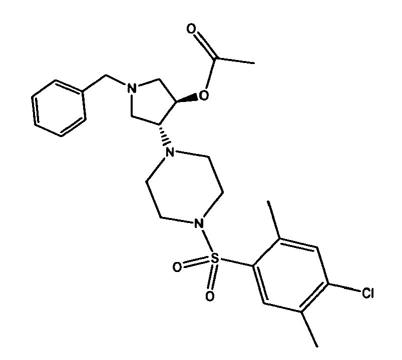

### Example 35: (3R,4R)-1-benzyl-4-(4-tosylpiperazin-1-yl)pyrrolidin-3-yl acetate

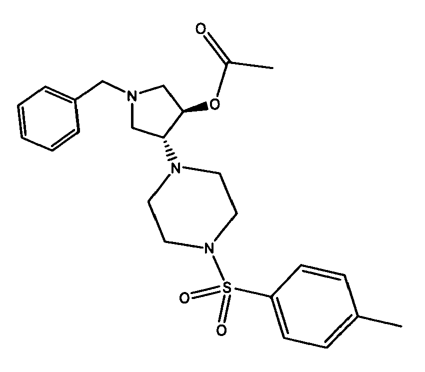

### Example 36: (3,4-trans)-1-benzyl-4-(butylamino)pyrrolidin-3-ol

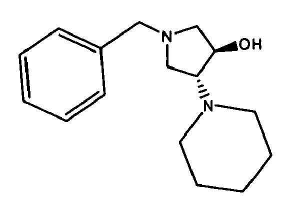

From 1-benzyl-3,4-epoxypyrroline (90 mg, 0.51 mmol), piperidine (65 mg, 0.77 mmol), LiC104 (111 mg, 1.02 mmol) and acetonitrile (4 ml). Reaction time: 4 hours. Purification: silica gel, gradient dichloromethane to dichloromethane:methanol 5%, afforded the product (64 mg, 48%) as yellow oil.
**1H NMR** (400 MHz, CDC13): 8 (ppm) 7.30 (m, 5H), 4.17 (m, 1H), 3.56 (AB system, 2H), 3.03 (t, J=8Hz, 1H), 2.76 (m, 1H), 2.62 (m, 2H), 2.53 (m, 3H), 2.37 (m, 2H), 2.18 (t, J=8Hz, 1H), 1.59 (m, 4H), 1.45 (m, 2H). **¹³C NMR** (75 MHz, CDC1₃): 8 (ppm) 138.02, 128.86, 128.21, 127.04, 75.31, 74.34, 62.23, 60.21, 57.20, 52.83, 25.60, 24.22. MS (El+) m/z: 260.1 (M⁺).

### Example 37: (3R,4R)-1-benzyl-4-(4-methoxyphenylamino)pyrrolidin-3-ol

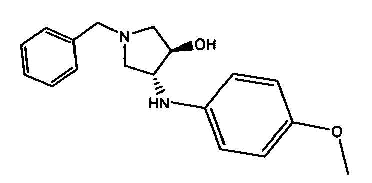

From 1-benzyl-3,4-epoxypyrroline (121 mg, 0.69 mmol), p-anisidine (129 mg, 1.04 mmol) and LiC104 (148 mg, 1.39 mmol), no solvent was employed.
Reaction time: 24 hours. Purification: silica gel, gradient dichloromethane to dichloromethanc:methanol 10%, afforded the product (106 mg, 51%) as yellow solid.
**1H NMR** (400 MHz, CDC13): S (ppm) 7.31 (m, 5H), 6.78 (d AB, 2H), 6.62 (d AB, 2H), 4.03 (m, 1H), 3.73 (s, 3H), 3.72 (m, 1H), 3.64 (AB system, 2H), 3.22 (m, 1H), 2.80 (m, 1H), 2.62 (m, 1H), 2.25 (m, 1H). **¹³C NMR** (75 MHz, CDC1₃): S (ppm) 141.18, 137.99, 128.77, 128.41, 127.26, 114.95, 114.82, 76.55, 62.62, 61.14, 59.82, 59.50, 55.75. MS (El+) m/z: 299.2 (M+H⁺).

### Example 38: Methyl 4-((3,4-trans)-1-benzyl-4-hydroxypyrrolidin-3-ylamino)benzoate

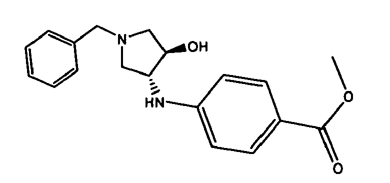

From 1-benzyl-3,4-epoxypyrroline (126 mg, 0.72 mmol), methy14-aminobenzoate (163 mg, 1.07 mmol), LiC104 (153 mg, 1.43 mmol) and acetonitrile (5 ml).
Reaction time: 48 hours. Purification: silica gel, gradient dichloromethane to dichloromethane:methanol 10%, afforded the product (80 mg, 34%) as yellow solid.
**1H NMR** (400 MHz, CDC13): S (ppm) 7.85 (d AB, 2H), 7.31 (m, 5H), 6.61 (d AB, 2H), 4.28 (d, J=7.5Hz, 1H), 4.06 (m, 1H), 3.84 (s, 3H), 3.82 (m, 1H), 3.67 (AB system, 2H), 3.21 (m, 1H), 2.89 (m, 1H), 2.59 (m, 1H), 2.34 (m, 1H). **¹³C NMR** (75 MHz, CDC1₃): S (ppm) 167.33, 137.93, 131.66, 128.84, 128.46, 127.39, 118, 99, 112.26, 76.59, 61.30, 61.26, 59.77, 59.11, 51.58. **MS (EI+)** m/z: 327.2 (M+H⁺).

### Example 39: (3R,4R)-1-benzyl-4-(4-(naphthalen-1-ylsulfonyl)piperazin-1-yl)pyrrolidin-3-yl acetate

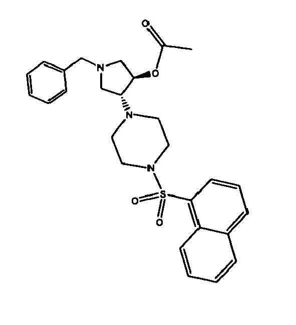

### Example 40: (3R,4R)-1-benzyl-4-(4-(2-chlorophenylsulfonyl)piperazin-1-yl)pyrrolidin-3-ol

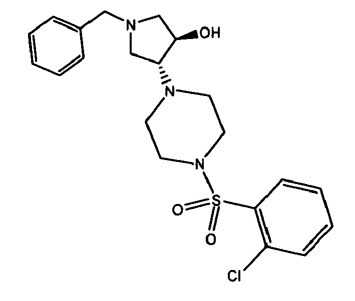

### Example 41: (3R,4R)-1-benzyl-4-(4-(2-bromophenylsulfonyl)piperazin-1-yl)pyrrolidin-3-yl acetate

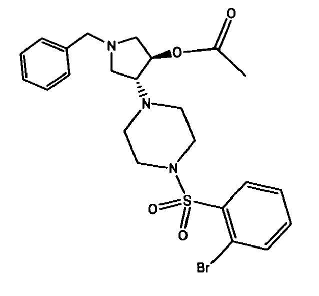

### Example 42: (3R,4R)-1-benzyl-4-(4-(2-bromophenylsulfonyl)piperazin-1-yl)pyrrolidin-3-ol

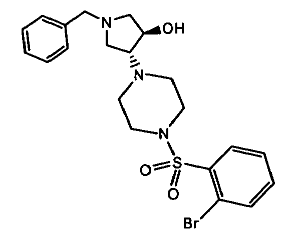

### Example 43: (3R,4R)-1-benzyl-4-(4-(5-fluoro-2-methylphenylsulfonyl)piperazin-1-yl)pyrrolidin-3-yl acetate

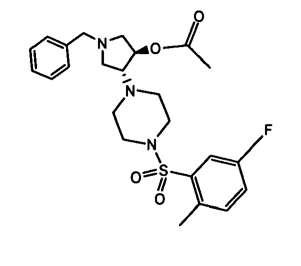

### Example 44: (3R,4R)-1-benzyl-4-(4-(5-fluoro-2-methylphenylsulfonyl)piperazin-1-yl)pyrrolidin-3-ol

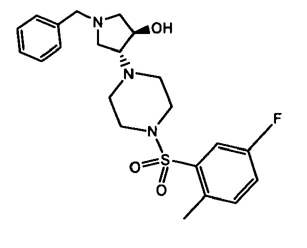

### Example 45: (3R,4R)-1-benzyl-4-(4-(phenylsulfonyl)piperazin-1-yl)pyrrolidin-3-yl acetate

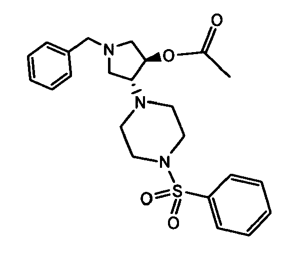

### Example 46: (3R,4R)-1-benzyl-4-(4-(phenylsulfonyl)piperazin-1-yl)pyrrolidin-3-ol

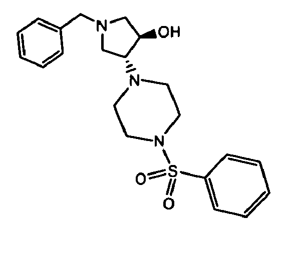

### Example 47: (3R,4R)-1-benzyl-4-(4-(2-fluorophenylsulfonyl)piperazin-1-yl)pyrrolidin-3-yl acetate

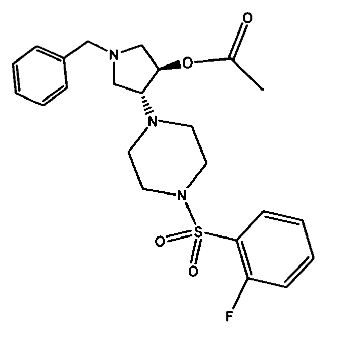

### Example 48: (3R,4R)-1-benzyl-4-(4-(2-fluorophenylsulfonyl)piperazin-1-yl)pyrrolidin-3-ol

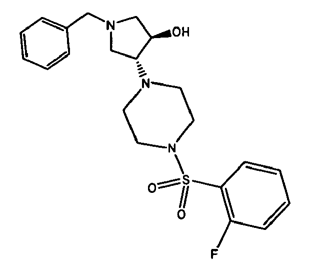

### Example 49: (3R,4R)-1-benzyl-4-(4-(naphthalen-1-ylsulfonyl)piperazin-1-yl)pyrrolidin-3-ol

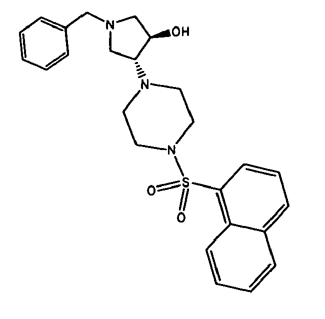

### Example 50: (3R,4R)-1-benzyl-4-(4-(4-chloro-2,5-dimethylphenylsulfonyl)piperazin-1 -yl)pyrrolidin-3-ol

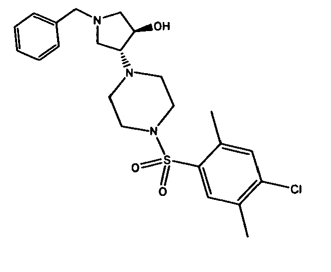

### Example 51: (3R,4R)-1-benzyl-4-(4-tosylpiperazin-1-yl)pyrrolidin-3-ol

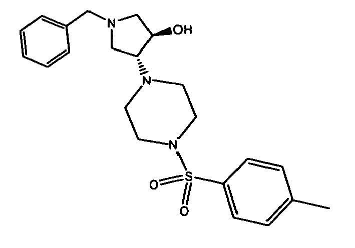

### Example 52: (3R,4R)-1-benzyl-4-(4-(2-chlorophenylsulfonyl)piperazin-1-yl)pyrrolidin-3-yl acetate

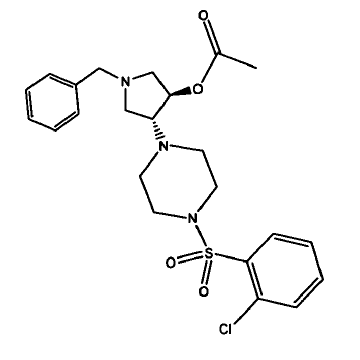

### BIOLOGICAL ACTIVITY

Some representative compounds of the invention were tested for their activity as sigma (sigma-1) inhibitors. The following protocol was followed:

### Sigma-1

Brain membrane preparation and binding assays for the σ1-receptor were performed as described (DeHaven-Hudkins et al., 1992, Eur. J. Pharmacol. 227, 371-378.) with some modifications. In brief, guinea pig brains were homogenized in 10 vols. (w/v) of Tris-HCl 50 mM 0.32 M sucrose, pH 7.4, with a Kinematica Polytron PT 3000 at 15000 r.p.m. for 30 s. The homogenate was centrifuged at 1000g for 10 min at 4°C and the supernatants collected and centrifuged again at 48000g for 15 min at 4°C. The pellet was resuspended in 10 volumes of Tris-HCl buffer (50 mM, pH 7.4), incubated at 37°C for 30 min, and centrifuged at 48000g for 20 min at 4°C. Following this, the pellet was resuspended in fresh Tris-HCl buffer (50 mM, pH 7.4) and stored on ice until use.

Each assay tube contained 10 µL of [³H](+)-pentazocine (final concentration of 0.5 nM), 900 µL of the tissue suspension to a final assay volume of 1 mL and a final tissue concentration of approximately 30 mg tissue net weight/mL. Non-specific binding was defined by addition of a final concentration of 1 µM haloperidol. All tubes were incubated at 37°C for 150 min before termination of the reaction by rapid filtration over Schleicher & Schuell GF 3362 glass fibre filters [previously soaked in a solution of 0,5% polyethylenimine for at least 1 h]. Filters were then washed with four times with 4 mL of cold Tris-HCl buffer (50 mM, pH 7.4). Following addition of scintillation cocktail, the samples were allowed to equilibrate overnight. The amount of bound radioactivity was determined by liquid scintillation spectrometry using a Wallac Winspectral 1414 liquid scintillation counter. Protein concentrations were determined by the method of Lowry et al. (1951; J. Biol. Chem, 193, 265.).

Some of the results with respect to sigma-1 receptor binding are shown in table I.

**Table I**

| Example | Percent Replacement Sigma-1 receptor [10⁻⁷ M] |
|---|---|
| 1 | 84.9 |
| 5 | 63.5 |
| 9 | 38.6 |
| 10 | 77.8 |
| 11 | 81.1 |
| 12 | 96.5 |
| 16 | 49.9 |
| 17 | 82.1 |
| 18 | 90.9 |
| 24 | 73.0 |
| 29 | 39.3 |
| 31 | 89.9 |
| 32 | 59.1 |
| 35 | 38.0 |
| 37 | 65.4 |
| 38 | 55.9 |
| 39 | 40.8 |
| 52 | 92.4 |

## Claims

**1.** Substituted pyrrolidine compounds of general formula (I), 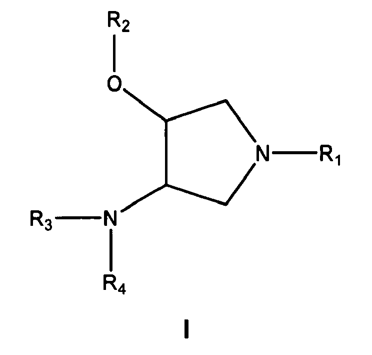 wherein
R¹ represents a hydrogen atom, an unbranched or branched, saturated, optionally at least mono-substituted C₁₋₆-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, optionally at least mono-substituted alkyl-aryl group in which the aryl group may be optionally at least mono-substituted and/or condensed with a mono- or polycyclic ring system; a branched or unbranched, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a hydrogen atom; a substituted or unsubstituted aliphatic group; a (C=O)-R⁸ group; a benzyl group which is optionally substituted by one or more substituents independently selected from the group consisting of F, Cl, Br, I, NH₂, SH, OH;
R³ and R⁴, equal or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
or
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵ and R⁶ represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁷ and R^{7a}, identical or different, represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁸ represents a hydrogen atom; a branched or unbranched, saturated or unsaturated, unsubstituted aliphatic group;
R⁹, R¹⁰ and R¹¹ represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R¹² and R^{12a}, identical or different, represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
with the provisos that
if R² represents a hydrogen atom;
and R¹ represents a hydrogen atom or a condensed aryl group;
R³ and R⁴ may not form together with their bridging nitrogen atom a triazole group;
if R² represents a hydrogen atom;
and R¹ represents a hydrogen atom; a substituted C₁₋₂ alkyl group; a condensed aryl group; a benzyl group;
R³ and R⁴ may not form together with their bridging nitrogen atom a substituted, unsaturated, condensed ring system containing at least 4 nitrogen atoms as ring members; an unsaturated, (C=O)-substituted, uncondensed, 6-membered ring system in which one additonal carbon atom is replaced by nitrogen.
if R² represents a hydrogen atom;
and R³ and R⁴ form together with their bridging nitrogen atom a pyrrolidine ring, a piperidine ring, a piperazine ring or a morpholine ring;
R¹ may not represent a hydrogen atom; a methyl group; an unsaturated tert-butyl group; a (C=O)-substituted alkyl-heteroaryl group;
if R² represents a hydrogen atom;
and R¹ does not represent a benzyl group;
and either R³ or R⁴ represents a hydrogen atom;
either R³ or R⁴ may not represent a substituted alkyl-heteroaryl group; an alkyl-aryl group; a substituted heteroaryl group; an unsubstituted methyl group; a substituted alkyl group; an aryl group; a cyclohexyl group; an alkyl-cycloalkyl group wherein the cycloalkyl group containins at least one heteroatom;
if R² represents a hydrogen atom;
and R¹ represents an alkyl group;
and either R³ or R⁴ represents a hydrogen atom;
the other of R³ or R⁴ may not represent a substituted or unsubstituted C₁₋₂ alkyl group; a branched C₁₋₄ alkyl group; a substituted or unsubstituted C₃₋₆ cycloalkyl group in which optionally at least one carbon atom is optionally replaced by N, S or O; a cycloheptyl group;
if R² represents a hydrogen atom;
and R¹ represents an alkyl group;
either R³ or R⁴ may not represent a cyclohexyl group while the other represents at the same time an alkyl-aryl group; or either R³ or R⁴ may not represent an alkyl group while the other represents at the same time an alkyl-aryl group;
if R² represents a hydrogen atom;
and R¹ represents a benzhydryl group;
and either R³ or R⁴ represents a hydrogen atom;
either R³ or R⁴ may not represent a a (C=O)-substituted alkyl-aryl group; a (C=O)-substituted aryl group;
if R² represents a hydrogen atom;
and R¹ represents benzyl;
and either R³ or R⁴ represents a hydrogen atom;
the other of R³ or R⁴ may not represent a substituted or unsubstituted C₁₋₂ alkyl group; an unsubstituted benzyl group; a C₃₋₈ substituted alkyl group; a substituted alkyl-aryl group;
or
R³ or R⁴, together with their bridging nitrogen atom may not form a a tetrahydropyrimidine ring; a triazolo pyrimidine ring;
if R² represents a benzyl group;
and either R³ or R⁴ represents a hydrogen atom
either R³ or R⁴ may not represent a (C=O)-substituted alkyl-aryl group;
if R² represents a C₁₋₃ alkyl group;
and either R³ or R⁴ represents a hydrogen atom
either R³ or R⁴ may not represent an unsubstituted C₁₋₂ alkyl group; a substituted alkyl group; a substituted or unsubstituted aryl group; a substituted heteroaryl group; a (C=O)-substituted alkyl-aryl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**2.** Compounds according to claim 1, **characterized in that** R¹ represents a hydrogen atom, an unbranched or branched, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom; an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
or
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.
Compounds according to claim 1, **characterized in that** R¹ represents a hydrogen atom, an unbranched or branched, saturated, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=0)-NR⁷R^{7a} group;
R² represents a linear, substituted or unsubstituted C₁₋₆ alkyl group; or a benzyl group which is optionally substituted by one or more substituents independently selected from the group consisting of F, Cl, Br, I, NH₂, SH,
OH;
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-atkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.
Compounds according to claim 1, **characterized in that** R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group; a branched or unbranched, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl group; or a benzyl group which is optionally substituted by one or more substituents independently selected from the group consisting of F, Cl, Br, I, NH₂, SH, OH;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group; an unsubstituted heteroaryl group; a saturated or unsaturated, optionally at least mono-substituted alkyl-cycloalkyl group; an unsubstituted alkyl-aryl group; a saturated or unsaturated, optionally at least mono-substituted alkyl-heteroaryl group; with the condition that R³ and R⁴ may not at the same time represent a hydrogen atom;
R⁵, R⁶, R⁷ and R^{7a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**5.** Compounds according to claim 1, **characterized in that** R¹ represents an unbranched or branched, optionally at least mono-substituted C₂₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated unsubstituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; a (C=O)-NR⁷R^{7a} group;
R² represents a hydrogen atom;
R³ and R⁴ together with their bridging nitrogen atom form
1) a ring system A in which the ring containing the nitrogen atom is saturated, but may be condensed with an unsaturated ring, comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**6.** Compounds according to claim 1, **characterized in that** R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
R⁵, R⁶, R⁷ and R^{7a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**7.** Compounds according to any of claims 1 or 2, **characterized in that** R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆-alkyl-cycloalkyl group wherein the C₁₋₆-alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom, an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group; a branched or unbranched, optionally at least mono-substituted, optionally at least one heteroatom as ring member containing C₁₋₆-alkyl-cycloalkyl group; an optionally at least mono-substituted aryl group; an optionally at least mono-substituted heteroaryl group; a branched or unbranched, optionally at least mono-substituted C₁₋₆-alkyl-aryl group; a branched or unbranched, saturated or unsaturated, optionally at least mono-substituted C₁₋₆-alkyl-heteroaryl group; with the condition that R³ and R⁴ may at the same time not represent a hydrogen atom;
or
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-_{NR}¹²_{R}^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**8.** Compounds according to claims 1 or 3, **characterized in that** R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a group; a (SO₂)-R⁶ group; or a (C=O)-NR⁷R^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or O and which is optionally at least mono-substituted by F, CI, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**9.** Compounds according to claims 1 or 4, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a (C=O)-R⁵ group; a (SO₂)-R⁶ group; or a (C=0)-N_{R}⁷_{R}^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; an optionally at least mono-substituted C₁₋₆ alkyl-cycloalkyl group; an unsubstituted C₁₋₆ alkyl-aryl group; an unsubstituted C₁₋₆ alkyl-heteroaryl group; with the condition that R³ and R⁴ may not at the same time represent a hydrogen atom;
R⁵, R⁶, R⁷ and R^{7a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**10.** Compounds according to claims 1 or 5, **characterized in that** R¹ represents an unbranched or branched, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a hydrogen atom;
R³ and R⁴ together with their bridging nitrogen atom form
1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=0)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**11.** Compounds according to any of claims 1 or 6, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a branched or unbranched C₁₋₆-alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or Cₗ₋₆-alkoxy; a benzhydryl group which is optionally substituted by one or more substituents independently selected from the group consisting of F, CI, Br, I, NH₂, SH, OH, SO₂, CF₃, CN, nitro, -SO₂NH₂ or C₁₋₆-alkoxy; a (C=O)-R⁵ group; a (S0₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
R⁵, R⁶, R⁷ and R^{7a} have the meaning according to claim 1;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**12.** Compounds according to any of claims 1, 2 or 7, **characterized in that** R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, - SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a (C=O)-R⁸ group, with R⁸ representing a hydrogen atom; a methyl group; an ethyl group; a propyl group; a butyl group; a pentyl group; a hexyl group;
R³ and R⁴, equal or different, represent a hydrogen atom, an unbranched or branched C₁₋₈-alkyl group, which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a saturated or unsaturated, optionally at least one heteroatom as ring member containing cycloalkyl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, optionally at least one heteroatom as ring member containing C₁₋₆-alkyl-cycloalkyl group which is optionally substituted by one or more substituents independently selected from the group consisting of F, Cl, Br, I, NH₂, SH, OH, S0₂, CF₃, CN, nitro,-S0₂NH₂ or C₁₋₆-alkoxy; an aryl group which is optionally substituted by one or more substituents independently selected from the group consisting of F, Cl, Br, I, NH₂, SH, OH, S0₂, CF₃, CN, CO-OH, CO-O-CH₃, O-CH₃, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; with the condition that R³ and R⁴ may at the same time not represent a hydrogen atom;
or
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
2) an A-X-B group,
wherein
A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, CI, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, CI, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**13.** Compounds according to claims 1, 3 or 8, **characterized in that** R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, S0₂, carboxy, amido, cyano, carbamyl, nitro, -S0₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a group; a (SO₂)-R⁶ group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=0)-OR¹⁰ group; a CH₂-(C=O)-O-R¹¹ group; a (SO₂)-R¹¹ group; a NH-R¹¹ group;
2) an A-X-B group,
wherein
A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁-₆-alkyl-heteroaryl group; a C₁-₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**14.** Compounds according to claims 1, 4 or 9, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a methyl, ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a C₁₋₆ alkyl-cycloalkyl group which is optionally substituted by one or more substituents independently selected from the group consisting of F, Cl, Br, I, NH₂, SH, OH, CF₃; an unsubstituted C₁₋₆ alkyl-aryl group; an unsubstituted C₁₋₆ alkyl-heteroaryl group; with the condition that R³ and R⁴ may not at the same time represent a hydrogen atom;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, S0₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**15.** Compounds according to claims 1, 5 or 10, **characterized in that** R¹ represents an unbranched or branched, saturated, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro,-SO₂NH₂ or C₁₋₆-alkoxy; a benzhydryl group which is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a (S0₂)-R⁶ group;
R² represents a hydrogen atom;
R³ and R⁴ together with their bridging nitrogen atom form
1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-¹¹ group; a (SO₂)-R¹¹ group; a NH-R¹¹ group;
2) an A-X-B group,
wherein
A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
3) an A-X-B-Y-C group,
wherein
A represents a 4 to 18 membered ring system in which the ring containing the nitrogen atom is saturated, but may be condensed with an unsaturated ring comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 6membered ring system, in which at least 1 carbon atom is optionally replaced by N or 0 and which is optionally at least mono-substituted by F, CI, Br, I, NH₂, SH, OH, oxo, or CF₃;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, CI, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**16.** Compounds according to claims 1, 6 or 11, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro,-S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**17.** Compounds according to any of claims 1, 2, 7 or 12, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a hydrogen atom; a C₃₋₈ alkyl group which is optionally substituted by one or more substituents independently selected from the group consisting of F, CI, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN; with the condition that R³ and R⁴ may not at the same time represent a hydrogen atom;
or
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a saturated or unsaturated 4 to 18 membered ring system comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**18.** Compounds according to any of claims 1, 3, 8 or 13, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a saturated or unsaturated 4 to 18 membered ring system comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**19.** Compounds according to any of claims 1, 4, 9 or 14, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, optionally at least mono-substituted C₃₋₈ aliphatic group with substituents independently selected from the group consisting of F, Cl, Br, I, NH₂, SH, OH, CF₃, CN, nitro; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a saturated or unsaturated, optionally at least mono-substituted C₁₋₆ alkyl-cycloalkyl group; a saturated or unsaturated, unsubstituted C₁₋₆ alkyl-aryl group; a saturated or unsaturated, unsubstituted C₁₋₆ alkyl-heteroaryl group; with the condition that R³ and R⁴ may not at the same time represent a hydrogen atom;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**20.** Compounds according to any of claims 1, 5, 10 or 15, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ and R⁴ together with their bridging nitrogen atom form
1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N or 0 and which is optionally at least mono-substituted by F, Cl, Br, l, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
3) an A-X-B-Y-C group,
wherein
A represents a 4 to 18 membered ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**21.** Compounds according to any of claims 1, 6, 11 or 16, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**22.** Compounds according to any of claims 1, 2, 7, 12 or 17, **characterized in that** R¹ represents a benzyl group or a (SO₂)-R⁶ group with R⁶ being an aryl group;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN;
or
R³ and R⁴ together with their bridging nitrogen atom form
1) a ring system A, comprising: 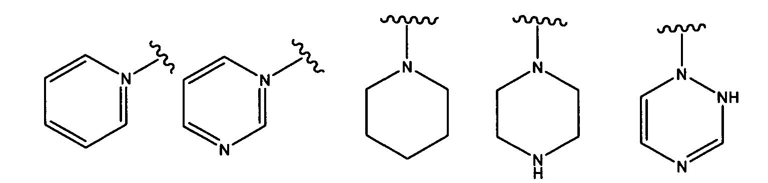 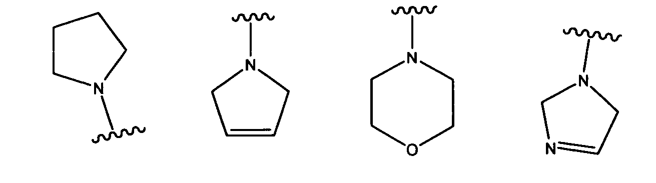 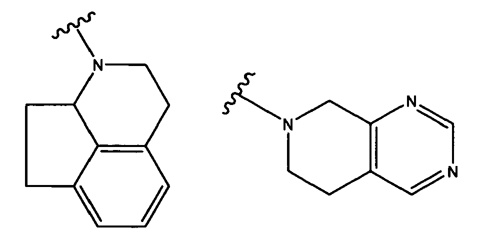 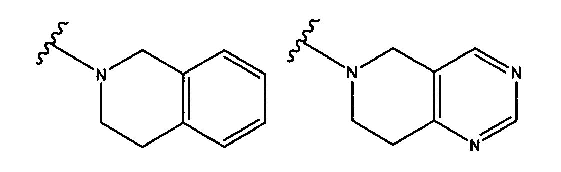 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a ring system comprising: 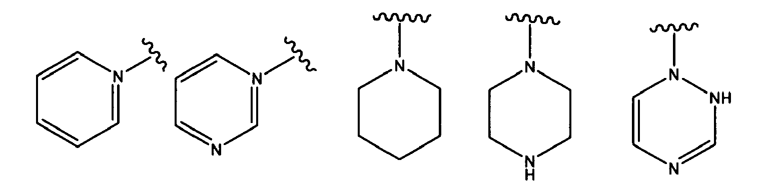 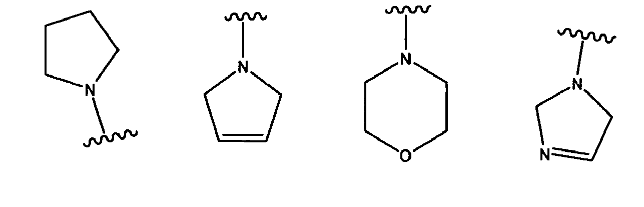 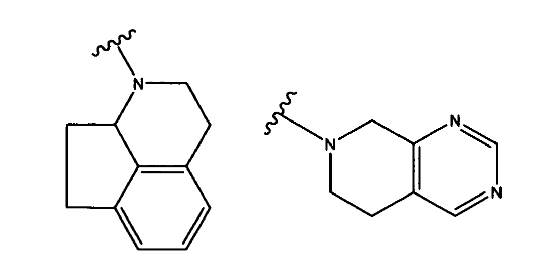 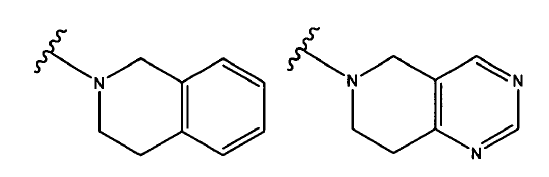 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear optionally substituted C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 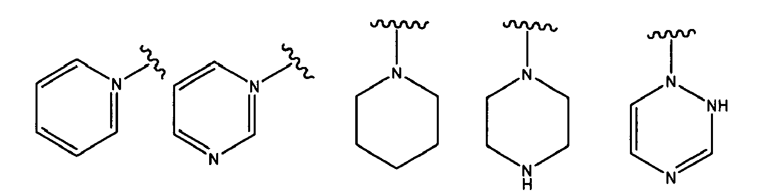 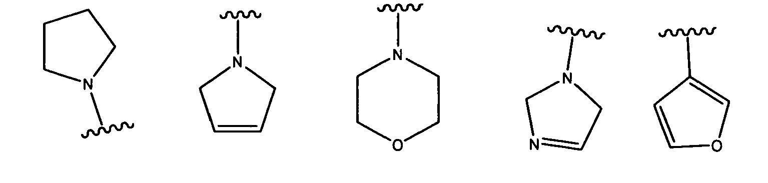 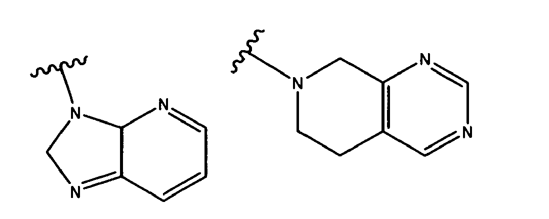 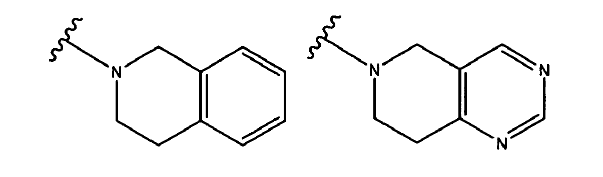 which is optionally at least mono-substituted by F, Cl, Br, I, NH₂, SH, OH, CF₃, a C₁₋₆-aliphatic group; a C₁₋₆-alkoxy group;
3) an A-X-B-Y-C group,
wherein
A represents a ring system comprising: 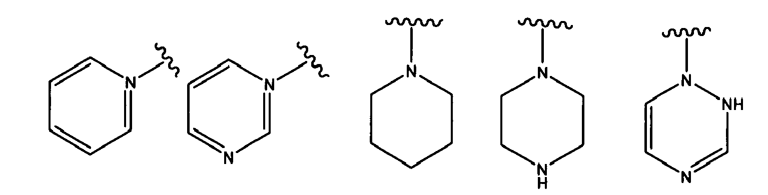 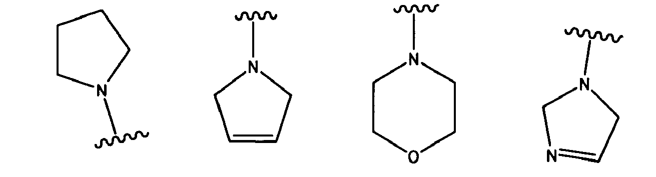 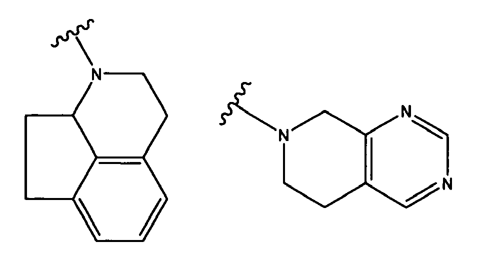 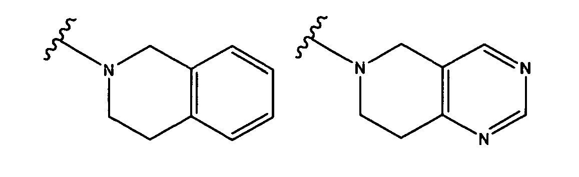 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 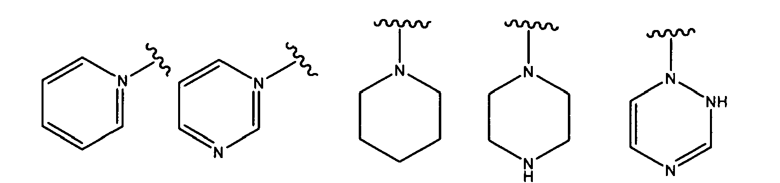 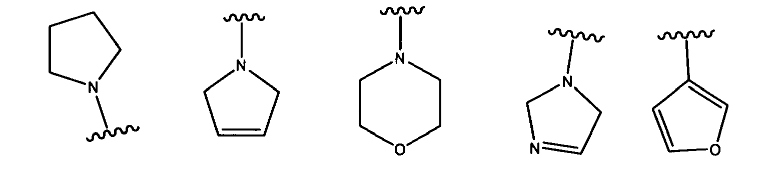 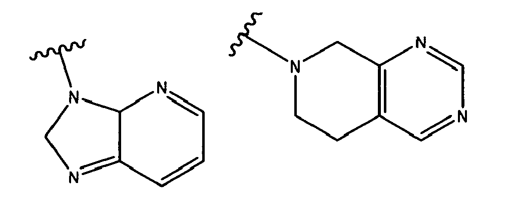 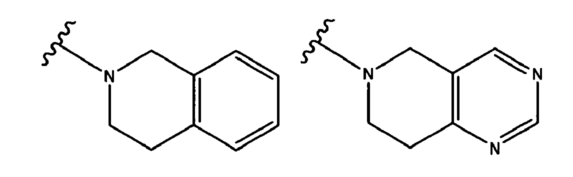 which is optionally at least mono-substituted by F, Cl, Br, I, NH₂, SH, OH, CF₃, a C₁₋₆-aliphatic group; a C₁₋₆-alkoxy group;
Y represents a bond, a -NH- group, a linear C₁₋₂-aliphatic group;
C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**23.** Compounds according to any of claims 1, 3, 8, 13 or 18, **characterized in that** R¹ represents a benzyl group or a (SO₂)-R⁶ group with R⁶ being an aryl group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a ring system A, comprising: 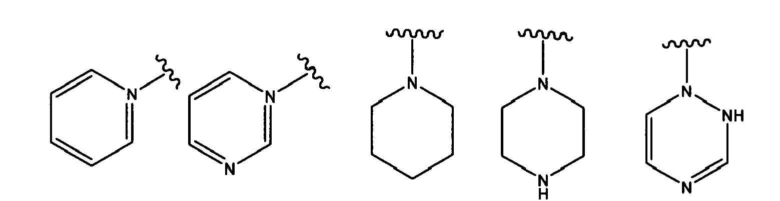 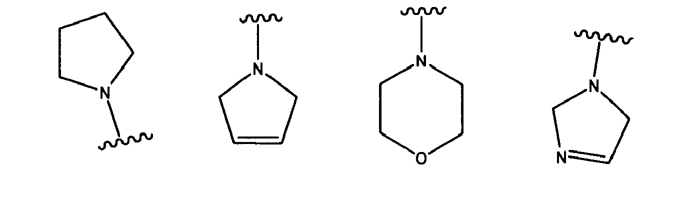 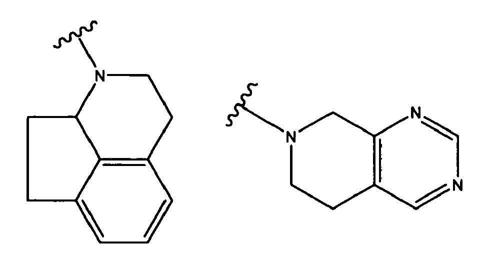 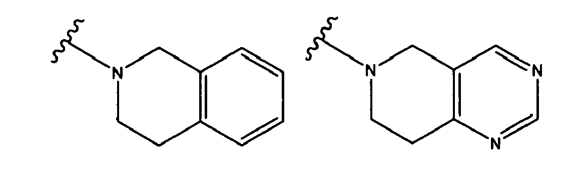 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a ring system comprising: 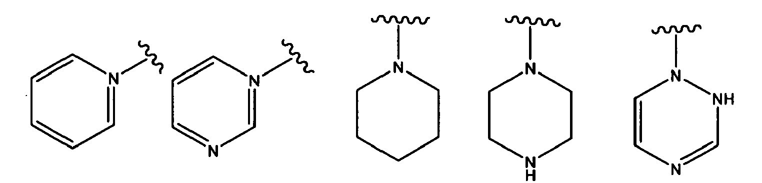 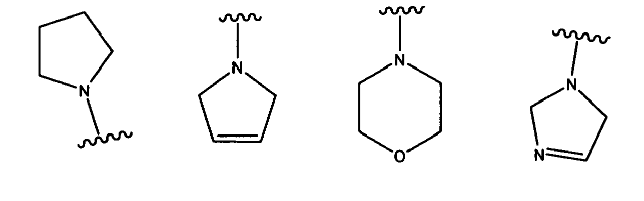 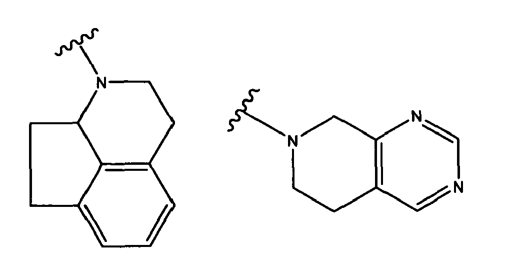 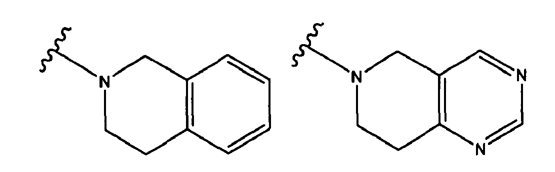 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -0-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 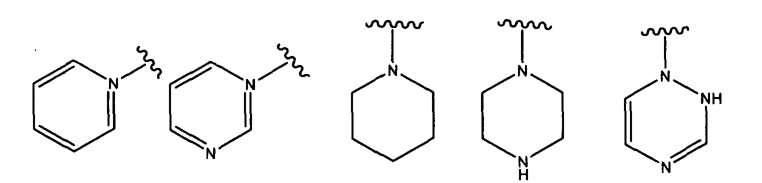 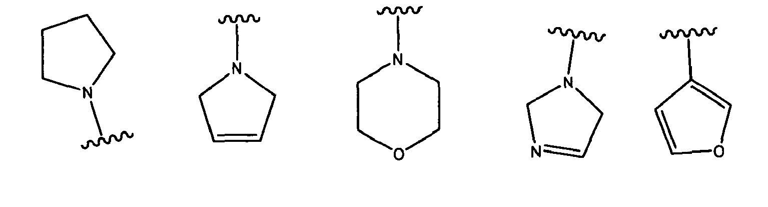 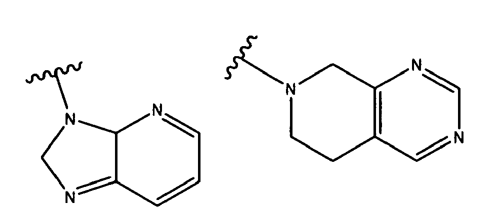 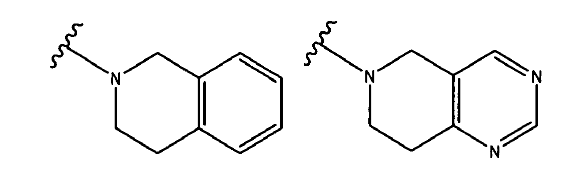 which is optionally at least mono-substituted by F, Cl, Br, I, NH₂, SH, OH, CF₃, a C₁₋₆-aliphatic group; a C₁₋₆-alkoxy group;
3) an A-X-B-Y-C group,
wherein
A represents a ring system comprising: 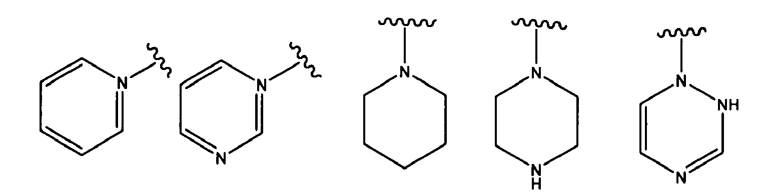 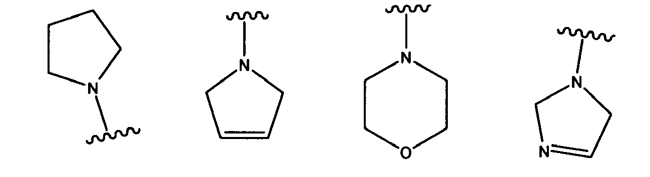 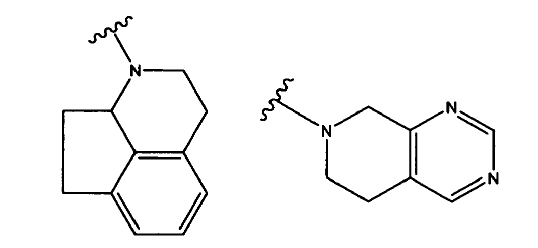 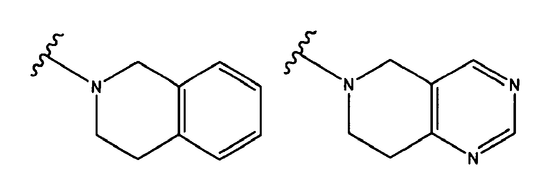 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 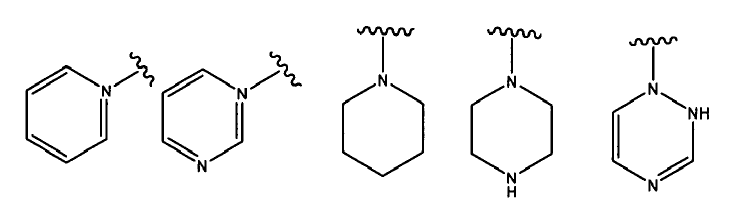 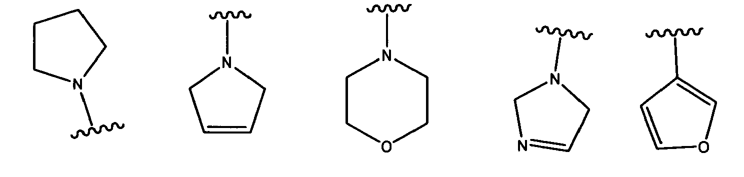 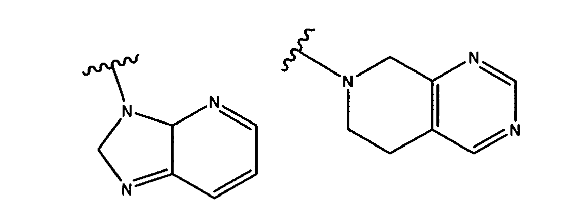 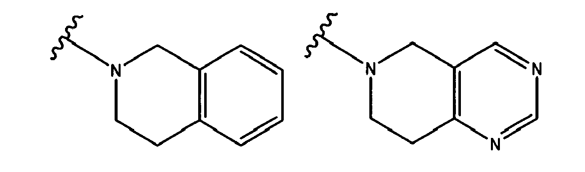 which is optionally at least mono-substituted by F, Cl, Br, I, NH₂, SH, OH, CF₃, a C₁₋₆-aliphatic group; a C₁₋₆-alkoxy group;
Y represents a bond, a -NH- group, a linear C₁₋₂ alkyl group;
C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**24.** Compounds according to any of claims 1, 5, 10, 15 or 20, **characterized in that** R¹ represents a benzyl group or a (SO₂)-R⁶ group with R⁶ being an aryl group;
R² represents a hydrogen atom;
R³ and R⁴ together with their bridging nitrogen atom form
1) a ring system A, comprising: 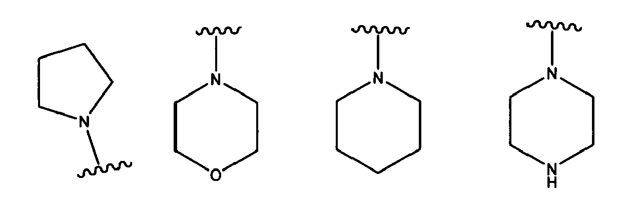 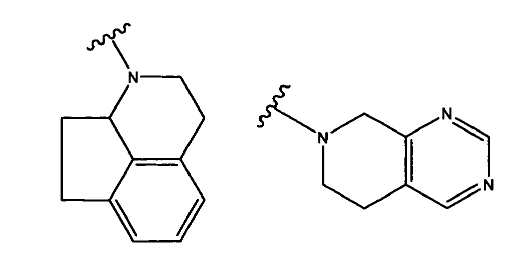 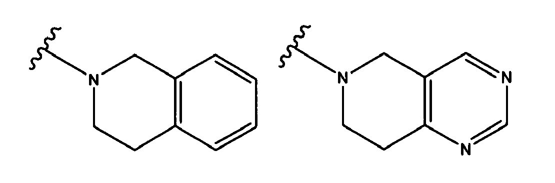 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a ring system comprising: 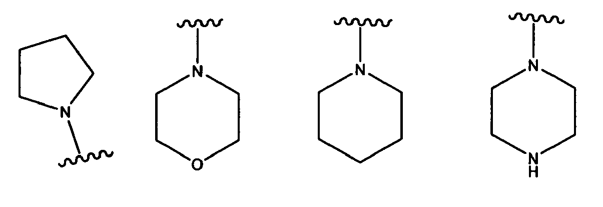 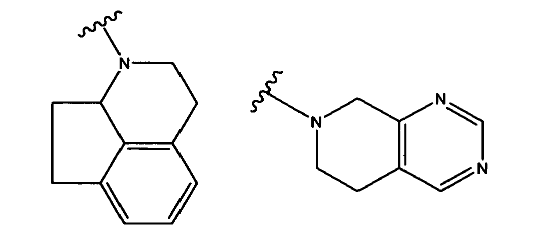 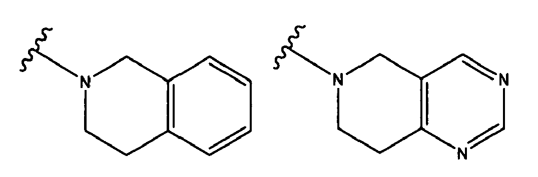 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 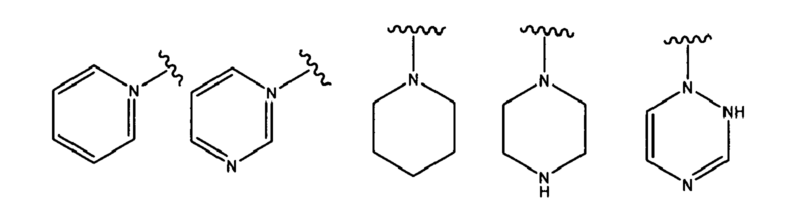 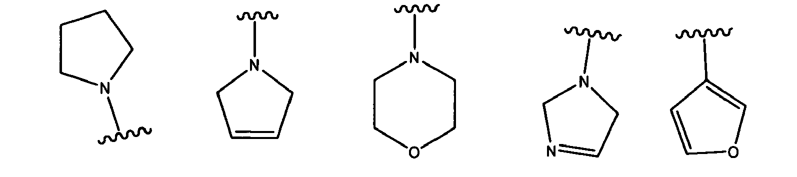 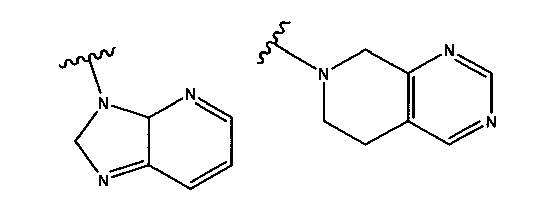 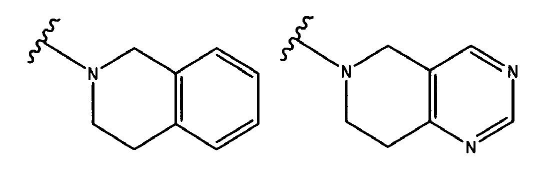 which is optionally at least mono-substituted by F, Cl, Br, I, NH₂, SH, OH, CF₃, a C₁₋₆-aliphatic group; a C₁₋₆-alkoxy group;
3) an A-X-B-Y-C group,
wherein
A represents a ring system comprising: 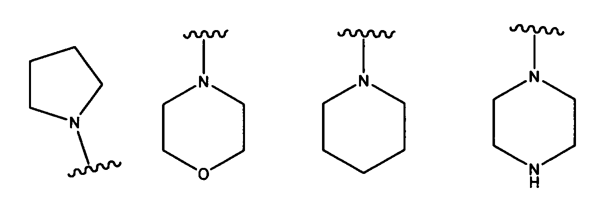 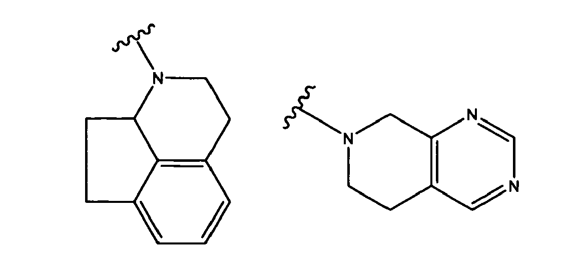 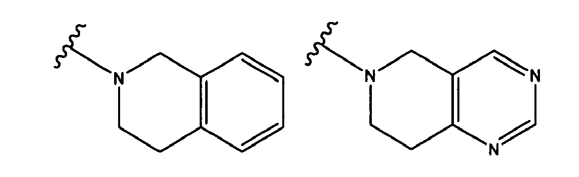 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 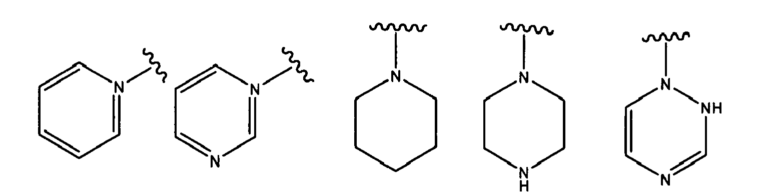 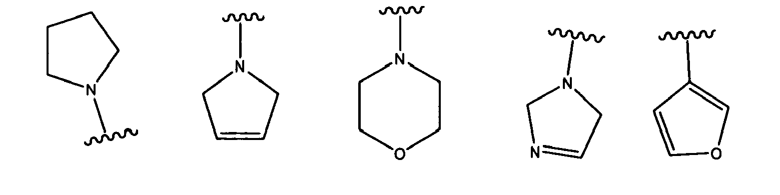 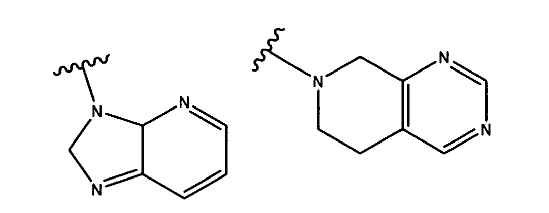 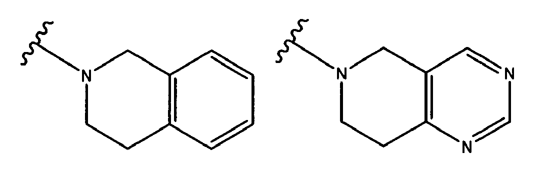 which is optionally at least mono-substituted by F, Cl, Br, I, NH₂, SH, OH, CF₃, a C₁₋₆-aliphatic group; a C₁₋₆ alkoxy group;
Y represents a bond, a -NH- group, a linear C₁₋₂-alkyl group;
C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

**25.** Compounds according to any of claims 1 to 24 selected from the group consisting of
[1] (3,4-trans)-1-benzyl-4-(3,4-dihydroisoquinolin-2(1H)-yl)pyrrolidine-3-ol,
[2] (3,4-trans)-1-benzyl-4-morpholinopyrrolidine-3-ol,
[3] (3,4-trans)-1-benzyl-4-(4-(2-methyl-3H-imidazo[4,5-b]pyridin-3-yl)piperidin-1-yl)pyrrolidine-3-ol,
[4] (3,4-trans)-1-benzyl-4-(3-(6,7-dimethoxy-3,4-dihydroisoquinolin-2(1H)-yl)propyl amino)pyrrolidine-3-ol,
[5] (3,4-trans)-1-benzyl-4-(4-((3,4-dihydroisoquinolin-2(1 H)-yl)methyl)piperidin-1-yl)pyrrolidine-3-ol,
[6] 1-(4-(6-((3,4-trans)-1-benzyl-4-hydroxypyrrolidine-3-yl)-5,6,7,8-tetrahydropyrido [4,3-d]pyrimidin-2-ylamino)phenyl)ethanone,
[7] 1-(1-((3,4-trans)-1-benzyl-4-hydroxypyrrolidine-3-yl)piperidin-4-yl)-1H-benzo[d][1,3]oxazin-2(4H)-one,
[8] (3,4-trans)-1-benzyl-4-(2-(4-phenoxyphenylamino)-7,8-dihydropyrido[4,3-d]pyrimidin-6(5H)-yl)pyrrolidine-3-ol,
[9] (3,4-trans)-1-benzyl-4-(3-(3,4-dihydroisoquinolin-2(1H)-yl)propylamino)pyrrolidine -3-ol,
[10] (3,4-trans)-1-benzyl-4-(4-(4-methoxypyrimidin-2-yl)piperazin-1-yl)pyrrolidine-3-ol,
[11] (3,4-trans)-1-benzyl-4-(2,3,8,8a-tetrahydrocyclopenta[ij]isoquinolin-1 (7H)-yl) pyrrolidine-3-ol,
[12] tert-butyl-4-((3,4-trans)-l -benzyl-4-hydroxypyrrolidine-3-yl)piperazine-1-carboxylate,
[13] tert-butyl 4-((3,4-trans)-4-hydroxy-1-(phenylsulfonyl)pyrrolidine-3-yl)piperazine-1-carboxylate,
[14] (3,4-trans)-4-morpholino-1-(phenylsulfonyl)pyrrolidine-3-ol,
[15] (3,4-trans)-4-(3,4-dihydroisoquinolin-2(1H)-yl)-1-(phenylsulfonyl)pyrrolidine-3-ol,
[16] (3,4-trans)-4-(3,4-dihydroisoquinolin-2(1 H)-yl)-1 ((S)-1 - phenylethyl)pyrrolidine-3-ol,
[17] (3,4-trans)-1-benzyl-4-(4-benzylpiperazin-1 -yl)pyrrolidine-3-ol,
[18] (3,4-trans)-1-benzyl-4-(4-phenylpiperazin-1-yl)pyrrolidine-3-ol,
[19] Ethyl 2-(4-((3,4-trans)-1-benzyl-4-hydroxypyrrolidine-3-yl)piperazin-1-yl)acetate,
[20] (4-((3,4-trans)-1-benzyl-4-hydroxypyrrolidine-3-yl)piperazin-1-yl)(phenyl) methanone,
[21] Ethyl 4-((3,4-trans)-1-benzyl-4-hydroxypyrrotidine-3-yl)piperazine-1-carboxilate,
[22] tert-butyl 4-((3,4-trans)-1-(4-fluorobenzyl)-4-hydroxypyrrolidine-3-yl)piperazine-1-carboxylate,
[23] (3,4-trans)-1-benzyl-4-(4-methylpiperazin-1-yl)pyrrolidine-3-ol,
[24] tert-butyl-4-(3,4-trans)-4-acetoxy-1-benzylpyrrolidine-3-yl)piperazine-1-carboxylate,
[25] (3,4-trans)-1-benzyl-4-(piperazin-1-yl)pyrrolidine-3-yl acetate,
[26] tert-butyl 4-((3,4-trans)-4-acetoxypyrrolidine-3-yl)piperazine-1-carboxylate,
[27] trans 1-Benzyl-4-(4-(2-phenyl-phenylsulfonyl)piperazin-1-yl)pyrrolidin-3-yl acetate,
[28] trans 1-Benzyl-4-(4-(2-phenyl-phenylsulfonyl)piperazin-1-yl)pyrrolidin-3-ol dihydrochloride,
[29] (3,4-trans)-1-benzyl-4-(phenylamino)pyrrolidin-3-ol,
[30] (3,4-trans)-1-benzyl-4-(benzylamino)pyrrolidin-3-ol,
[31] (3R,4R)-1-benzyl-4-(octylamino)pyrrolidin-3-ol,
[32] (3,4-trans)-1-benzyl-4-(4-methoxybenzylamino)pyrrolidin-3-ol,
[33] (3,4-trans)-1-benzyl-4-(butylamino)pyrrolidin-3-ol,
[34] (3R,4R)-1-benzyl-4-(4-(4-chloro-2,5-dimethylphenylsulfonyl)piperazin-1-yl)pyrrolidin-3-yl acetate,
[35] (3R,4R)-1-benzyl-4-(4-tosylpiperazin-1-yl)pyrrolidin-3-yl acetate,
[36] (3,4-trans)-1-benzyl-4-(butylamino)pyrrolidin-3-ol,
[37] (3R,4R)-1-benzyl-4-(4-methoxyphenylamino)pyrrolidin-3-ol,
[38] Methyl 4-((3,4-trans)-1-benzyl-4-hydroxypyrrolidin-3-ylamino)benzoate,
[39] (3R,4R)-1-benzyl-4-(4-(naphthalen-1-ylsulfonyl)piperazin-1-yl)pyrrolidin-3-yl acetate,
[40] (3R,4R)-1-benzyl-4-(4-(2-chlorophenylsulfonyl)piperazin-1-yl)pyrrolidin-3-ol,
[41] (3R,4R)-1-benzyl-4-(4-(2-bromophenylsulfonyl)piperazin-1-yl)pyrrolidin-3-yl acetate,
[42] (3R,4R)-1-benzyl-4-(4-(2-bromophenylsulfonyl)piperazin-1-yl)pyrrolidin-3-ol,
[43] (3R,4R)-1-benzyl-4-(4-(5-fluoro-2-methylphenylsulfonyl)piperazin-1-yl)pyrrolidin-3-yl acetate,
[44] (3R,4R)-1-benzyl-4-(4-(5-fluoro-2-methylphenylsulfonyl)piperazin-1-yl)pyrrolidin-3-ol,
[45] (3R,4R)-1-benzyl-4-(4-(phenylsulfonyl)piperazin-1-yl)pyrrolidin-3-yl acetate,
[46] (3R,4R)-1-benzyl-4-(4-(phenylsulfonyl)piperazin-1-yl)pyrrolidin-3-ol,
[47] (3R,4R)-1-benzyl-4-(4-(2-fluorophenylsulfonyl)piperazin-1-yl)pyrrolidin-3-yl acetate,
[48] (3R,4R)-1-benzyl-4-(4-(2-fluorophenylsulfonyl)piperazin-1-yl)pyrrolidin-3-ol,
[49] (3R,4R)-1-benzyl-4-(4-(naphthalen-1-ylsulfonyl)piperazin-1-yl)pyrrolidin-3-ol,
[50] (3R,4R)-1-benzyl-4-(4-(4-chloro-2,5-dimethylphenylsulfonyl)piperazin-1-yl)pyrrolidin-3-ol,
[51] (3R,4R)-1-benzyl-4-(4-tosylpiperazin-1-yl)pyrrolidin-3-ol,
[52] (3R,4R)-1-benzyl-4-(4-(2-chlorophenylsulfonyl)piperazin-1-yl)pyrrolidin-3-yl acetate,
optionally in form of a corresponding salt, or a corresponding solvate.

**26.** A process for the manufacture of substituted pyrrolidine compounds of general formula (I) according to one or more of claims 1 to 25, **characterized in that** at least one pyrroline compound of general formula (II), 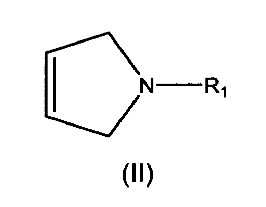 wherein R¹ has the meaning given above, is reacted with a per-acid, preferably MCPBA to form a compound of general formula (III), 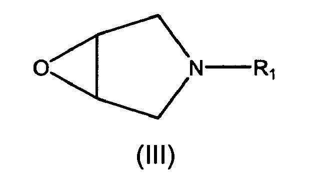 wherein R¹ has the meaning given above, followed by reacting the epoxyo-pyrroline compound of formula III with a compound of formula (IV) 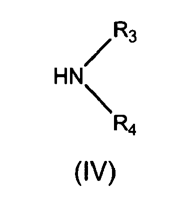 together with LiClO₄, ACN at preferably 90° C to give a compound of formula I, in which R² is H; alternatively followed by reacting the pyrrolidine compound of formula I with a base to give a compound of formula I, in which R² has the meaning according to one or more of claims 1 to 22.

**27.** A Medicament comprising at least one substituted pyrrolidine compound of general formula (I) according to one or more of claims 1 to 25, 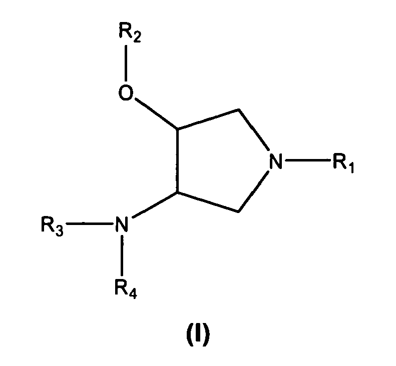 wherein
R¹ represents a hydrogen atom, an unbranched or branched, saturated, optionally at least mono-substituted C₁₋₆-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a hydrogen atom; a substituted or unsubstituted aliphatic group; a (C=O)-R⁸ group; a benzyl group which is optionally substituted by one or more substituents independently selected from the group consisting of F, Cl, Br, I, NH₂, SH, OH;
R³ and R⁴, equal or different, represent a hydrogen atom; an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
or
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁-₆-alkoxy group; a (C=O)-_{R}⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵ and R⁶ represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁷ and R^{7a}, identical or different, represent a hydrogen atom, an unbranched or branched, saturated or unsaturated, optionally at least mono-substituted aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R⁸ represents a hydrogen atom; a branched or unbranched, saturated or unsaturated, unsubstituted aliphatic group;
R⁹, R¹⁰ and R¹¹ represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
R¹² and R^{12a}, identical or different, represent a hydrogen atom, an unbranched or branched, saturated or unsaturated, optionally at least mono-substituted aliphatic radical; a branched or unbranched, saturated or unsaturated, optionally at least mono-substituted, optionally at least one heteroatom as ring member containing alkyl-cycloalkyl group; a branched or unbranched, saturated or unsaturated, optionally at least mono-substituted alkyl-aryl group in which the aryl group may be condensed with a mono- or polycyclic ring system; a branched or unbranched, saturated or unsaturated, optionally at least mono-substituted alkyl-heteroaryl group in which the heteroaryl group may be condensed with a mono- or polycyclic ring system;
with the provisos that
if R² represents a hydrogen atom;
and R¹ represents a hydrogen atom or a condensed aryl group;
R³ and R⁴ may not form together with their bridging nitrogen atom a triazole group;
if R² represents a hydrogen atom;
and R¹ represents a hydrogen atom; a substituted C₁₋₂ alkyl group; a condensed aryl group; a benzyl group;
R³ and R⁴ may not form together with their bridging nitrogen atom a substituted, unsaturated, condensed ring system containing at least 4 nitrogen atoms as ring members; an unsaturated, (C=O)-substituted, uncondensed, 6-membered ring system in which two carbon atoms are replaced by nitrogen.
if R² represents a hydrogen atom;
and R³ and R⁴ form together with their bridging nitrogen atom a pyrrolidine ring, a piperidine ring, a piperazine ring or a morpholine ring;
R¹ may not represent a hydrogen atom; a methyl group; an unsaturated tert-butyl group; a (C=O)-substituted alkyl-heteroaryl group;
if R² represents a hydrogen atom;
and R¹ does not represent a benzyl group;
and either R³ or R⁴ represents a hydrogen atom;
either R³ or R⁴ may not represent a substituted alkyl-heteroaryl group; an alkyl-aryl group; a substituted heteroaryl group; an unsubstituted methyl group; a substituted alkyl group; an aryl group; a cyclohexyl group; an alkyl-cycloalkyl group wherein the cycloalkyl group containins at least one heteroatom;
if R² represents a hydrogen atom;
and R¹ represents an alkyl group;
and either R³ or R⁴ represents a hydrogen atom;
either R³ or R⁴ may not represent a substituted or unsubstituted C₁₋₂ alkyl group; a branched C₁₋₄ alkyl group; a substituted or unsubstituted C₃₋₆ cycloalkyl group in which optionally at least one carbon atom is optionally replaced by N, S or O; a cycloheptyl group;
if R² represents a hydrogen atom;
and R¹ represents an alkyl group;
either R³ or R⁴ may not represent a cyclohexyl group while the other represents at the same time an alkyl-aryl group; or either R³ or R⁴ may not represent an alkyl group while the other represents at the same time an alkyl-aryl group;
if R² represents a hydrogen atom;
and R¹ represents a benzhydryl group;
and either R³ or R⁴ represents a hydrogen atom;
either R³ or R⁴ may not represent a a (C=O)-substituted alkyl-aryl group; a (C=O)-substituted aryl group;
if R² represents a hydrogen atom;
and R¹ represents benzyl;
and either R³ or R⁴ represents a hydrogen atom;
either R³ or R⁴ may not represent a substituted or unsubstituted C₁₋₂ alkyl group; a substituted C₃₋₈ alkyl group; a substituted aryl group; a substituted alkyl-aryl group;
or
R³ or R⁴, together with their bridging nitrogen atom may not form a a tetrahydropyrimidine ring; a triazolo pyrimidine ring;
if R² represents a benzyl group;
and either R³ or R⁴ represents a hydrogen atom;
either R³ or R⁴ may not represent a (C=O)-substituted alkyl-aryl group;
if R² represents a C₁₋₃ alkyl group;
and either R³ or R⁴ represents a hydrogen atom
either R³ or R⁴ may not represent an unsubstituted C₁₋₂ alkyl group; a substituted alkyl group; a substituted or unsubstituted aryl group; a substituted heteroaryl group; a (C=O)-substituted alkyl-aryl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**28.** Medicament according to claim 27, **characterized in that** R¹ represents a hydrogen atom, an unbranched or branched, saturated, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom; an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated or unsaturated, optionally at least mono-substituted alkyl-aryl group in which the aryl group may be optionally at least mono-substituted and/or condensed with a mono- or polycyclic ring system; a branched or unbranched, saturated or unsaturated, optionally at least mono-substituted alkyl-heteroaryl group in which the heteroaryl group may be optionally at least mono-substituted and/or condensed with a mono- or polycyclic ring system; with the condition (proviso) that R³ and R⁴ may not at the same time represent a hydrogen atom;
or
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-atkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶ ,R⁷, R^{7a}, R⁹, R¹⁰, R¹¹, R¹² and R^{12a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**29.** Medicament according to claim 27, **characterized in that** R¹ represents a hydrogen atom, an unbranched or branched, saturated, optionally at least mono-substituted C₁₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl, group; or a benzyl, group which is optionally substituted by one or more substituents independently selected from the group consisting of F, Cl, Br, I, NH₂, SH, OH;
R³ and R⁴ together with their bridging nitrogen atom form
1) a saturated or unsaturated ring system A, comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹⁰ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵ R⁶, R⁷, R^{7a}, R⁹, R¹⁰, R¹¹, R¹² and R^{12a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**30.** Medicament according to claim 27, **characterized in that** R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group; a branched or unbranched, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a group; a (SO₂)-R⁶ group; or a (C=O)-NR⁷R^{7a} group;
R² represents a linear, substituted or unsubstituted C₁₋₆ alkyl group; or a benzyl group which is optionally substituted by one or more substituents independently selected from the group consisting of F, Cl, Br, I, NH₂, SH, OH;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group; an unsubstituted heteroaryl group; a saturated or unsaturated, optionally at least mono-substituted alkyl-cycloalkyl group; an unsubstituted alkyl-aryl group; a saturated or unsaturated, optionally at least mono-substituted alkyl-heteroaryl group; with the condition that R³ and R⁴ may not at the same time represent a hydrogen atom;
R⁵, R⁶, R⁷ and R^{7a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**31.** Medicament according to claim 27, **characterized in that** R¹ represents an unbranched or branched, optionally at least mono-substituted C₂₋₆-alkyl radical; a saturated or unsaturated, optionally at least mono-substituted, optionally at least one heteroatom as ring member containing cycloalkyl group, which may be condensed with an optionally at least mono-substituted mono- or polycyclic ring system; a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted aryl group which may be condensed with an optionally at least mono-substituted mono- or polycyclic ring system; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated unsubstituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; a (C=O)-NR⁷R^{7a} group;
R² represents a hydrogen atom;
R³ and R⁴ together with their bridging nitrogen atom form
1) a ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a ring system in which the ring containing the nitrogen atom is saturated, but may be condensed with an unsaturated ring, comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**32.** Medicament according to claim 27, **characterized in that** R¹ represents a branched or unbranched, saturated or unsaturated, optionally at least mono-substituted, optionally at least one heteroatom as ring member containing alkyl-cycloalkyl group in which the cycloalkyl group may be optionally at least mono-substituted; an optionally at least mono-substituted heteroaryl group which may be condensed with an optionally at least mono-substituted mono- or polycyclic ring system; a branched or unbranched, saturated, optionally at least mono-substituted alkyl-aryl group in which the aryl group may be optionally at least mono-substituted and/or condensed with a mono- or polycyclic ring system; a branched or unbranched, saturated, optionally at least mono-substituted alkyl-heteroaryl group in which the heteroaryl group may be optionally at least mono-substituted and/or condensed with a mono- or polycyclic ring system; an optionally at least mono-substituted benzhydryl group; a (C=O)-R⁵ group; a (SO₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted alkyl-aryl group; an unsubstituted C₁₋₆ alkyl-heteroaryl group;
R⁵, R⁶, R⁷ and R^{7a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**33.** Medicament according to any of claims 27 or 28, **characterized in that** R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a (SO₂)-R⁶ group; or a (C=O)-NR⁷R^{7a} group;
R² represents a (C=O)-R⁸ group, with R⁸ representing a hydrogen atom; a branched or unbranched, saturated or unsaturated, unsubstituted aliphatic group;
R³ and R⁴, equal or different, represent a hydrogen atom, an unbranched or branched, saturated or unsaturated, optionally at least mono-substituted C₁₋₈-aliphatic radical; a saturated or unsaturated, optionally at least mono-substituted, optionally at least one heteroatom as ring member containing cycloalkyl group; a branched or unbranched, optionally at least mono-substituted, optionally at least one heteroatom as ring member containing C₁₋₆-alkyl-cycloalkyl group; an optionally at least mono-substituted aryl group; an optionally at least mono-substituted heteroaryl group; a branched or unbranched, optionally at least mono-substituted C₁₋₆-alkyl-aryl group; a branched or unbranched, saturated or unsaturated, optionally at least mono-substituted C₁₋₆-alkyl-heteroaryl group; with the condition that R³ and R⁴ may at the same time not represent a hydrogen atom;
or
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**34.** Medicament according to claims 27 or 29, **characterized in that** R¹ represents a hydrogen atom, an unbranched or branched, saturated C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (C=O)-R⁵ group; a group; a (SO₂)-R⁶ group; or a (C=O)-NR⁷R^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a saturated or unsaturated ring system A, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁-₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a saturated or unsaturated ring system comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a saturated or unsaturated ring system comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵ R⁶, R⁷, R^{7a}, R⁹, R¹⁰, R¹¹, R¹² and R^{12a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**35.** Medicament according to claims 27 or 30, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a benzhydryl group which is optionally substituted by one or more substituents independently selected from the group consisting of F, Cl, Br, I, NH₂, SH, OH, S0₂, CF₃, CN, nitro, -SO₂NH₂ or C₁₋₆-alkoxy; a (C=O)-R⁵ group; a (S0₂)-R⁶ group; or a (C=0)-NR⁷R^{7a} group;
R² represents a C₁₋₆ alkyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; an optionally at least mono-substituted C₁₋₆ alkyl-cycloalkyl group; an unsubstituted C₁₋₆ alkyl-aryl group; an unsubstituted C₁₋₆ alkyl-heteroaryl group; with the condition that R³ and R⁴ may not at the same time represent a hydrogen atom;
R⁵, R⁶, R⁷ and R^{7a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**36.** Medicament according to claims 27 or 31, **characterized in that** R¹ represents an unbranched or branched, optionally at least mono-substituted C₁₋₆-alkyl radical which is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, S0₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆-alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or Cₗ₋₆-alkoxy; a (C=O)-R⁵ group; a (SO₂)-R⁶ group; or a (C=O)-NR⁷R^{7a} group;
R² represents a hydrogen atom;
R³ and R⁴ together with their bridging nitrogen atom form
1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
2) an A-X-B group,
wherein
A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
3) an A-X-B-Y-C group,
wherein
A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group; or a (C=O)-NR¹²R^{12a} group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group; or a (C=O)-NR¹²R^{12a} group;
R⁵, R⁶, R⁷, R^{7a}, R⁹, R¹⁰, R¹¹, R¹² and R^{12a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**37.** Medicament according to any of claims 27 or 32, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a (C=O)-R⁵ group; a (SO₂)-R⁶ group; or a (C=O)-NR⁷R^{7a} group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted allkyl-aryl group; an unsubstituted C₁₋₆ alkyl-heteroaryl group;
R⁵, R⁶, R⁷ and R^{7a} have the meaning according to claim 27;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**38.** Medicament according to any of claims 27, 28 or 33, **characterized in that** R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a (C=O)-R⁸ group, with R⁸ representing a hydrogen atom; a methyl group; an ethyl group; a propyl group; a butyl group; a pentyl group; a hexyl group;
R³ and R⁴, equal or different, represent a hydrogen atom, an unbranched or branched C₁₋₈-alkyl group, which is optionally substituted by one or more substituents independently selected from the group consisting of F, CI, Br, I, NH₂, SH, OH, SO₂, CF₃, CN, nitro, -SO₂NH₂ or C₁₋₆-alkoxy; a saturated or unsaturated, optionally at least one heteroatom as ring member containing cycloalkyl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, optionally at least one heteroatom as ring member containing C₁₋₆-alkyl-cycloalkyl group which is optionally substituted by one or more substituents independently selected from the group consisting of F, Cl, Br, I, NH₂, SH, OH, SO₂, CF₃, CN, nitro, - SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-aryl group which is optionally substituted by one or more substituents independently selected from the group consisting of F, Cl, Br, I, NH₂, SH, OH, SO₂, CF₃, CN, CO-OH, CO-O-CH₃, O-CH₃, nitro, -SO₂NH₂ or C₁₋₆-alkoxy; a branched or unbranched C₁₋₆-alkyl-heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; with the condition that R³ and R⁴ may at the same time not represent a hydrogen atom;
or
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
2) an A-X-B group,
wherein
A represents a saturated or unsaturated 4 to 18 membered ring system comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁-₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, S0₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, CI, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, CI, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**39.** Medicament according to any of claims 27, 29 or 34, **characterized in that** R¹ represents a hydrogen atom, an unbranched or branched C₁₋₆ alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, carboxy, amido, cyano, carbamyl, nitro, -SO₂NH₂ or C₁₋₆-alkoxy; an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated, C₁₋₆₋alkyl-heteroaryl group wherein the C₁₋₆₋alkyl and/or the heteroaryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least mono-substituted benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a group; a (SO₂)-R⁶ group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
2) an A-X-B group,
wherein
A represents a saturated or unsaturated 4 to 18 membered ring system comprising 1 to 3 optionally condensed rings, in which at least 1 carbon atom is optionally replaced by N, S or 0 and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
R⁶ represents a hydrogen atom; a C₁₋₆ alkyl group which is optionally at least mono-substituted by substituents independently selected from the group consisting of F, CI, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**40.** Medicament according to any of claims 27, 30 or 35, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a methyl, ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, unsubstituted C₃₋₈ aliphatic group; a saturated or unsaturated, optionally at least mono-substituted, optionally at least one heteroatom as ring member containing cycloalkyl group with substituents independently selected from the group consisting of F, Cl, Br, I, NH₂, SH, OH, CF₃, CN, nitro; an unsubstituted heteroaryl group; a C₁₋₆ alkyl-cycloalkyl group which is optionally substituted by one or more substituents independently selected from the group consisting of F, Cl, Br, I, NH₂, SH, OH, CF₃; an unsubstituted C₁₋₆ alkyl-aryl group; an unsubstituted C₁₋₆ alkyl-heteroaryl group; with the condition that R³ and R⁴ may not at the same time represent a hydrogen atom;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**41.** Medicament according to any of claims 27, 31 or 36, **characterized in that** R¹ represents an unbranched or branched, saturated, optionally at least mono-substituted C₁₋₆-alkyl radical which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an optionally at least one heteroatom as ring member containing C₁₋₆-alkyl-cycloalkyl group wherein the C₁₋₆-alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; an aryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁-₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, - S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ and R⁴ together with their bridging nitrogen atom form
1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
2) an A-X-B group,
wherein
A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
3) an A-X-B-Y-C group,
wherein
A represents a 4 to 18 membered ring system in which the ring containing the nitrogen atom is saturated, but may be condensed with an unsaturated ring comprising 1 to 3 optionally condensed rings, in which additionally at least 1 carbon atom is optionally replaced by N, S or 0 and which is optionally at least mono-substituted by F, Cl, Br, I, NH₂, SH, OH, CF₃, a C₁-₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH₂-(C=O)-O-R¹¹ group; a (SO₂)-R¹¹ group; a NH-R¹¹ group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkyl-aryl group; a C₁₋₆-alkyl-heteroaryl group; a C₁₋₆-alkoxy group; a (C=O)-R⁹ group; a (C=O)-OR¹⁰ group; a CH2-(C=O)-O-R¹¹ group; a (SO₂)-R¹¹ group; a NH-R¹¹ group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 6membered ring system, in which at least 1 carbon atom is optionally replaced by N or O and which is optionally at least mono-substituted by F, Cl, Br, I, NH₂, SH, OH, oxo, or CF₃;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
R⁹, R¹⁰ and R¹¹ represent a hydrogen atom, an unbranched or branched C₁₋₆ alkyl group which is optionally at least mono-substituted by substituents independently selected from the group consisting of F, Cl, Br, I, NH₂, SH, OH, CF₃; an alkyl-cycloalkyl group which is optionally at least mono-substituted by substituents independently selected from the group consisting of F, CI, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**42.** Medicament according to any of claims 27, 32 or 37, **characterized in that** R¹ represents an optionally at least one heteroatom as ring member containing C₁₋₆₋alkyl-cycloalkyl group wherein the C₁₋₆₋alkyl and/or the cycloalkyl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a heteroaryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a branched or unbranched, saturated C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched, saturated C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, - SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted alkyl-aryl group; an unsubstituted C₁₋₆ alkyl-heteroaryl group;
R⁶ represents a hydrogen atom; a C₁₋₆ alkyl group which is optionally at least mono-substituted by substituents independently selected from the group consisting of F, Cl, Br, I, NH₂, SH, OH, CF₃; a cycloalkyl group selected from the group consisting of cyclopropyl, cyclobutyl, cyclopentyl, cyclohexyl, cycloheptyl, cyclooctyl, adamantyl, pyrroline, pyrrolidine, pyrrolidineone, pyrazoline, pyrazolinone, oxopyrazolinone, aziridine, acetidine, tetrahydropyrrole, oxirane, oxetane, dioxetane, tetrahydrofurane, dioxane, dioxolane, oxathiolane, oxazolidine, thiirane, thietane, thiolane, thiane, thiazolidine, piperidine, piperazine or morpholine which is optionally substituted by one or more substituents independently selected from the group consisting of F, Cl, Br, I, NH₂, SH, OH, SO₂, CF₃, or CN; an alkyl-cycloalkyl group which is optionally at least mono-substituted by substituents independently selected from the group consisting of F, Cl, Br, I, NH₂, SH, OH, CF₃; an aryl group which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, CI, Br, I, NH₂, SH, OH, CF₃; a branched or unbranched alkyl-aryl group which is optionally at least mono-substituted by substituents independently selected from the group consisting of F, Cl, Br, I, NH₂, SH, OH, CF₃; an alkyl-heteroaryl group which is optionally at least mono-substituted by substituents independently selected from the group consisting of F, Cl, Br, I, NH₂, SH, OH, CF₃;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**43.** Medicament according to any of claims 27, 28, 30, 33 or 38, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -S0₂NH₂ or C₁₋₆-alkoxy; a (S0₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, represent a hydrogen atom; a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN; with the condition that R³ and R⁴ may not at the same time represent a hydrogen atom;
or
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a saturated or unsaturated 4 to 18 membered ring system comprising 1 to 3 optionally condensed rings, in which at least 1 carbon atom is optionally replaced by N or 0 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**44.** Medicament according to any of claims 27, 29, 34, or 39, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a saturated or unsaturated 4 to 18 membered ring system A, comprising 1 to 3 optionally condensed rings, in which additionally at least 1 carbon atom is optionally replaced by N or 0 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a saturated or unsaturated 4 to 18 membered ring system comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
3) an A-X-B-Y-C group,
wherein
A represents a saturated or unsaturated 4 to 18 membered ring system comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃; a C₁₋₆-aliphatic group; a C₁₋₆-alkoxy group; a (C=O)-OR¹⁰ group with R¹⁰ being a linear or branched C₁₋₆ alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**45.** Medicament according to any of claims 27, 30, 35 or 40, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴, identical or different, represent a hydrogen atom; a saturated or unsaturated, optionally at least mono-substituted C₃₋₈ aliphatic group with substituents independently selected from the group consisting of F, Cl, Br, I, NH₂, SH, OH, CF₃, CN, nitro; a saturated or unsaturated, optionally at least mono-substituted, optionally at least one heteroatom as ring member containing cycloalkyl group with substituents independently selected from the group consisting of F, CI, Br, I, NH₂, SH, OH, CF₃, CN, nitro; an unsubstituted heteroaryl group; a saturated or unsaturated, optionally at least mono-substituted C₁₋₆ alkyl-cycloalkyl group; a saturated or unsaturated, unsubstituted C₁₋₆ alkyl-aryl group; a saturated or unsaturated, unsubstituted C₁₋₆ alkyl-heteroaryl group; with the condition that R³ and R⁴ may not at the same time represent a hydrogen atom;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**46.** Medicament according to any of claims 27, 31, 36 or 41, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, S0₂, CF₃, CN, nitro, -S0₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ and R⁴ together with their bridging nitrogen atom form
1) a 4 to 18 membered ring system A in which the ring containing the nitrogen atom is saturated, but may be condensed with an unsaturated ring, comprising 1 to 3 optionally condensed rings, in which additionally at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a 4 to 18 membered ring system in which the ring containing the nitrogen atom is saturated, but may be condensed with an unsaturated ring, comprising 1 to 3 optionally condensed rings, in which at least 1 carbon atom is optionally replaced by N or O and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
3) an A-X-B-Y-C group,
wherein
A represents a 4 to 18 membered ring system in which the ring containing the nitrogen atom is saturated, but may be condensed with an unsaturated ring, comprising 1 to 3 optionally condensed rings, in which additionally at least 1 carbon atom is optionally replaced by N, S or O and which is optionally at least mono-substituted by F, Cl, Br, I, NH₂, SH, OH, CF₃, a C₁₋₆-aliphatic group; a C₁₋₆-alkyl-aryl group; a (C=O)-OR¹⁰ group with R¹⁰ being a linear or branched C₁₋₆ alkyl group;
X represents a bond, a -NH- group, a -(C=O)- group, a branched or linear, optionally substituted C₁₋₆-aliphatic chain, -O-, or a -SO₂-group;
B represents a saturated or unsaturated 4 to 12 membered ring system comprising 1 to 2 optionally condensed rings, in which at least 1 carbon atom is optionally replaced by N, S or O and which is optionally at least mono-substituted by F, Cl, Br, I, NH₂, SH, OH, oxo, CF₃, a C₁₋₆-aliphatic group; a C₁₋₆-alkoxy group; a (C=O)-OR¹⁰ group with R¹⁰ being a linear or branched C₁₋₆ alkyl group;
Y represents a bond, a -NH- group, a -(C=O)- group, a branched or linear, optionally substituted C₁₋₆-aliphatic chain, -O-, or a -SO₂-group;
C represents a saturated or unsaturated 4 to 12 membered ring system comprising 1 to 2 optionally condensed rings, in which at least 1 carbon atom is optionally replaced by N, S or 0 and which is optionally at least mono-substituted by F, Cl, Br, I, NH₂, SH, OH, oxo, CF₃; a C₁₋₆-aliphatic group; a C₁-₆-alkoxy group; a (C=O)-OR¹⁰ group with R¹⁰ being a linear or branched C₁₋₆ alkyl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**47.** Medicament according to any of claims 27, 32, 37 or 42, **characterized in that** R¹ represents a branched or unbranched C₁₋₆₋alkyl-aryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a branched or unbranched C₁₋₆₋alkyl-heteroaryl group wherein the C₁₋₆₋alkyl and/or the aryl group is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a benzhydryl group which is optionally substituted by one or more substituents independently selected from the group consisting of F, Cl, Br, I, NH₂, SH, OH, SO₂, CF₃, CN, nitro, -SO₂NH₂ or C₁₋₆-alkoxy; a (SO₂)-R⁶ group with R⁶ being an aryl group, preferably a phenyl or a naphtyl group, which is optionally at least mono-substituted by substituents independently selected from the group consisting of F, Cl, Br, I, NH₂, SH, OH, CF₃; a heteroaryl group which is optionally at least mono-substituted by substituents independently selected from the group consisting of F, Cl, Br, I, NH₂, SH, OH, CF₃;
R² represents a hydrogen atom;
R³ represents a hydrogen atom;
R⁴ represents a saturated or unsaturated, unsubstituted, linear C₃₋₈ aliphatic group; a saturated or unsaturated, optionally at least mono-substituted, optionally at least one heteroatom as ring member containing 4 or 5-membered cycloalkyl group; an unsubstituted heteroaryl group; a saturated or unsaturated, unsubstituted alkyl-cycloalkyl group; an unsubstituted C₁₋₆ alkyl-heteroaryl group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**48.** Medicament according to any of claims 27, 28, 33, 38 or 43, **characterized in that** R¹ represents a benzyl group or a (S0₂)-R⁶ group with R⁶ being an aryl group;
R² represents a(C=O)-R⁸ group, with R⁸ representing a hydrogen atom; a methyl group; an ethyl group; a propyl group; a butyl group; a pentyl group; a hexyl group;
R³ and R⁴, identical or different, a C₃₋₈ alkyl group which is optionally substituted by one or more substituents independently selected from the group consisting of F, Cl, Br, I, NH₂, SH, OH, CF₃, CN; an optionally at least mono-substituted quinoline group; a phenyl group which is optionally substituted by one or more substituents independently selected from the group consisting of F, Cl, Br, I, NH₂, SH, OH, CF₃, CN; a benzyl group which is optionally substituted by one or more substituents independently selected from the group consisting of F, Cl, Br, I, NH₂, SH, OH, CF₃, CN;
or
R³ and R⁴ together with their bridging nitrogen atom form
1) a ring system A, comprising: 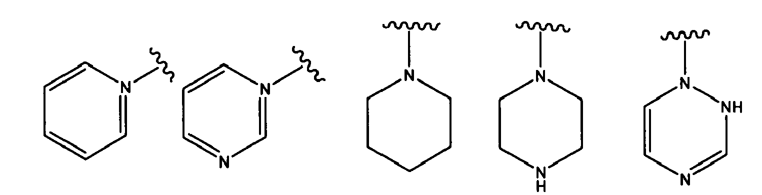 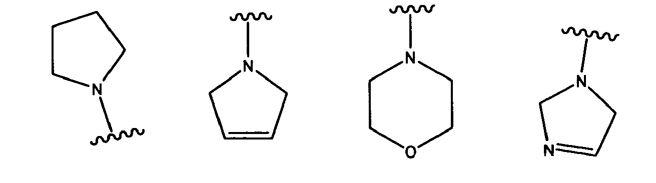 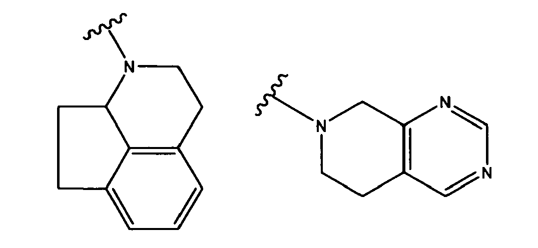 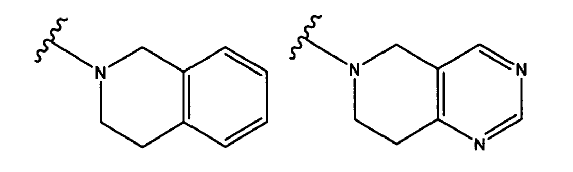 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a ring system comprising: 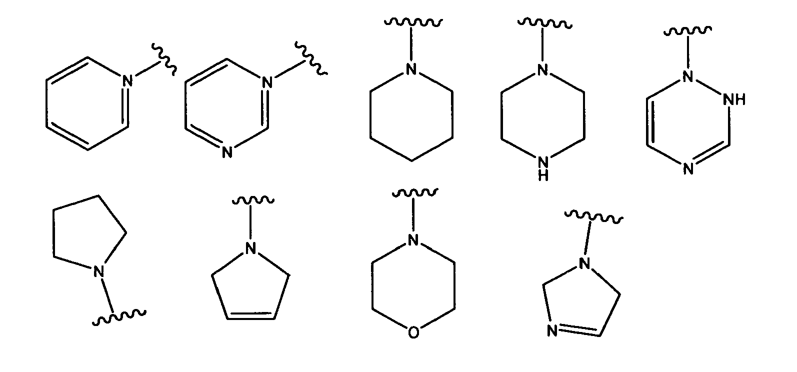 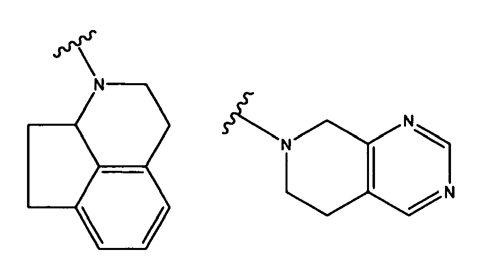 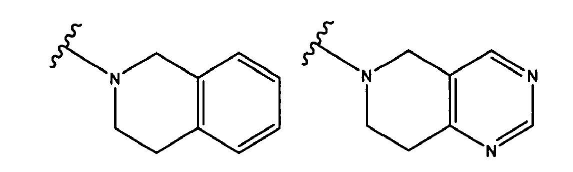 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear optionally substituted C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 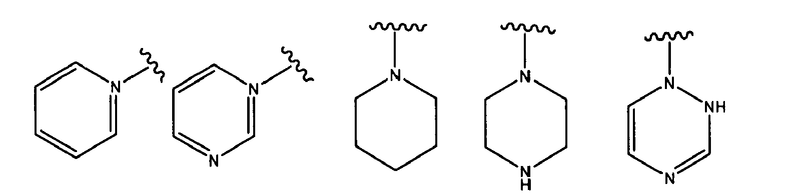 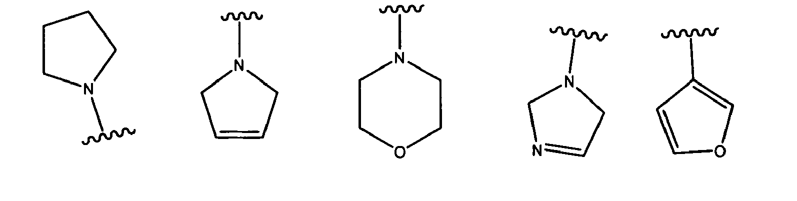 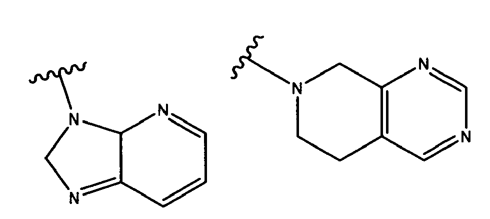 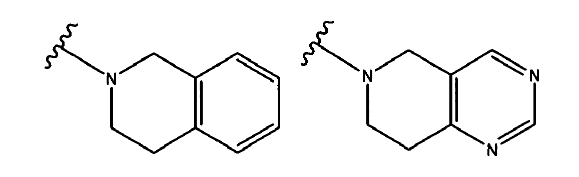 which is optionally at least mono-substituted by F, Cl, Br, I, NH₂, SH, OH, CF₃, a C₁₋₆-aliphatic group; a C₁₋₆-alkoxy group;
3) an A-X-B-Y-C group,
wherein
A represents a ring system comprising: 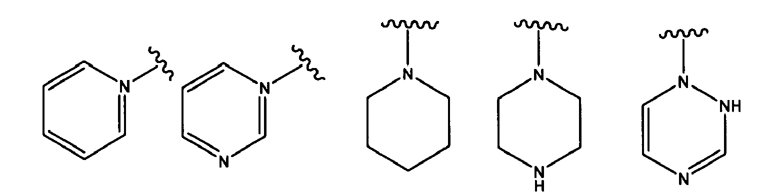 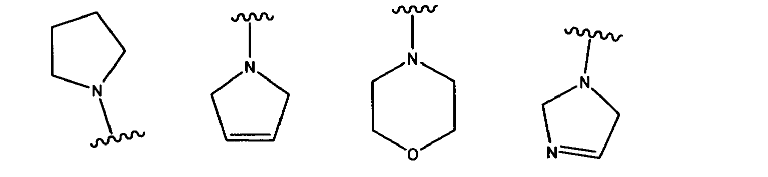 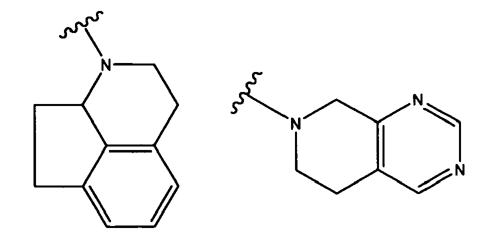 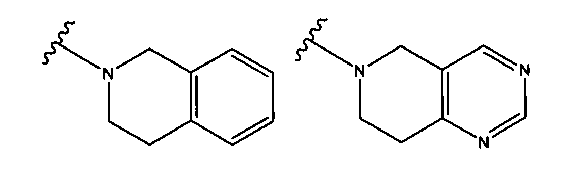 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 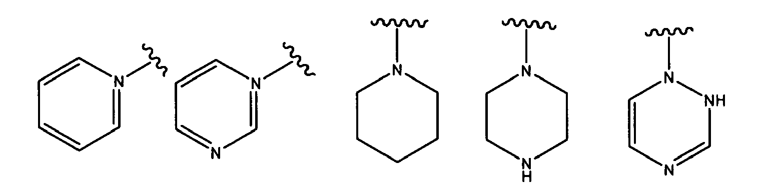 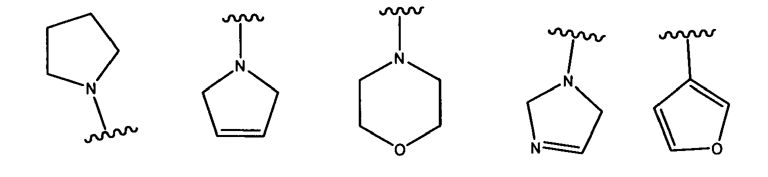 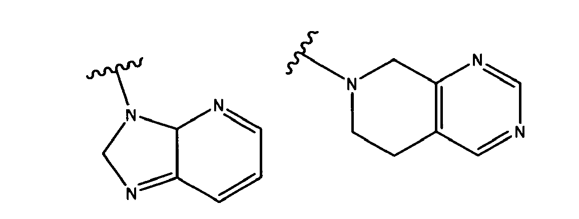 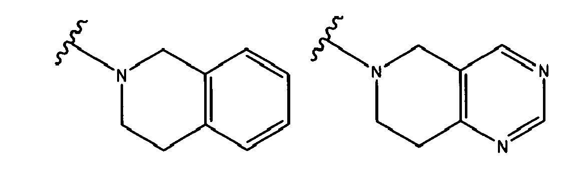 which is optionally at least mono-substituted by F, Cl, Br, I, NH₂, SH, OH, CF₃, a C₁₋₆-aliphatic group; a C₁₋₆-alkoxy group;
Y represents a bond, a -NH- group, a linear C₁₋₂-aliphatic group;
C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**49.** Medicament according to any of claims 27, 29, 34, 39, or 44, **characterized in that** R¹ represents a benzyl group or a (SO₂)-R⁶ group with R⁶ being an aryl group;
R² represents a methyl ,ethyl, propyl or butyl group which is optionally substituted by one or more substituents independently selected from the group consisting of F, Cl, Br, I, NH₂, SH, OH, CF₃; or a benzyl group which is optionally substituted by one or more substituents independently selected from the group consisting of F, Cl, Br, I, NH₂, SH, OH, CF₃;
R³ and R⁴ together with their bridging nitrogen atom form
1) a ring system A, comprising: 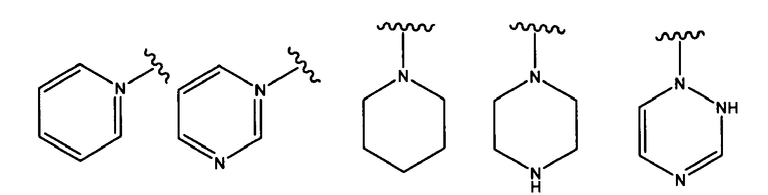 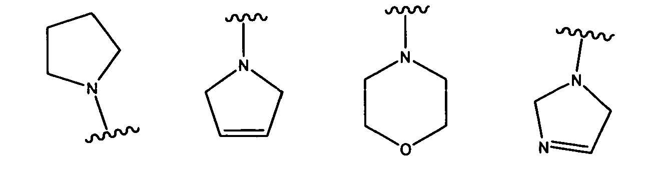 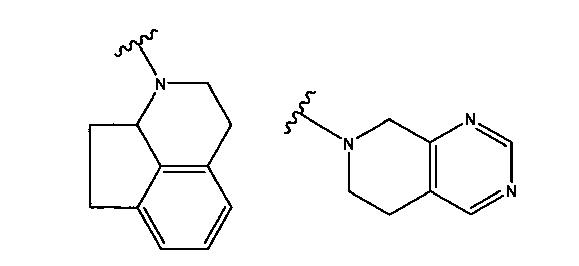 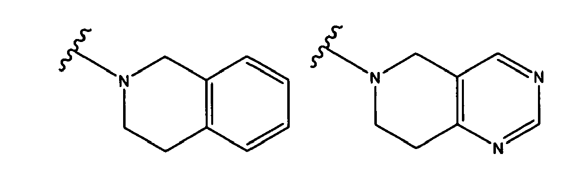 and which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a ring system comprising: 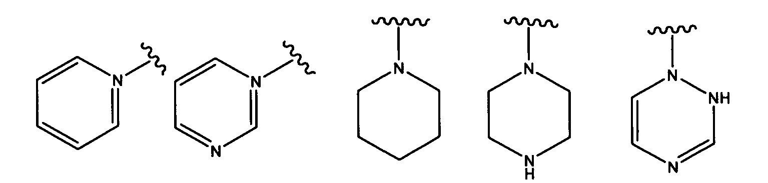 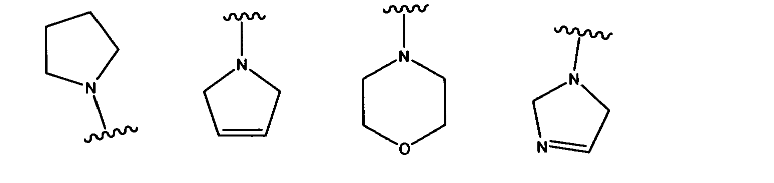 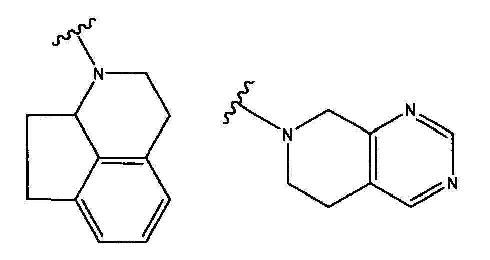 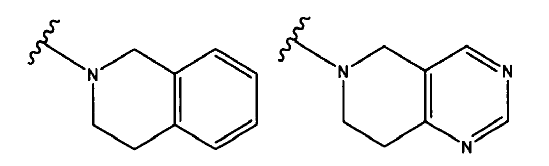 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -0-, or a -S0₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 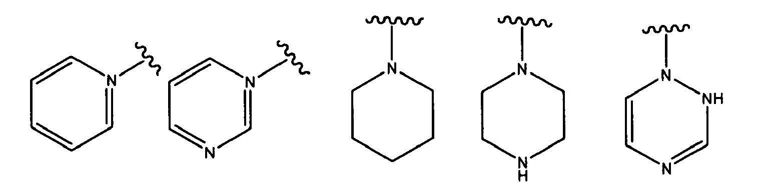 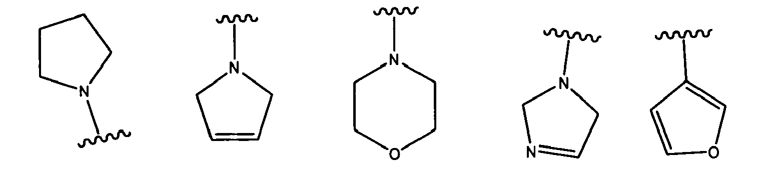 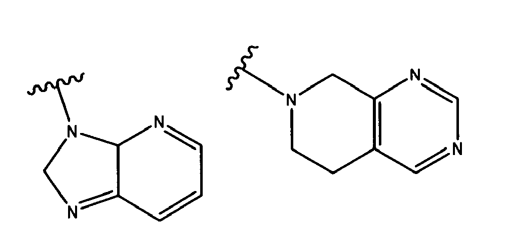 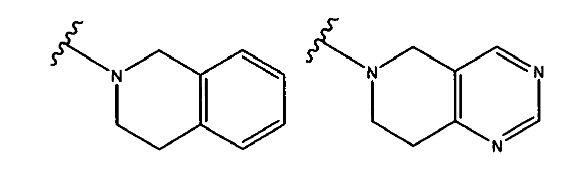 which is optionally at least mono-substituted by F, Cl, Br, I, NH₂, SH, OH, CF₃, a C₁₋₆-aliphatic group; a C₁₋₆-alkoxy group;
3) an A-X-B-Y-C group,
wherein
A represents a ring system comprising: 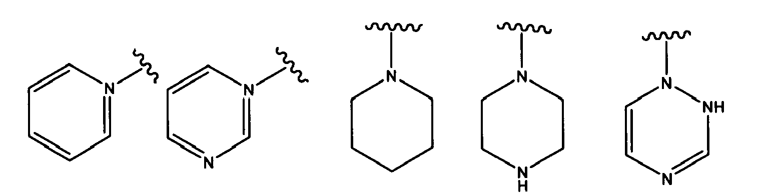 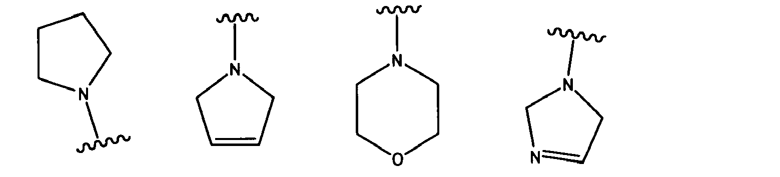 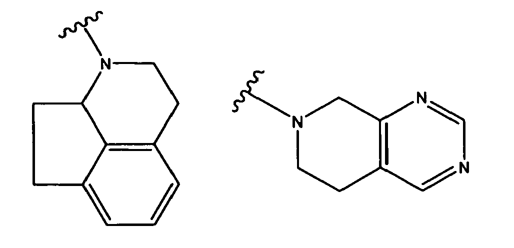 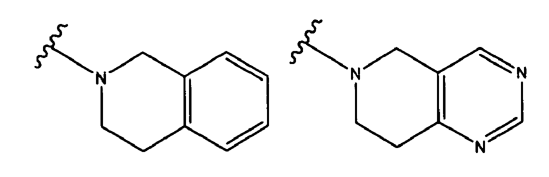 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 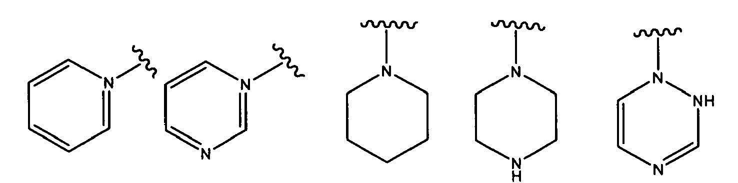 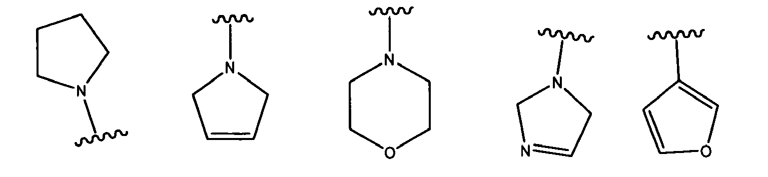 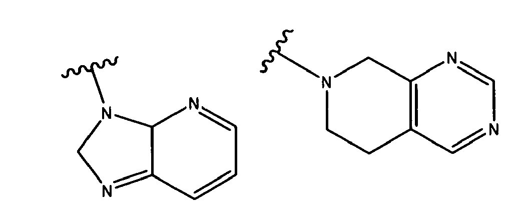 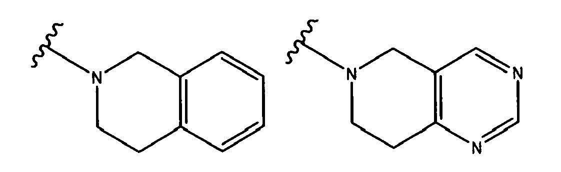 which is optionally at least mono-substituted by F, Cl, Br, I, NH₂, SH, OH, CF₃, a C₁₋₆-aliphatic group; a C₁₋₆-alkoxy group;
Y represents a bond, a -NH- group, a linear C₁-₂ alkyl group;
C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**50.** Medicament according to any of claims 27, 31, 36, 41, or 46, **characterized in that** R¹ represents a benzyl group or a (SO₂)-R⁶ group with R⁶ being an aryl group;
R² represents a hydrogen atom;
R³ and R⁴ together with their bridging nitrogen atom form
1) a ring system A, comprising: 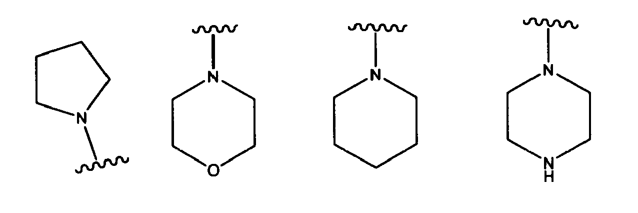 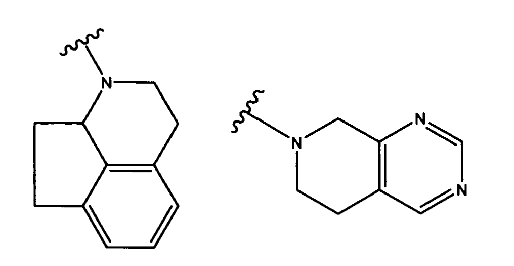 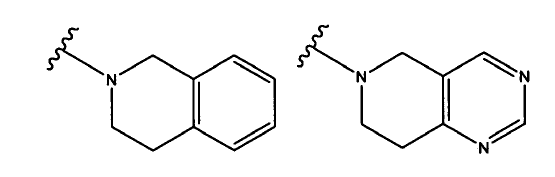 and which is optionally at least mono-substituted by F, CI, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
2) an A-X-B group,
wherein
A represents a ring system comprising: 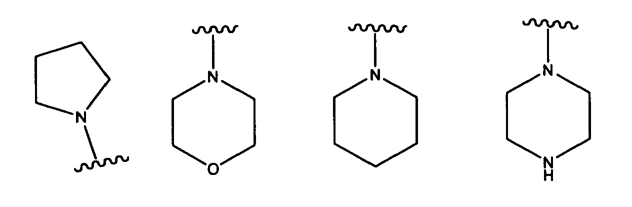 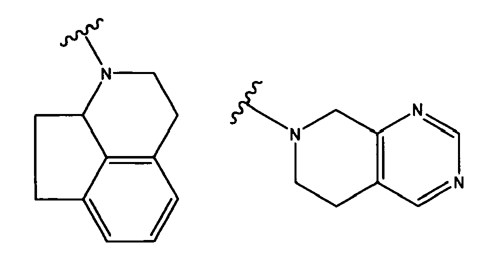 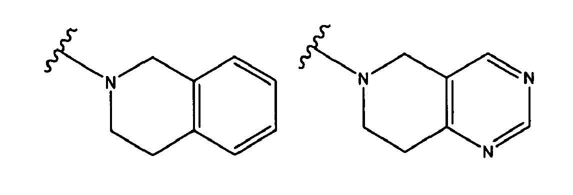 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 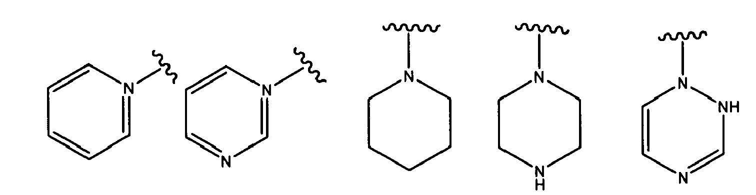 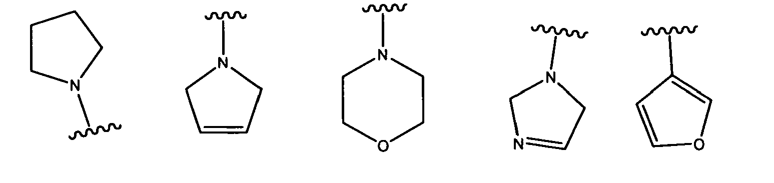 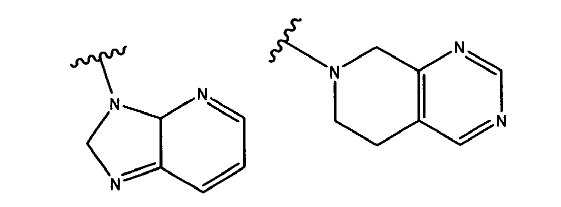 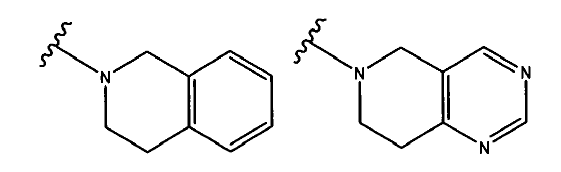 which is optionally at least mono-substituted by F, Cl, Br, I, NH₂, SH, OH, CF₃, a C₁₋₆-aliphatic group; a C₁₋₆-alkoxy group;
3) an A-X-B-Y-C group,
wherein
A represents a ring system comprising: 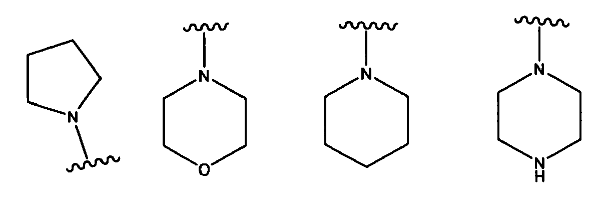 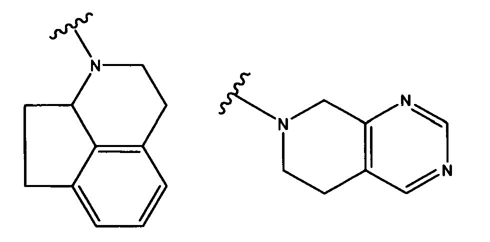 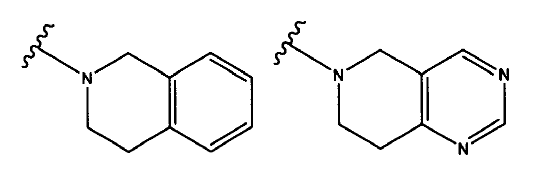 which is optionally at least mono-substituted by F, Cl, Br, I, NH₂, SH, OH, CF₃; a C₁₋₆-aliphatic group; a C₁₋₆-alkoxy group; a (C=O)-OR¹⁰ group with R¹⁰ being a linear or branched C₁₋₆ alkyl group;
X represents a bond, a -NH- group, a -(C=O)- group, a linear C₁₋₂ alkyl chain, -O-, or a -SO₂- group;
B represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group; or a ring system comprising: 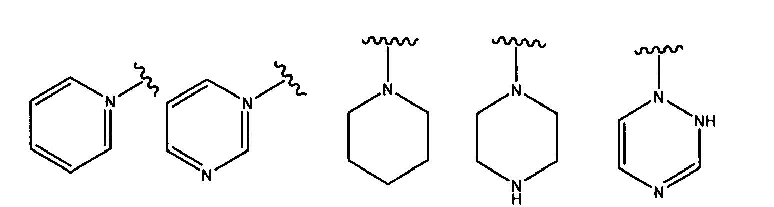 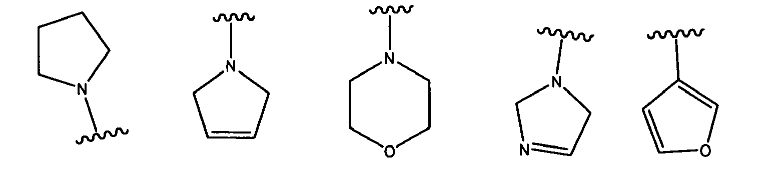 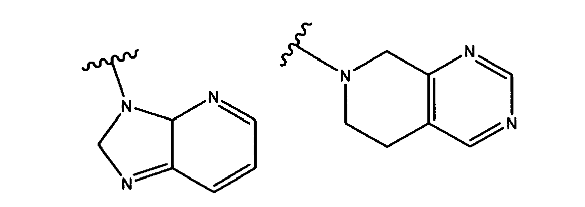 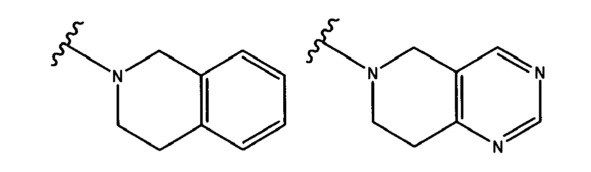 which is optionally at least mono-substituted by F, Cl, Br, I, NH₂, SH, OH, CF₃, a C₁₋₆-aliphatic group; a C₁₋₆ alkoxy group;
Y represents a bond, a -NH- group, a linear C₁₋₂-alkyl group;
C represents an aryl group which is optionally at least mono-substituted by F, Cl, Br, I, NH₂, SH, OH, oxo, CF₃, a C₁₋₆-aliphatic group; a C₁₋₆-alkoxy group; a naphtyl group which is optionally at least mono-substituted by F, Cl, Br, I, NH₂, SH, OH, CF₃, a C₁₋₆-aliphatic group; a C₁₋₆-alkoxy group;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, or a prodrug thereof,
and optionally one or more pharmaceutically acceptable excipients.

**51.** Medicament according to one or more of claims 27 to 50, **characterized in that** it comprises at least one compound selected from the group consisting of
[1] (3,4-trans)-1-benzyl-4-(3,4-dihydroisoquinolin-2(1H)-yl)pyrrolidine-3-ol,
[2] (3,4-trans)-1-benzyl-4-morpholinopyrrolidine-3-ol,
[3] (3,4-trans)-1-benzyl-4-(4-(2-methyl-3H-imidazo[4,5-b]pyridin-3-yl)pipeddin-1 -yl)pyrrolidine-3-ol,
[4] (3,4-trans)-1-benzyl-4-(3-(6,7-dimethoxy-3,4-dihydroisoquinolin-2(1H)-yl)propyl amino)pyrrolidine-3-ol,
[5] (3,4-trans)-1-benzyl-4-(4-((3,4-dihydroisoquinolin-2(1H)-yl)methyl)piperidin-1-yl)pyrrolidine-3-ol,
[6] 1-(4-(6-((3,4-trans)-1-benzyl-4-hydroxypyrrolidine-3-yl)-5,6,7,8-tetrahydropyrido [4,3-d]pyrimidin-2-ylamino)phenyl)ethanone,
[7] 1-(1-((3,4-trans)-1-benzyl-4-hydroxypyrrolidine-3-yl)piperidin-4-yl)-1H-benzo[d] [1,3] oxazin-2(4H)-one,
[8] (3,4-trans)-1-benzyl-4-(2-(4-phenoxyphenylamino)-7,8-dihydropyrido[4,3-d]pyrimidin-6(5H)-yl)pyrrolidine-3-ol,
[9] (3,4-trans)-1-benzyl-4-(3-(3,4-dihydroisoquinolin-2(1 1 H)-yl)propylamino)pyrrolidine -3-ol,
[10] (3,4-trans)-1-benzyl-4-(4-(4-methoxypyrimidin-2-yl)piperazin-1-yl)pyrrolidine-3-ol,
[11] (3,4-trans)-1-benzyl-4-(2,3,8,8a-tetrahydrocyclopenta[ij]isoquinolin-1 (7H)-yl) pyrrolidine-3-ol,
[12] tert-butyl-4-((3,4-trans)-1-benzyl-4-hydroxypyrrolidine-3-yl)piperazine-1-carboxylate,
[13] tert-butyl 4-((3,4-trans)-4-hydroxy-1-(phenylsulfonyl)pyrrolidine-3-yl)piperazine-1-carboxylate,
[14] (3,4-trans)-4-morpholino-1-(phenylsulfonyl)pyrrolidine-3-ol,
[15] (3,4-trans)-4-(3,4-dihydroisoquinolin-2(1 H)-yl)-1-(phenylsulfonyl)pyrrolidine-3-ol,
[16] (3,4-trans)-4-(3,4-dihydroisoquinolin-2(1H)-yl)-1((S)-1-phenylethyl)pyrrolidine-3-ol,
[17] (3,4-trans)-1-benzyl-4-(4-benzylpiperazin-1-yl)pyrrolidine-3-ol,
[18] (3,4-trans)-1-benzyl-4-(4-phenylpiperazin-1-yl)pyrrolidine-3-ol,
[19] Ethyl 2-(4-((3,4-trans)-1-benzyl-4-hydroxypyrrolidine-3-yl)piperazin-1-yl)acetate,
[20] (4-((3,4-trans)-1-benzyl-4-hydroxypyrrolidine-3-yl)piperazin-1-yl)(phenyl) methanone,
[21] Ethyl 4-((3,4-trans)-1-benzyl-4-hydroxypyrrolidine-3-yl)piperazine-1-carboxilate,
[22] tert-butyl 4-((3,4-trans)-1-(4-fluorobenzyl)-4-hydroxypyrrolidine-3-yl)piperazine-1-carboxylate,
[23] (3,4-trans)-1-benzyl-4-(4-methylpiperazin-1-yl)pyrrolidine-3-ol,
[24] tert-butyl-4-(3,4-trans)-4-acetoxy-1-benzylpyrrolidine-3-yl)piperazine-1-carboxylate,
[25] (3,4-trans)-1-benzyl-4-(piperazin-1-yl)pyrrolidine-3-yl acetate,
[26] tert-butyl 4-((3,4-trans)-4-acetoxypyrrotidine-3-yl)piperazine-1-carboxylate,
[27] trans 1-Benzyl-4-(4-(2-phenyl-phenylsulfonyl)piperazin-1-yl)pyrrolidin-3-yl acetate,
[28] trans 1-Benzyi-4-(4-(2-phenyl-phenylsulfonyl)piperazin-1-yl)pyrrolidin-3-ol dihydrochloride,
[29] (3,4-trans)-1-benzyl-4-(phenylamino)pyrrolidin-3-ol,
[30] (3,4-trans)-1-benzyl-4-(benzylamino)pyrrolidin-3-ol,
[31] (3R,4R)-1-benzyl-4-(octylamino)pyrrolidin-3-ol,
[32] (3,4-trans)-1-benzyl-4-(4-methoxybenzylamino)pyrrolidin-3-ol,
[33] (3,4-trans)-1-benzyl-4-(butylamino)pyrrolidin-3-ol,
[34] (3R,4R)-1-benzyl-4-(4-(4-chloro-2,5-dimethylphenylsulfonyl)piperazin-1-yl)pyrrolidin-3-yl acetate,
[35] (3R,4R)-1-benzyl-4-(4-tosylpiperazin-1-yl)pyrrolidin-3-yl acetate,
[36] (3,4-trans)-1-benzyl-4-(butylamino)pyrrolidin-3-ol,
[37] (3R,4R)-1-benzyl-4-(4-methoxyphenylamino)pyrrolidin-3-ol,
[38] Methyl 4-((3,4-trans)-1-benzyt-4-hydroxypyrro)idin-3-ylamino)benzoate,
[39] (3R,4R)-1-benzyl-4-(4-(naphthalen-1-ylsulfonyl)piperazin-1-yl)pyrrolidin-3-yl acetate,
[40] (3R,4R)-1-benzyl-4-(4-(2-chlorophenylsulfonyl)piperazin-1-yl)pyrrolidin-3-ol,
[41] (3R,4R)-1-benzyl-4-(4-(2-bromophenylsulfonyl)piperazin-1-yl)pyrrolidin-3-yl acetate,
[42] (3R,4R)-1-benzyl-4-(4-(2-bromophenylsulfonyl)piperazin-1-yl)pyrrolidin-3-ol,
[43] (3R,4R)-1-benzyl-4-(4-(5-fluoro-2-methylphenylsulfonyl)piperazin-1-yl)pyrrolidin-3-yl acetate,
[44] (3R,4R)-1-benzyl-4-(4-(5-fluoro-2-methylphenylsulfonyl)piperazin-1-yl)pyrrolidin-3-ol,
[45] (3R,4R)-1-benzyl-4-(4-(phenylsulfonyl)piperazin-1-yl)pyrrolidin-3-yl acetate,
[46] (3R,4R)-1-benzyl-4-(4-(phenylsulfonyl)piperazin-1-yl)pyrrolidin-3-ol,
[47] (3R,4R)-1-benzyl-4-(4-(2-fluorophenylsulfonyl)piperazin-1-yl)pyrrolidin-3-yl acetate,
[48] (3R,4R)-1-benzyl-4-(4-(2-fluorophenylsulfonyl)piperazin-1-yl)pyrrolidin-3-ol,
[49] (3R,4R)-1-benzyl-4-(4-(naphthalen-1-ylsulfonyl)piperazin-1-yl)pyrrolidin-3-ol,
[50] (3R,4R)-1-benzyl-4-(4-(4-chloro-2,5-dimethylphenylsulfonyl)piperazin-1-yl)pyrrolidin-3-ol,
[51] (3R,4R)-1-benzyl-4-(4-tosylpiperazin-1-yl)pyrrolidin-3-ol,
[52] (3R,4R)-1-benzyl-4-(4-(2-chlorophenylsulfonyl)piperazin-1-yl)pyrrolidin-3-yl acetate,
optionally in form of a corresponding salt, or a corresponding solvate.

**52.** Use of at least one substituted pyrrolidine compound according to one or more of claims 1 to 25 and optionally one or more pharmaceutically acceptable excipients, for the preparation of a medicament for the prophylaxis and/or treatment of of a sigma receptor-mediated disease or condition.

**53.** Use according to claim 52, **characterized in that** the sigma receptor-mediated disease or condition is Alzheimer's disease (AD).

**54.** Use according to claim 52, **characterized in that** the sigma receptor-mediated disease or condition is selected from the group consisting of diarrhoea, lipoprotein disorders, migraine, obesity, arthritis, hypertension, arrhythmia, ulcer, learning, memory and attention deficits, cognition disorders, neurodegenerative diseases, demyelinating diseases, addiction to drugs and chemical substances including cocaine, amphetamine, ethanol and nicotine; tardive diskinesia, ischemic stroke, epilepsy, stroke, stress, cancer or psychotic conditions, in particular depression, anxiety, psychosis or schizophrenia; inflammation, or autoimmune diseases.

**55.** Use according to claim 52, **characterized in that** the sigma receptor-mediated disease or condition is selected from the group consisting of elevated trigyceride levels, chylomicronemia, dysbetalipoproteinemia, hyperlipoproteinemia, hyperlipidemia, mixed hyperlipidemia, hypercholesterolemia, lipoprotein disorders, hypertriglyceridemia, sporadic hypertriglyceridemia, inherited hypertriglyceridemia and/or dysbetalipoproteinemia.

**56.** Use according to claim 52, **characterized in that** the sigma receptor-mediated disease or condition is selected from the group consisting of pain, preferably neuropathic pain, inflammatory pain or other pain conditions involving allodynia and/or hyperalgesia.

**57.** Use of a compound of general formula (I) according to one or more of claims 1 to 25 as a pharmacological tool.
